# EUROPEAN PATENT SPECIFICATION

(11) **EP 2 625 171 B1**
(45) Date of publication and mention of the grant of the patent: **30.07.2014**
(21) Application number: 11764186.0
(22) Date of filing: 04.10.2011
(51) Int. Cl.: C07D 307/79, C07D 405/04, C09K 11/06

(54) **PHENANTHRO[9,10-B]FURANS FOR ELECTRONIC APPLICATIONS**
PHENANTHRO[9,10-B]FURANE FÜR ELEKTRONISCHE ANWENDUNGEN
PHÉNANTHRO[9,10-B]FURANES POUR APPLICATIONS ÉLECTRONIQUES

(30) Priority: 07.10.2010 EP 10186807
(43) Date of publication of application: 14.08.2013
(73) Proprietor: BASF SE, 67056 Ludwigshafen (DE)
(72) Inventor: SCHÄFER, Thomas, CH-4410 Liestal (CH); SCHILDKNECHT, Christian, San Diego CA 92124 (US); MURER, Peter, CH-4104 Oberwil (CH); LENNARTZ, Christian, 67105 Schifferstadt (DE); LANGER, Nicolle, 64646 Heppenheim (DE); WAGENBLAST, Gerhard, 67157 Wachenheim (DE); METZ, Stefan, 68165 Mannheim (DE)
(74) Representative: Leybach, Holger Hugo
(86) International application number: PCT/EP2011/067255
(87) International publication number: WO 2012/045710

(56) References cited:
- EP-A2- 1 067 165
- WO-A1-2008/031743
- WO-A1-2008/119666
- US-A- 5 077 142
- WILLIAM M. HORSPOOL ET AL: "Substituent and wavelength effects in the photochemistry of 5,6,7,8-tetrachloro-3a,9a-dihydro-2,3,9a-t riarylfuro(2,3-b)(1,4)benzodioxin derivatives", TETRAHEDRON LETTERS, vol. 24, no. 35, 1 January 1983 (1983-01-01), pages 3745-3748, XP55010655, ISSN: 0040-4039, DOI: 10.1016/S0040-4039(00)94524-6

## Description

The present invention relates to an electronic device, especially an electroluminescent device, comprising a compound of the formula I, especially as host for phosphorescent compounds. The hosts may function with phosphorescent materials to provide improved efficiency, stability, manufacturability, or spectral characteristics of electroluminescent devices.

US5077142 relates to electroluminescent devices comprising an anode and a cathode sandwiching ≥1 organic layer(s) in which the organic layer(s) include a compound represented by the general formula (B)ₘ-(A)ₙ (B = selected cyclic hydrocarbons, condensed polycyclic hydrocarbons, O-contg. heterocycles, N-contg. heterocycles, and S-contg. heterocycles; A = benzene, biphenyl, methoxybenzene, or naphthalene groups; m = an integer in the range 1-6; and n = an integer in the range 1-6). The following compound is explicitly disclosed:

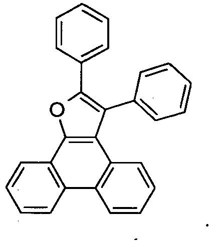

EP1067165 describes organic electroluminescent elements comprising a light emitting layer comprised of ≥1 thin layers of an organic compound put between an anode and a cathode in which ≥1 org. compound thin layer contains an organometallic complex having both an ionic coordinate bond formed by a nitrogen anion (e.g., included in an arom. heterocyclic (ring) and a metal cation and a coordinate bond formed between a nitrogen atom or a chalcogen and a metal. The metal cation of the organic metal complex may be selected from Al, Ga, In, TI, Be, Mg, Sr, Ba, Ca, Zn, Cd, Hg, Pd, or Cu. The following metal complexes are explicitly disclosed: 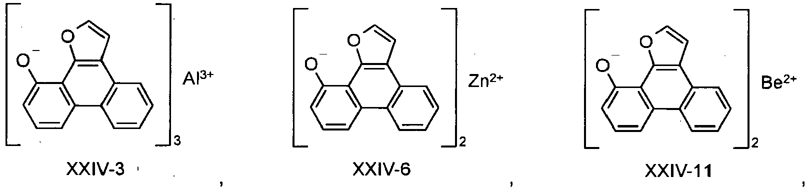 and

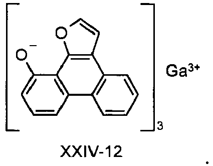

US7183010 relates to org. light-emitting devices which comprise a substrate; an anode and a cathode disposed over the substrate; a luminescent layer disposed between the anode and the cathode are described in which the luminescent layer includes a host and 1 dopant; the host including a solid org. material comprising a mixt. of 2 components including a first component that is an org. compd. capable of transporting either electrons and/or holes and of forming both monomer state and an aggregate state and a second component of that is an org. compd. that upon mixing with the first host component is capable of forming a continuous and substantially pin-hole-free layer, while the dopant of is selected to produce light from the light-emitting device. Dinaphtho[1',2':2,3;2",1":10,11]perylo[1,12]furan (194-45-6; Tetrabenzo[1,2:5,6:7,8:11,12] pentapheno [13,14-bcd]furan (8CI,9CI)) is explicitly disclosed

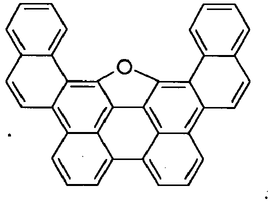

US6828044 describes a device wherein the dopant comprises a benzofurane as represented by the following formula 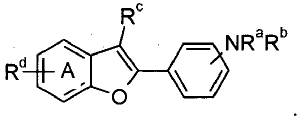 R^{a} and R^{b} independently represent an aryl or heteroaryl group and the nitrogen to which they are bonded is located at the 3- or 4-position of the phenyl ring; and R^{c} represents hydrogen or an alkyl, aryl or heteroaryl group; and R^{d} represents one or more hydrogen or alkyl, substituted nitrogen, aryl or heteroaryl groups which may join to form a ring fused to ring A.

WO2006097419 describes polymers which can contain repeating units of formula 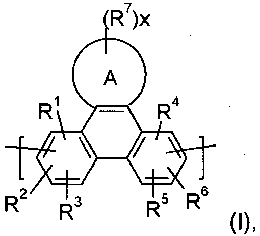 wherein A is a 5-, 6-, or 7-membered heteroaromatic ring, containing at least one heteroatom selected from nitrogen, oxygen and sulfur, especially one nitrogen atom and at least one further heteroatom selected from nitrogen, substituted nitrogen, oxygen and sulfur.

WO2007/090773 relates to polymers comprising repeating unit(s) of the formula 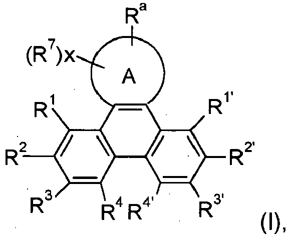 wherein A is a 5-, 6-, or 7-membered heteroaromatic ring, containing at least one heteroatom selected from nitrogen, oxygen and sulfur, especially one nitrogen atom and at least one further heteroatom selected from nitrogen, substituted nitrogen, oxygen and sulfur, at least one of R^{a}, R¹, R², R³, R⁴, R^{1'}, R²', R^{3'} and R^{4'} is a group R¹⁰, wherein R¹⁰ is a group -(Sp)ₓ₁-[PG']<, wherein Sp is a spacer unit, PG' is a group derived from a polymerisable group, x1 is 0, or 1, and x is 0, or an integer of 1 to 4.

WO2008031743 relates electroluminescent devices, comprising a compound of the formula 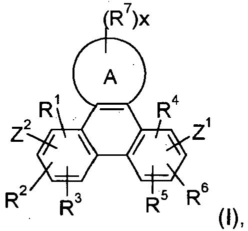 especially as host for phosphorescent compounds. A is a 5-, 6-, or 7-membered heteroaromatic ring, containing at least one heteroatom selected from nitrogen, oxygen and sulfur, especially one nitrogen atom and at least one further heteroatom selected from nitrogen, substituted nitrogen, oxygen and sulfur.

WO2008/119666 discloses compounds of the formula 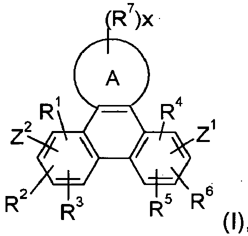 a process for their preparation and their use in organic light emitting diodes (OLEDs), especially as host for phosphorescent compounds. A is a 5-, 6-, or 7-membered heteroaromatic ring, containing at least one heteroatom selected from nitrogen, oxygen and sulfur, especially one nitrogen atom and at least one further heteroatom selected from nitrogen, substituted nitrogen, oxygen and sulfur.

### Examples of A are:

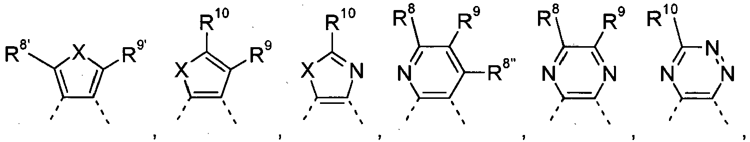

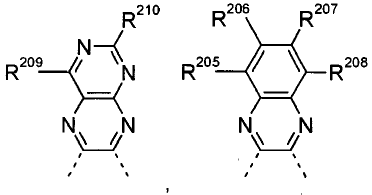

or 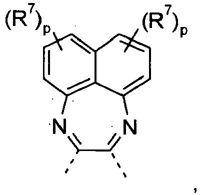 wherein R⁷ has the meaning of R⁸, R^{8"} has the meaning of R⁸, X is O, S, N-R¹⁷, wherein R²⁰⁵, R²⁰⁶, R²⁰⁶, R²⁰⁷, R²⁰⁹, R²¹⁰, R8, R⁹, R^{8'}, R^{9'}, R¹⁰ and R¹⁷ are as defined below, p is 0, 1, 2, or 3 and the dotted lineindicates the bonding to the biphenyl unit.

Notwithstanding these developments, there remains a need for organic light emitting devices comprising new host and transport materials to provide improved efficiency, stability, manufacturability, and/or spectral characteristics of electroluminescent devices.

Accordingly, it was the object of the present invention to provide compounds, which when used in organic electronic devices, especially organic light emitting devices showing good efficiencies, good operative lifetimes, good manufacturability, good spectral characteristics, a high stability to thermal stress, and/or a low operating voltage.

Said object has been solved by compounds of the formula 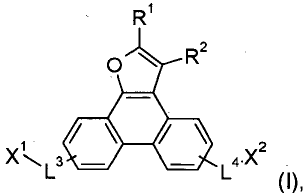 wherein R¹ and R² are independently of each other H, F, C₁-C₁₈alkyl, C₁-C₁₈alkyl which is substituted by E and/or interrupted by D, C₆-C₂₄aryl, C₆-C₂₄aryl which is substituted by G, C₂-C₂₀heteroaryl, C₂-C₂₀heteroaryl which is substituted by G, or R¹ and R² form together a group 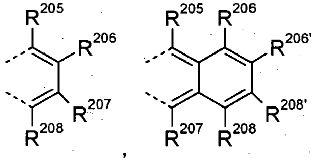 or 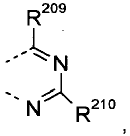 wherein R^{206'}, R^{208'}, R²⁰⁵, R²⁰⁶, R²⁰⁷, R²⁰⁸, R²⁰⁹ and R²¹⁰ are independently of each other H, C₁-C₁₈alkyl, C₁-C₁₈alkyl which is substituted by E and/or interrupted by D, C₁-C₁₈alkoxy, or C₁-C₁₈alkoxy which is substituted by E and/or interrupted by D, C₁-C1₈fluoroalkyl, C₆-C₂₄aryl, C₆-C₂₄aryl which is substituted by G, C₂-C₂₀heteroaryl, C₂-C₂₀heteroaryl which is substituted by G, C₂-C₁₈alkenyl, C₂-C₁₈alkynyl, C₇-C₂₅aralkyl, C₇-C₂₅aralkyl which is substituted by G; CN, or -CO-R₂₈,
X¹ and X² are independently of each other a group -NA¹A^{1'},
A¹ and A^{1'} are independently of each other a C₆-C₂₄aryl group, a C₆-C₂₄aryl group, which is substituted by G; a C₂-C₂₀heteroarylL group, or a C₂-C₂₀heteroaryl group which is substituted by G; or
A¹ and A^{1'} together with the nitrogen atom to which they are bonded form a heteroaromatic ring, or ring system, such as 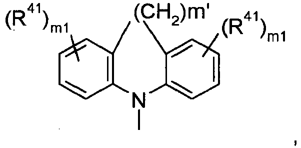 or 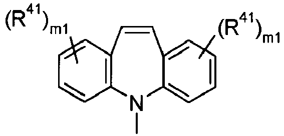 (= group I); m' is 0, 1, or 2; a C₁₀-C₂₈aryl group (= group III), which can optionally be substituted by G; and/or -L³-X¹ and -L⁴-X² are independently of each other a group of formula 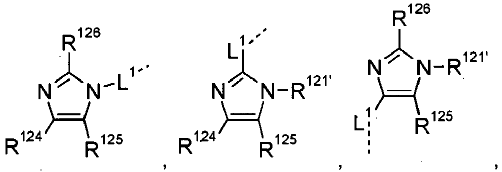 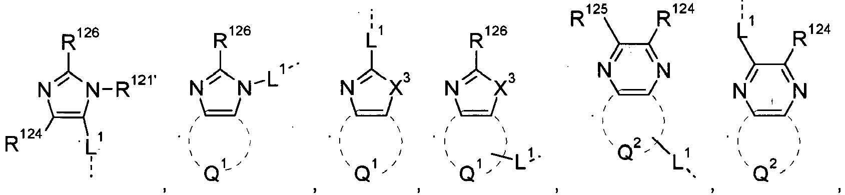 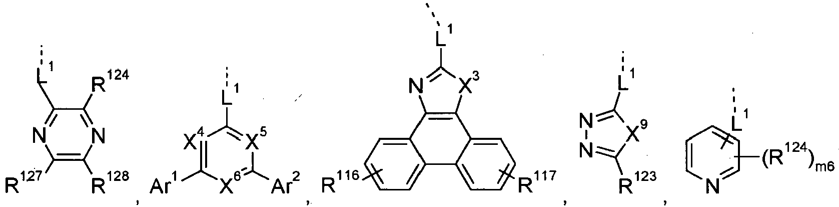 or 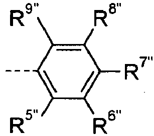 (= group II); wherein
R^{5"}, R^{6"}, R^{7"}, and R^{8"} are independently of each other C₆-C₁₈aryl; which may optionally be substituted by G; or C₂-C₂₀heteroaryl, which may optionally be substituted by G, R^{9"} is hydrogen, or has the meaning of R^{5"}, m6 is an integer of 1 to 4,
X³ represents O, S or N-R^{121'}, especially N-R^{121'},
X⁹ represents O, S or N-R^{121'}, especially O,
Q¹ and Q² represents atoms necessary for forming a carbocyclic aromatic, or heterocyclic aromatic ring, which can optionally be condensed with other ring(s) to form a condensed ring, and/or can optionally be substituted by G,
R¹¹⁶ and R¹¹⁷ are independently of each other H, halogen, -CN, C₁-C₁₈alkyl, C₁-C₁₈alkyl which is substituted by E and/or interrupted by D, C₆-C₂₄aryl, C₆-C₂₄aryl which is substituted by G, C₂-C₂₀heteroaryl, C₂-C₂₀heteroaryl which is substituted by G, C₂-C₁₈alkenyl, C₂-C₁₈alkynyl_{,} C₁-C₁₈alkoxy, C₁-C₁₈alkoxy which is substituted by E and/or interrupted by D, C₇-C₂₅aralkyl, C₇-C₂₅ₐralkyl, which is substituted by G; -C(=O)-R⁷⁸, -C(=O)OR⁷⁷, or-C(=O)NR⁷⁵R⁷⁶, or
substituents R¹¹⁶, R¹¹⁷ and R^{117'}, which are adjacent to each other, can form 8 ring,
R⁷⁵, R⁷⁶ and R⁷⁸ are independently of each other H; C₆-C₁₈aryl; C₁-C₁₈aryl which is substituted by C₁-C₁₈alkyl, or C₁-C₁₈alkoxy; C₁-C₁₈alkyl; or C₁-C₁₀alkyl which is interrupted by -O-R⁷⁷ is C₆-C₁₈aryl: C₆-C₁₈aryl which is substituted by C₁-C₁₈alkyl, or C₁-C₁₈alkoxy; C₁-C₁₈alkyl; or C₁-C₁₈alkyl which is interrupted by -O-,
R¹²¹' is C₆-C₁₈aryl: or C₂-C₂₀heteroaryl; wh ich can optionally be substituted by C₁-C₁₈alkyl, C₁-C₁₈fluoroalkyl, or C₁-C₁₈alkoxy, C₁-C₁₈alkyl; or C₁-C₁₈alkyl which is interrupted by -O-; R¹⁴¹, R¹²⁴, R¹²⁵ and R¹²⁸ are independently of each other H, C₁-C₁₈alkyl, C₁-C₁₈alkyl which is substituted by E and/or interrupted by D, C₁-C₁₈fluoroalkyl, C₆-C₁₄aryl, which can optionally be substituted by G, C₂-C₂₀heteroaryl, which can optionally be substituted by G, C₂-C₁₀alkenyl, C₂-C₁₈alkynyl, C₁-C₁₈alkoxy, C₁-C₁₈alkoxy which is substituted by E and/or intarrupted by D, or C₇-C₂₅arylkyl,
R¹²⁷ and R¹²⁸ are independenfly of each other H, CN. C₁-C₁₈alkyl, C₁-C₁₈alkyl which is substituted by E and/or interrupted by D, C₁-C₁₈fluoroalkyl, C₆-C₂₄aryl, which can optionally be substituted by G, C₂-C₂₀heteroaryl, which can optionally be substituted by G, C₂-C₁₈alkanyl, C₂-C₁₈alkynyl, C₁-C₁₈alkoxy, C₁-C₁₈alkoxy which is substituted by E and/or interrupted by D. or C₇-C₂₅aralkyl.
L¹ is a single bond, -(CR⁴⁷=CR⁴⁸)ₘ₂-, -(Ar¹)ₘ₃-, -[Ar³(Y¹)ₘ₅]ₘ₄-, -[(Y¹)ₘ₅Ar³]m₄-, or
-[Ar³(Y²)ₘ₅Ar⁴]ₘ₄-. wherein
Y¹ is -(CR⁴⁷=CR⁴⁸)-,
Y² is NR⁴⁹, O, S, C=O, C(=O)O, wherein R⁴⁹ is H; C₅-C₁₈aryl which can optionally be substituted by C₁-C₁₈alkyl, or C₁-C₁₈alkoxy; C₁-C₁₈alkyl; or C₁-C₁₈-alkyl which is interrupted by -OR⁴⁷ and R⁴⁸ are independently of each other hydrogen, fluorine, C₁-C₂₅alkyl, or C₆-C₂₄aryl, which can optionally be substituted by G,
m2 is an integer of 1 to 10, m3 is an integer of 1 to 5, m4 is an integer of 1 to 5, m5 is an integer of 1 to 10,
Ar¹ and Ar² are independently of each other C₆-C₂₄aryl, which can optionally be substituted by G, or C₂-C₂₀heteroaryl, which can optionally be substituted by G.
Ar³ and Ar⁴ are independently of each other arylen, or heteroarylen, which can optionally be substituted
X⁴, X⁵ and X⁶ are independently of each other N, or CH, with the proviso that at least one.
preferably at least two of the substituents X⁴, X⁵ and X⁶ are N, and L³ and L⁴ are independently of each other a single bond, or a bridging unit BU, such as 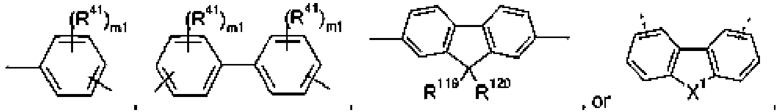 R⁴⁷ and R⁴⁸ are Independently of each other H, C₁-C₁₈alkyl, or C₆-C₁₀aryl, which may optionally be substituted by one, or more C₁-C₈alkyl groups, or C₁-C₆alkoxy groups;
R⁴¹ can be the same or different at each occurence and is Cl, F, CN, NR⁴⁵R^{45'}, a C₁-C₂₅alkyl group, a C₄-C₁₈cycloalkyl group, a C₁-C₂₆alkoxy group, in which one or more carbon atoms which are not in neighbourhood to each other could be replaced by -NR⁴⁵-, -O-, -S-, -C(=O)-O-, or-O-C(=O)-O-, and/or wherein one or more hydrogen atoms can be replaced by F, a C₆-C₂₄aryl group, or a C₆-C₂₄aryloxy group, wherein one or more carbon atoms can be replaced by O, S, or N, and/or which can be substituted by one or more non-aromatic groups R⁴¹, or
two or more groups R⁴¹ form a ring system;
X¹ is O, S, or N R⁴³;
R⁴³ is C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -O-; or C₂-C₂₀heteroaryl group;
R¹¹⁹ and R¹²⁰ are independently of each other C₁-C₁₈alkyl, C₁-C₁₈alkyl which is substituted by E and/or interrupted by D, C₆-C₂₄aryl, C₆-C₂₄aryl which is substituted by G, C₂-C₂₀heteroaryl, C₂-C₂₀heteroaryl which is substituted by G, C₂-C₁₈alkenyl, C₂-C₁₈alkynyl, C₁-C₁₈alkoxy, C₁-C₁₈alkoxy which is substituted by E and/or interrupted by D, or C₇-C₂₅aralkyl, or
R¹¹⁹ and R¹²⁰ together form a group of formula =CR¹²¹R¹²², wherein
R¹²¹ and R¹²² are independently of each other H, C₁-C₁₈alkyl, C₁-C₁₈alkyl which is substituted by E and/or interrupted by D, C₆-C₂₄aryl, C₆-C₂₄aryl which is substituted by G, or C₂-C₂₀heteroaryl, or C₂-C₂₀heteroaryl which is substituted by G, or
R¹¹⁹ and R¹²⁰ together form a five or six membered ring, which optionally can be substituted by C₁-C₁₈alkyl, C₁-C₁₈alkyl which is substituted by E and/or interrupted by D, C₆-C₂₄aryl, C₆-C₂₄aryl which is substituted by G, C₂-C₂₀heteroaryl, C₂-C₂₀heteroaryl which is substituted by G, C₂-C₁₈alkenyl, C₂-C₁₈alkynyl, C₁-C₁₈alkoxy, C₁-C₁₈alkoxy which is substituted by E
and/or interrupted by D, C₇-C₂₅aralkyl, or-C(=O)-R²⁸,
R²⁸ is H; C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -O-,
D is -CO-, -COO-, -S-, -SO-, -SO₂-, -O-, -NR⁶⁵-, -SiR⁷OR⁷¹-, -POR⁷²-, -CR⁶³=CR⁶⁴-, or-C≡C-, and
E is -OR⁶⁹, -SR⁶⁹, -NR⁶⁵R⁶⁶, -COR⁶⁸, -COOR⁶⁷, -CONR65R66, -CN, or halogen,
G is E, or C₁-C₁₈alkyl,
R63 and R⁶⁴ are independently of each other C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -O-;
R⁶⁵ and R⁶⁶ are independently of each other C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -O-; or
R65 and R⁶⁶ together form a five or six membered ring,
R⁶⁷ is C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -O-,
R⁶⁸ is H; C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -O-,
R⁶⁹ is C₆-C₁₈aryl; C₆-C₁₈aryl, which is substituted by C₁-C₁₈alkyl, or C₁-C₁₈alkoxy; C₁-C₁₈alkyl; or C₁-C₁₈alkyl which is interrupted by -O-,
R⁷⁰ and R⁷¹ are independently of each other C₁-C₁₈alkyl, C₆-C₁₈aryl, or C₆-C₁₈aryl, which is substituted by C₁-C₁₈alkyl, and
R⁷² is C₁-C₁₈alkyl, C₆-C₁₈aryl, or C₆-C₁₈aryl, which is substituted by C₁-C₁₈alkyl;
R⁴⁵ and R^{45'} are independently of each other a C₁-C₂₅alkyl group, a C₄-C₁₈cycloalkyl group, in which one or more carbon atoms which are not in neighbourhood to each other could be replaced by -NR^{45"}-, -O-, -S-, -C(=O)-O-, or, -O-C(=O)-O-, and/or wherein one or more hydrogen atoms can be replaced by F, a C₆-C₂₄aryl group, or a C₆-C₂₄aryloxy group, wherein one or more carbon atoms can be replaced by O, S, or N, and/or which can be substituted by one or more non-aromatic groups R⁴¹,
R^{45"} is a C₁-C₂₅alkyl group, or a C₄-C₁₈cycloalkyl group, and
m1 can be the same or different at each occurence and is 0, 1, 2, 3, or 4, especially 0, 1, or 2, very especially 0 or 1.

Among the groups of formula 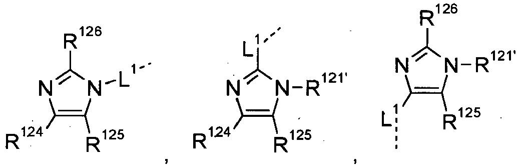 and 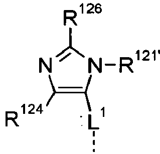 the groups of formula 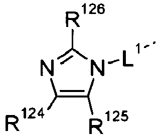 and 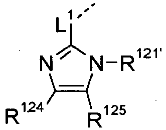 are preferred.

Compounds of formula 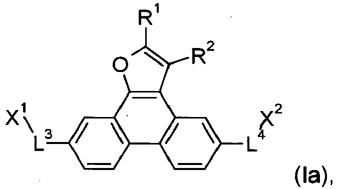 or 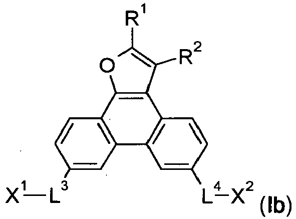 are preferred wherein X¹, X², L³, L⁴, R¹ and R² are as defined above.

Preferably, R¹ and R² are a group of formula 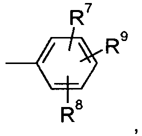 or 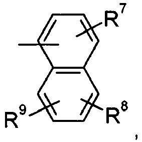 wherein R⁷, R⁸ and R⁹ are independently of each other H, C₁-C₁₈alkyl, C₁-C₁₈alkoxy, or C₁-C₁₈alkyl which is interrupted by O; or R¹ and R² form together a group 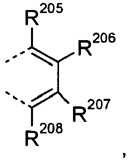 wherein R²⁰⁵, R²⁰⁶, R²⁰⁷, and R²⁰⁸ are independently of each other H, C₁-C₁₈alkyl, C₁-C₁₈alkyl which is interrupted by O, C₁-C₁₈alkoxy, or C₁-C₁₈alkoxy which is interrupted by O, or C₁-C₁₈fluoroalkyl.

L¹, L³ and L⁴ are independently of each other a single bond, or a bridging unit BU. Examples of the bridging unit BU are groups of formula 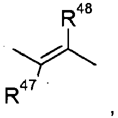 especially 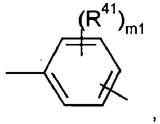 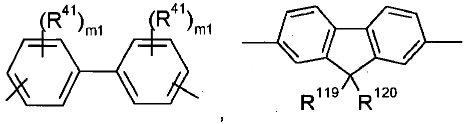 or 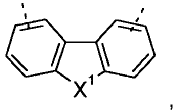 wherein m¹, X¹, R⁴¹, R¹¹⁹ and R¹²⁰ are as defined above and R⁴⁷ and R⁴⁸ are independently of each other H, C₁-C₁₈alkyl, or C₆-C₁₀aryl, which may optionally be substituted by one, or more C₁-C₈alkyl, or C₁-C₈alkoxy groups.

Preferably, R¹¹⁹ and R¹²⁰ are independently of each other C₁-C₁₂alkyl, such as methyl, ethyl, n-propyl, iso-propyl, n-butyl, sec-butyl, hexyl, octyl, or 2-ethyl-hexyl, C₁-C₁₂alkyl which is substituted by E and/or interrupted by D, such as -CH₂(OCH₂CH₂)_{w}OCH₃, w = 1, 2, 3, or 4, C₆-C₁₄aryl, such as phenyl, naphthyl, or biphenylyl, C₆-C₁₄aryl which is substituted by G, such as -C₆H₄OCH₃, -C₆H₄OCH₂CH₃, -C₆H₃(OCH₃)₂, -C₆H₃(OCH₂CH₃)₂, -C₆H₄CH₃, -C₆H₃(CH₃)₂, -C₆H₂(CH₃)₃, or -C₆H₄tBu, or R¹¹⁹ and R¹²⁰ together form a 4 to 8 membered ring, especially a 5 or 6 membered ring, such as cyclohexyl, or cyclopentyl, which can optionally be substituted by C₁-C₈alkyl.

Preferably, m1 is 0, or 1.

Preferably, R⁴¹ is C₁-C₁₂alkyl, C₁-C₁₂alkyl which is substituted interrupted by O, or C₁-C₁₂alkoxy.

Preferably, R⁴⁷ and R⁴⁸ are independently of each other H, or C₁-C₄alkyl.

Most preferred L¹, L³ and L⁴ are a single bond, or a group 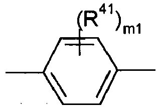 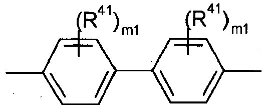 or 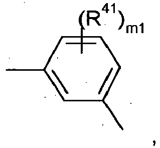 wherein m1 is 0, or 1, and R⁴¹ is a C₁-C₂₅alkyl group.
- In a preferred embodiment of the present invention -L³-X¹ and -L⁴-X² are independently of each other a group of formula
- NA¹A^{1'}, or a group 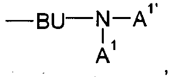 wherein
   A¹ and A^{1'} are independently of each other 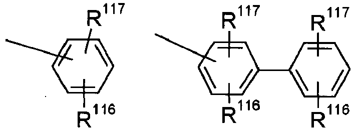 or 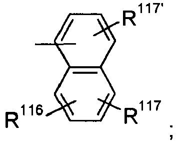 or
   A¹ and A^{1'} together with the nitrogen atom to which they are bonded form a heteroaromatic ring, or ring system 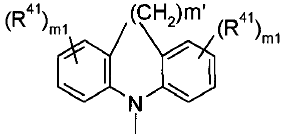 or 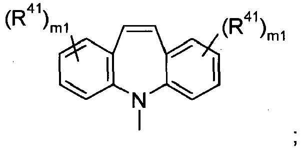 m' is 0, 1, or 2;
   m1 can be the same or different at each occurence and is 0, 1, 2, 3, or 4, especially 0, 1, or 2, very especially 0 or 1;
   R¹¹⁶ R¹¹⁷ and R¹¹⁷ are independently of each other H, halogen, especially F, -CN, C₁-C₁₈alkyl, C₁-C₁₈alkyl which is substituted by E and/or interrupted by D, C₆-C₂₄aryl, C₆-C₂₄aryl which is substituted by G, C₂-C₂₀heteroaryl, C₂-C₂₀heteroaryl which is substituted by G, C₂-C₁₈alkenyl, C₂-C₁₈alkynyl, C₁-C₁₈alkoxy, C₁-C₁₈alkoxy which is substituted by E and/or interrupted by D, C₇-C₂₅aralkyl, -C(=O)-R⁷⁷, -C(=O)OR⁷⁸, or-C(=O)NR⁷⁵R⁷⁶, or substituents R¹¹⁶, R¹¹⁷ and R^{117'}, which are adjacent to each other, can form a ring,
   R⁷⁵, R⁷⁶ and R⁷⁸ are independently of each other H; C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -O-,
   R⁷⁷ is C₆-C₁₈aryl; C₆-C₁₈aryl which is substituted by C₁-C₁₈alkyl, or C₁-C₁₈ alkoxy; C₁-C₁₈alkyl; or C₁-C₁₈alkyl which is interrupted by -O-,
   BU is 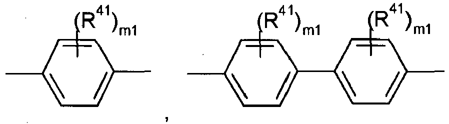 or 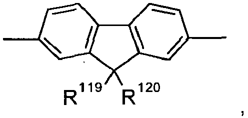 wherein R¹¹⁹ and R¹²⁰, ml and R⁴¹ is as defined above and ml is as defined above.

In said embodiment -L³-X¹ and -L⁴-X² are more preferably independently of each other a group of formula -NA¹A^{1'}, or a group 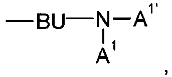 wherein
A¹ and A¹' are independently of each other 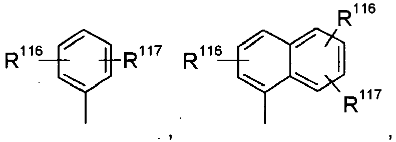 or 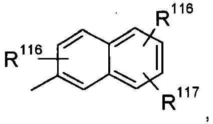 or A¹ and A^{1'} together with the nitrogen atom to which they are bonded form a group of formula 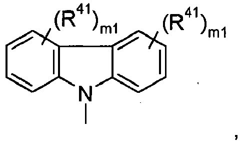 R¹¹⁶ and R¹¹⁷ are independently of each other C₁-C₁₈alkyl, which may optionally be interrupted by -O-, or C₁-C₁₈alkoxy;
Bu is 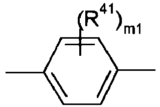 or 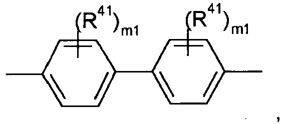 wherein R⁴¹ can be the same or different at each occurence and is C₁-C₂₅alkyl, which may optionally be interrupted by -O-, or C₁-C₂₅alkoxy; m1 is 0, 1, or 2.

In a further preferred embodiment of the present invention -L³-X¹ and -L⁴-X² are independently of each other a C₁₀-C₂₈aryl group, especially a group of formula 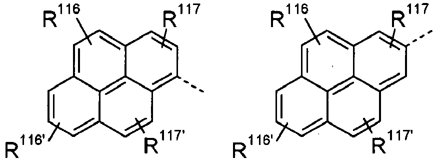 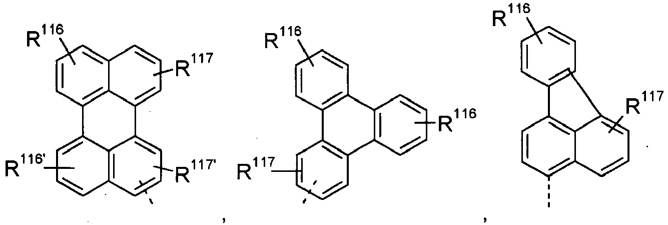 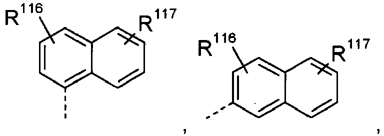 or 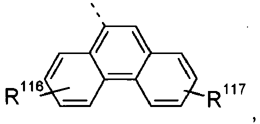 wherein
R^{116'} has the meaning of R¹¹⁶, R¹¹⁶, R¹¹⁷ and R^{117'} are as defined above, D, E and G are as defined above.

Preferably, R¹¹⁶, R^{116'}, R¹¹⁷ and R^{117'} are independently of each other H, C₁-C₁₂alkyl, such as methyl, ethyl, n-propyl, iso-propyl, n-butyl, isobutyl, sec-butyl, t-butyl, 2-methylbutyl, n-pentyl, isopentyl, n-hexyl, 2-ethylhexyl, or n-heptyl, C₁-C₁₂alkyl which is interrupted by O, such as -CH₂OCH₃, -CH₂OCH₂CH₃, -CH₂OCH₂CH₂OCH₃, or -CH₂OCH₂CH₂OCH₂CH₃; or C₁-C₁₂alkoxy.

In said embodiment -L³-X¹ and -L⁴-X² are more preferably independently of each other a group of formula 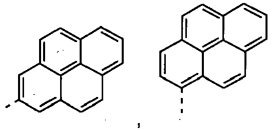 or

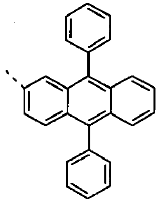

In a further preferred embodiment of the present invention -L³-X¹ and -L⁴-X² are independently of each other a group of formula 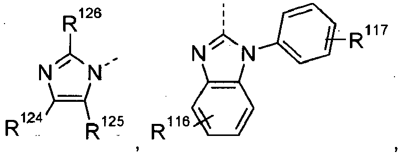 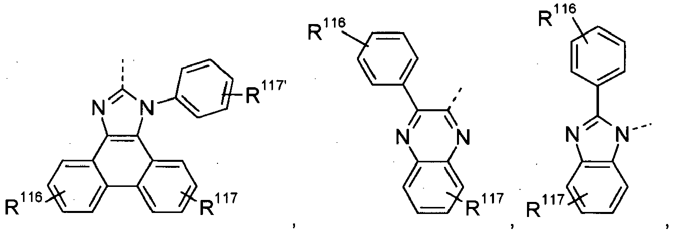 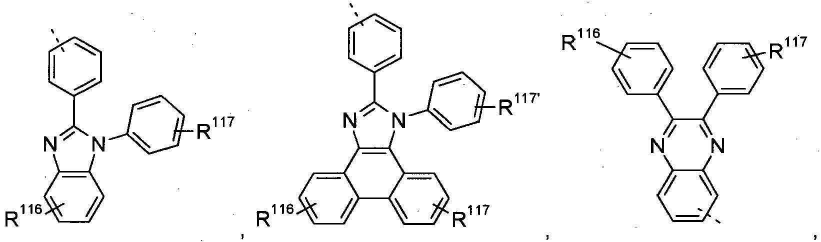 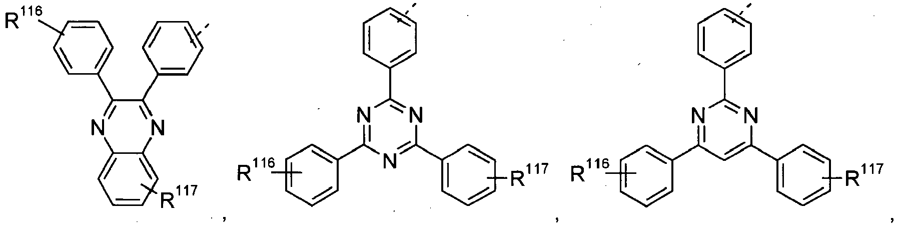 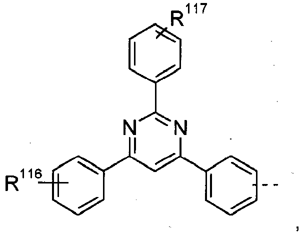 or 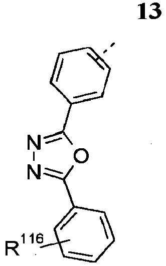 wherein R¹¹⁶, R¹¹⁷, R^{117'}, R¹²⁴, R¹²⁵ and R¹²⁶ are as defined above. In a further preferred embodiment of the present invention -L³-X¹ and -L⁴-X² are a group of formula 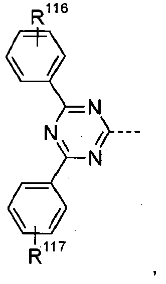 wherein wherein R¹¹⁶ and R¹¹⁷ are as defined above.

Preferably, R¹¹⁶, R^{116'}, R¹¹⁷ and R^{117'} are independently of each other H, C₁-C₁₂alkyl, such as methyl, ethyl, n-propyl, iso-propyl, n-butyl, isobutyl, sec-butyl, t-butyl, 2-methylbutyl, n-pentyl, isopentyl, n-hexyl, 2-ethylhexyl, or n-heptyl, C₁-C₁₂alkyl which is interrupted by O, such as -CH₂OCH₃, -CH₂OCH₂CH₃, -CH₂OCH₂CH₂OCH₃, or -CH₂OCH₂CH₂OCH₂CH₃; or C₁-C₁₂alkoxy.

In said embodiment -L³-X¹ and -L⁴-X² are more preferably independently of each other a group of formula 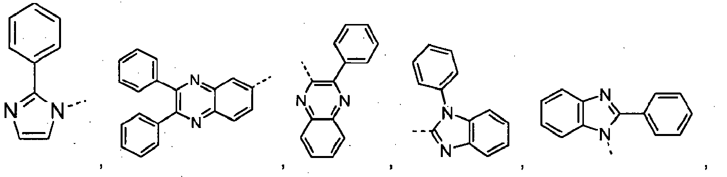 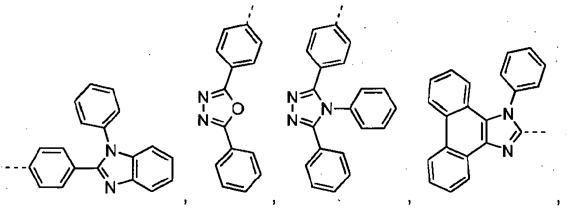 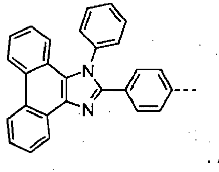 Alternatively -L³-X¹ and -L⁴-X² are a group of formula

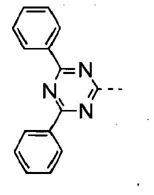

In a particularly preferred embodiment the preent invention is directed to compounds of formula 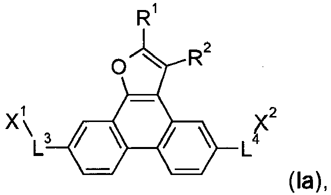 or 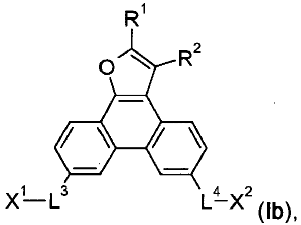 wherein R¹ and
R² are a group of formula 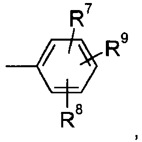 or 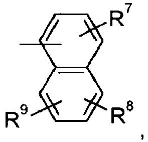 wherein R⁷, R⁸ and R⁹ are independently of each other H, C₁-C₁₈alkyl, C₁-C₁₈alkoxy, or C₁-C₁₈alkyl which is interrupted by O; or R¹ and R² form together a group , -L³-X¹ and -L⁴-X² are independently of each other a group of formula 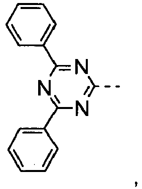 especially 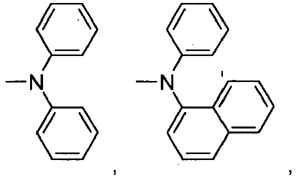 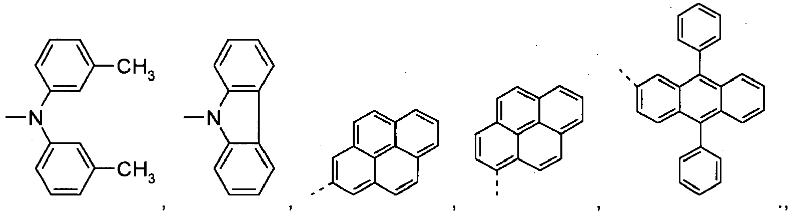 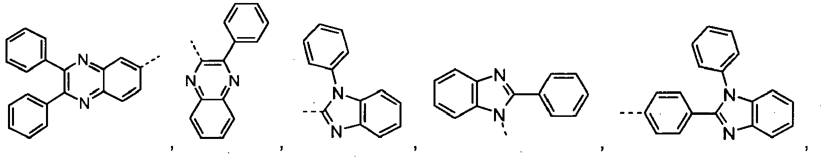 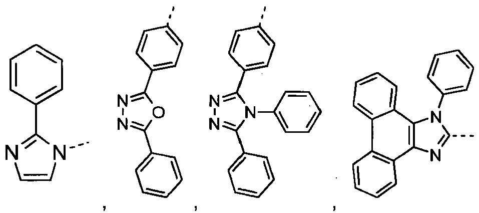 or

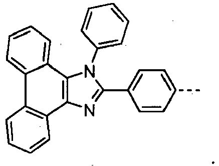

Compounds A-1 to A-9, B-1 to B-9, C-1 to C-6, D-1 to D-6, E-1 to E-18 and F-1 to F-18 are particularly preferred. In a further embodiment compounds E-19, E-20, F-19 and F-20 are preferred. Reference is made to claim 8.

The process for the preparation of compounds of the formula I, wherein X¹ and X² are independently of each other -NA¹A^{1'}, 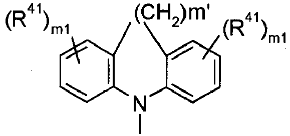 or 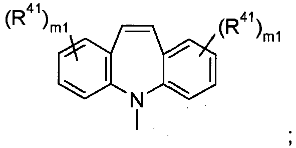 m' is 0, 1, or 2; comprises reacting a compound of formula 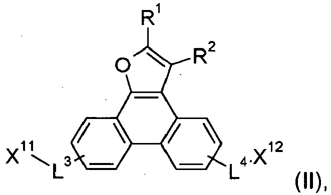 wherein X¹¹ and X¹² stand for halogen, such as bromo, or iodo, with a compound of formula HNA¹A^{1'}, 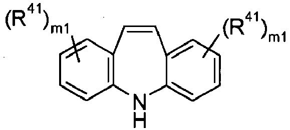 or 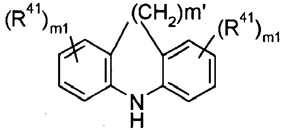 in the presence of a base, such as, for example, sodium hydride, potassium carbonate, or sodium carbonate, and a catalyst, such as, for example, copper (0) or copper (I) (such as copper, copper-bronze, copper bromide iodide, or copper bromide), in a solvent, such as, for example, toluene, dimethyl formamide, or dimethyl sulfoxide, wherein A¹, A^{1'}, L3, L⁴, R¹, R², R⁴¹ and m1 are as defined above.
This reaction, referred to as an Ullmann condensation, is described by Yamamoto & Kurata, Chem. and Industry, 737-738 (1981), J. Mater. Chem. 14 (2004) 2516, H. B. Good-brand et al., J. Org. Chem. 64 (1999) 670 and K. D. Belfield et al., J. Org. Chem. 65 (2000) 4475 using copper as catalyst. Additionally palladium catalysts can be used for the coupling of aryl halogen compounds with amines, as described in M. D. Charles et al., Organic Lett. 7 (2005) 3965, A. F. Littke et. al., Angew. Chem. Int. Ed. 41 (2002) 4176 and literature cited therein.

The compounds of formula I of the present invention can be prepared according to a process, which comprises reacting a derivative of formula 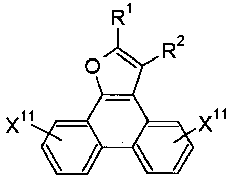 with boronic acid derivative X¹²-Ar; or reacting a derivative of formula 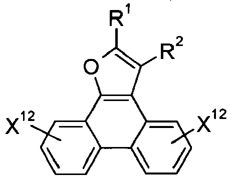 with X¹¹-Ar; wherein X¹¹ stands for halogen such as chloro or bromo, or iodo, preferably bromo, or iodo, most preferably bromo, X¹² having the meaning of -B(OH)₂, -B(OY¹)₂, 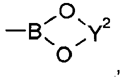 -BF₄Na, or -BF₄K, Ar is a group of formula 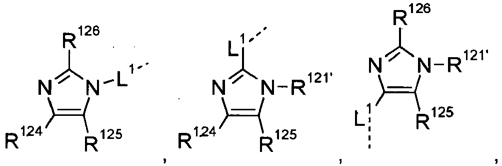 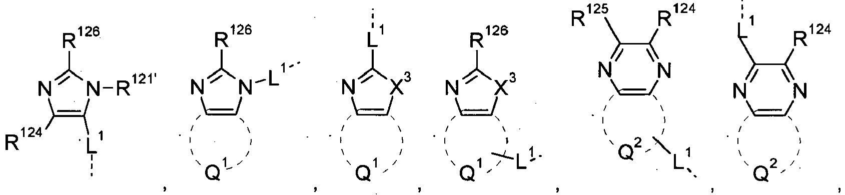 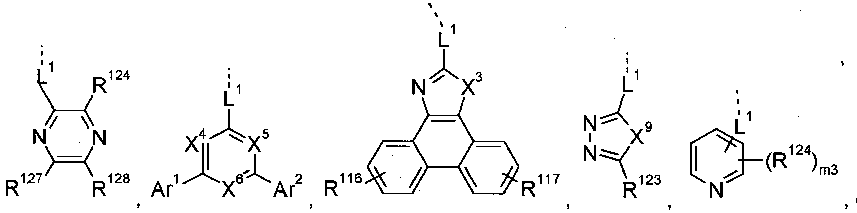 or 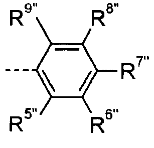 (= group II); or a C₁₀-C₂₈aryl group (= group III), in the presence of an allylpalladium catalyst of the µ-halo(triisopropylphosphine)(η³-allyl)palladium(II) type (see for example WO99/47474).

Preferably, the reaction is carried out in the presence of an organic solvent, such as an aromatic hydrocarbon or a usual polar organic solvent, such as benzene, toluene, xylene, tetrahydrofurane, or dioxane, or mixtures thereof, most preferred toluene. Usually, the amount of the solvent is chosen in the range of from 1 to 10 I per mol of boronic acid derivative. Also preferred, the reaction is carried out under an inert atmosphere such as nitrogen, or argon. Further, it is preferred to carry out the reaction in the presence of an aqueous base, such as an alkali metal hydroxide or carbonate such as NaOH, KOH, Na₂CO₃, K₂CO₃, Cs₂CO₃ and the like, preferably an aqueous K₂CO₃ solution is chosen. Usually, the molar ratio of the base to boronic acid or boronic ester derivative is chosen in the range of from 0.5:1 to 50:1, very especially 1:1. Generally, the reaction temperature is chosen in the range of from 40 to 180°C, preferably under reflux conditions. Preferred, the reaction time is chosen in the range of from 1 to 80 hours, more preferably from 20 to 72 hours. In a preferred embodiment a usual catalyst for coupling reactions or for polycondensation reactions is used, preferably Pd-based, which is described in WO2007/101820. The palladium compound is added in a ratio of from 1:10000 to 1:50, preferably from 1:5000 to 1:200, based on the number of bonds to be closed. Preference is given, for example, to the use of palladium(II) salts such as PdAc₂ or Pd₂dba₃ and to the addition of ligands selected from the group consisting of 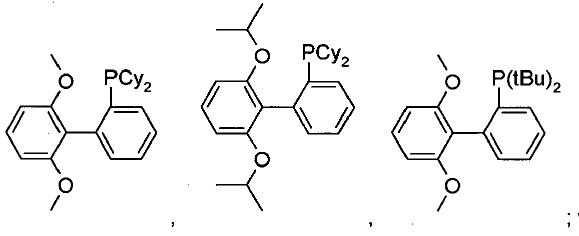 wherein 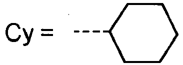 The ligand is added in a ratio of from 1:1 to 1:10, based on Pd. Also preferred, the catalyst is added as in solution or suspension. Preferably, an appropriate organic solvent such as the ones described above, preferably benzene, toluene, xylene, THF, dioxane, more preferably toluene, or mixtures thereof, is used. The amount of solvent usually is chosen in the range of from 1 to 10 l per mol of boronic acid derivative. Compounds of formula I, wherein -L³-X¹ and -L⁴-X² are identical and are a group of formula 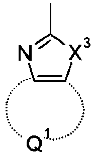 can be prepared according to, or in analogy to Synthesis 2005, 47 or Synthesis; 2003, 1683. An example of such a reaction is shown below:

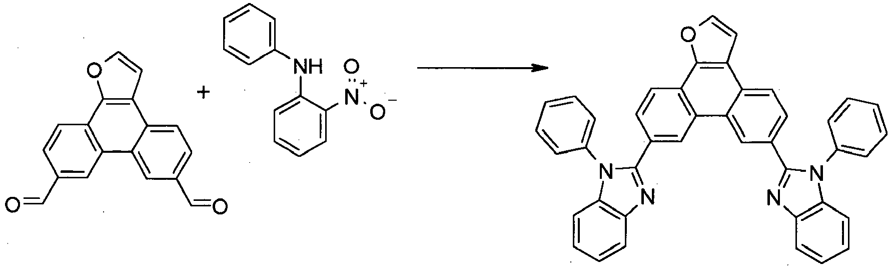

Compounds of formula I, wherein -L³-X¹ and -L⁴-X² are identical and are a group of formula 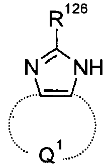 can be prepared (Ulmann reaction) according, or in analogy to Inorg. Chem. 2006, 45, 147, or Inorg. Chem. 2005, 44, 1232. 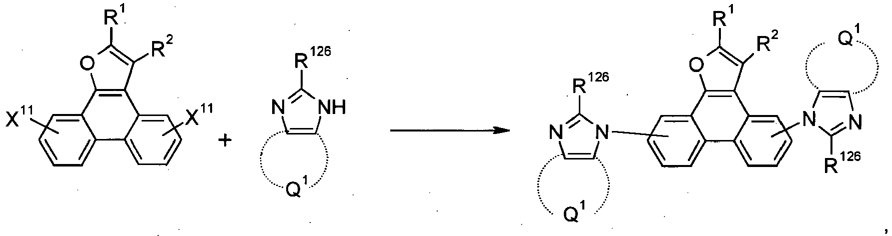 wherein X¹¹ stands for bromo, or iodo, preferably iodo. Compounds of formula I, wherein -L³-X¹ and -L⁴-X² are identical and are a group of formula 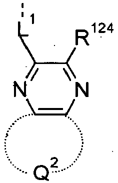 can be prepared according to, or in analogy to Adv. Funkt. Mater. 2006, 16, 1449. An example of such a reaction is shown below:

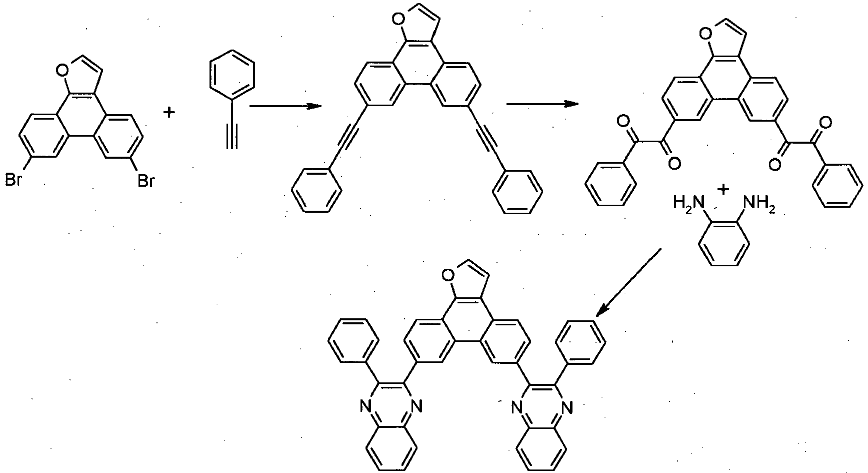

Compounds of the formula 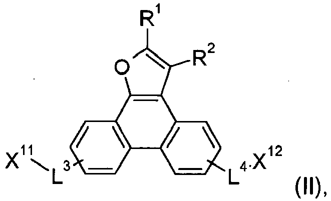 wherein L3, L⁴, R¹ and R² are as defined in claim 1; X¹¹ and X¹² are independently in each occurrence a halogen atom, or -OS(O)₂CF₃, - OS(O)₂-aryl, especially 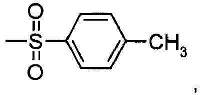 -OS(O)₂CH₃, -B(OH)₂, -B(OY¹)₂, 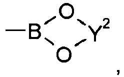 -BF₄Na, or -BF₄K, wherein Y¹ is independently in each occurrence a C₁-C₁₀alkyl group and Y² is independently in each occurrence a C₂-C₁₀alkylene group, such as -CY³Y⁴-CY⁵Y⁶-, or -CY⁷Y⁸-CY⁹Y¹⁰- CY¹¹Y¹²-, wherein Y³, Y⁴, Y⁵, Y⁶, Y⁷, Y⁸, Y⁹, Y¹⁰, Y¹¹ and Y¹² are independently of each other hydrogen, or a C₁-C₁₀alkyl group, especially -C(CH₃)₂C(CH₃)₂-, -CH₂C(CH₃)₂CH₂-, or -C(CH₃)₂CH₂C(CH₃)₂-, are intermediates in the production of the compounds of formula I and form a further subject of the present invention.

The preparation of phenanthro[9,10-b]furanes can be carried out as described in J. Chem. Soc. Perkin Trans. 1989, 2329; Tetrahedron Letters 1969, 457; J. Chem. Soc. Perkin Trans. 1991, 3159 and J. Chem. Soc. Perkin Trans. 1990, 2127. 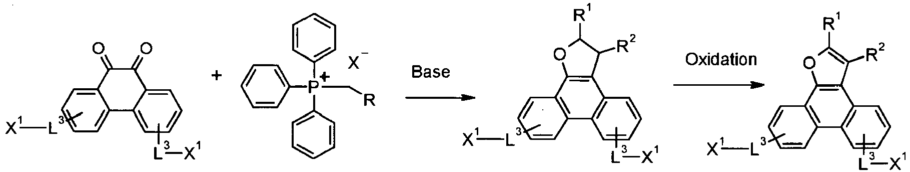 R has the meaning of R.
Tetrahedron 2010, 66, 5612 describes the synthesis phenanthro[9,10-b]furanes, in which R¹ and R² forms a ring.

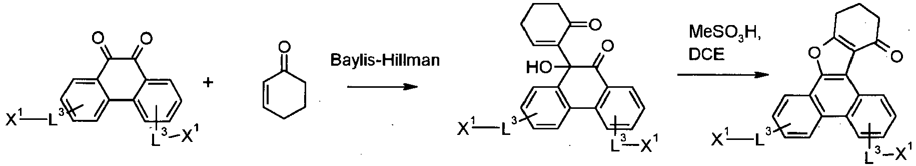

Aromatization of the ring can be achieved according to Synthetic Communications 2006, 36, 1983-1990, or Synthesis 2003, 13, 1977-1988.

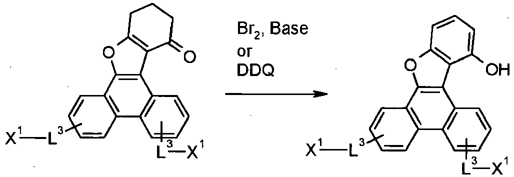

The hydroxy group can be removed in analogy to the process described in example 4 and 5 of WO2006000544.

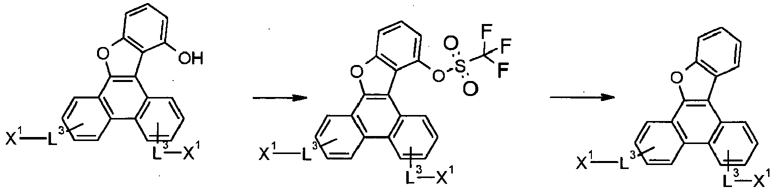

The compounds of of the present invention may be used for electrophotographic photoreceptors, photoelectric converters, organic solar cells (organic photovoltaics), switching elements, such as organic transistors, for example, organic FETs and organic TFTs, organic light emitting field effect transistors (OLEFETs), image sensors, dye lasers and electroluminescent devices (=organic light-emitting diodes (OLEDs)).

Accordingly, a further subject of the present invention is directed to an electronic device, comprising a compound according to the present invention.

The electronic device is preferably an electroluminescent device.

Group I substituted compounds of formula I, such as, for example, **A-1** to **A-9** and **B-1** to **B-9,** can be used as hole transport materials and/or hosts.

Group II substituted compounds of formula I, such as, for example, **C-1** to **C-6** and **D-1** to **D-6,** can be used as electron transport materials.

Group III substituted compounds of formula I, such as, for example, **E-1** to **E-20** and **F-1** to **F-20,** can be used as electron transport materials.

Hence, a further subject of the present invention is directed to a hole transport layer, or an electron transport layer, comprising a compound of the present invention.

C₁-C₂₅alkyl (C₁-C₁₈alkyl) is typically linear or branched, where possible. Examples are methyl, ethyl, n-propyl, isopropyl, n-butyl, sec.-butyl, isobutyl, tert.-butyl, n-pentyl, 2-pentyl, 3-pentyl, 2,2-dimethylpropyl, 1,1,3,3-tetramethylpentyl, n-hexyl, 1-methylhexyl, 1,1,3,3,5,5-hexamethylhexyl, n-heptyl, isoheptyl, 1,1,3,3-tetramethylbutyl, 1-methylheptyl, 3-methylheptyl, n-octyl, 1,1,3,3-tetramethylbutyl and 2-ethylhexyl, n-nonyl, decyl, undecyl, dodecyl, tridecyl, tetradecyl, pentadecyl, hexadecyl, heptadecyl, or octadecyl. C₁-C₈alkyl is typically methyl, ethyl, n-propyl, isopropyl, n-butyl, sec.-butyl, isobutyl, tert.-butyl, n-pentyl, 2-pentyl, 3-pentyl, 2,2-dimethyl-propyl, n-hexyl, n-heptyl, n-octyl, 1,1,3,3-tetramethylbutyl and 2-ethylhexyl. C₁-C₄alkyl is typically methyl, ethyl, n-propyl, isopropyl, n-butyl, sec.-butyl, iso-butyl, tert.-butyl.

C₂-C₂₅alkenyl groups (C₂-C₁₈alkenyl groups) are straight-chain or branched alkenyl groups, such as e.g. vinyl, allyl, methallyl, isopropenyl, 2-butenyl, 3-butenyl, isobutenyl, n-penta-2,4-dienyl, 3-methyl-but-2-enyl, n-oct-2-enyl, n-dodec-2-enyl, isododecenyl, n-dodec-2-enyl or n-octadec-4-enyl.

C₂₋₂₄alkynyl (C₂₋₁₈alkynyl) is straight-chain or branched and preferably C₂₋₈alkynyl, which may be unsubstituted or substituted, such as, for example, ethynyl, 1-propyn-3-yl, 1-butyn-4-yl, 1-pentyn-5-yl, 2-methyl-3-butyn-2-yl, 1,4-pentadiyn-3-yl, 1,3-pentadiyn-5-yl, 1-hexyn-6-yl, cis-3-methyl-2-penten-4-yn-1-yl, trans-3-methyl-2-penten-4-yn-1-yl, 1,3-hexadiyn-5-yl, 1-octyn-8-yl, 1-nonyn-9-yl, 1-decyn-10-yl, or 1-tetracosyn-24-yl.

C₁-C₂₅alkoxy groups (C₁-C₁₈alkoxy groups) are straight-chain or branched alkoxy groups, e.g. methoxy, ethoxy, n-propoxy, isopropoxy, n-butoxy, sec-butoxy, tert-butoxy, amyloxy, isoamyloxy or tert-amyloxy, heptyloxy, octyloxy, isooctyloxy, nonyloxy, decyloxy, undecyloxy, dodecyloxy, tetradecyloxy, pentadecyloxy, hexadecyloxy, heptadecyloxy and octadecyloxy. Examples of C₁-C₈alkoxy are methoxy, ethoxy, n-propoxy, isopropoxy, n-butoxy, sec.-butoxy, isobutoxy, tert.-butoxy, n-pentyloxy, 2-pentyloxy, 3-pentyloxy, 2,2-dimethylpropoxy, n-hexyloxy, n-heptyloxy, n-octyloxy, 1,1,3,3-tetramethylbutoxy and 2-ethylhexyloxy, preferably C₁-C₄alkoxy such as typically methoxy, ethoxy, n-propoxy, isopropoxy, n-butoxy, sec.-butoxy, isobutoxy, tert.-butoxy.

The term "cycloalkyl group" is typically C₄-C₁₈cycloalkyl, especially C₅-C₁₂cycloalkyl, such as cyclopentyl, cyclohexyl, cycloheptyl, cyclooctyl, cyclononyl, cyclodecyl, cycloundecyl, cyclododecyl, preferably cyclopentyl, cyclohexyl, cycloheptyl, or cyclooctyl, which may be unsubstituted or substituted.

C₁-C₁₈fluoroalkyl, especially C₁-C₄fluoroalkyl, is a branched or unbranched radical, wherein all, or part of the hydrogen atoms are replaced by F, such as for example -CF₃, -CF₂CF₃, -CF₂CF₂CF₃, -CF(CF₃)₂, -(CF₂)₃CF₃, and -C(CF₃)₃.

C₆-C₂₄aryl, which optionally can be substituted, is typically phenyl, 4-methylphenyl, 4-methoxyphenyl, naphthyl, especially 1-naphthyl, or 2-naphthyl, biphenylyl, terphenylyl, pyrenyl, 2- or 9-fluorenyl, phenanthryl, or anthryl, which may be unsubstituted or substituted.

C₂-C₂₀heteroaryl represents a ring with five to seven ring atoms or a condensed ring system, wherein nitrogen, oxygen or sulfur are the possible hetero atoms, and is typically a heterocyclic group with five to 30 atoms having at least six conjugated π-electrons such as thienyl, benzothiophenyl, dibenzothiophenyl, thianthrenyl, furyl, furfuryl, 2H-pyranyl, benzofuranyl, isobenzofuranyl, dibenzofuranyl, phenoxythienyl, pyrrolyl, imidazolyl, pyrazolyl, pyridyl, bipyridyl, triazinyl, pyrifnidinyl, pyrazinyl, pyridazinyl, indolizinyl, isoindolyl, indolyl, indazolyl, purinyl, quinolizinyl, chinolyl, isochinolyl, phthalazinyl, naphthyridinyl, chinoxalinyl, chinazolinyl, cinnolinyl, pteridinyl, carbazolyl, carbolinyl, benzotriazolyl, benzoxazolyl, phenanthridinyl, acridinyl, pyrimidinyl, phenanthrolinyl, phenazinyl, isothiazolyl, phenothiazinyl, isoxazolyl, furazanyl or phenoxazinyl, which can be unsubstituted or substituted.

The C₆-C₂₄aryl and C₂-C₂₀heteroaryl groups are preferably substituted by one, or more C₁-C₈alkyl groups.

The term "aryl ether group" is typically a C₆₋₂₄aryloxy group, that is to say O-C₆₋₂₄aryl, such as, for example, phenoxy or 4-methoxyphenyl.

The term "aralkyl group" is typically C₇-C₂₄aralkyl, such as benzyl, 2-benzyl-2-propyl, β-phenyl-ethyl, α,α,-dimethylbenzyl, ω-phenyl-butyl, ω,ω-dimethyl-ω-phenyl-butyl, ω-phenyl-dodecyl, ω-phenyl-octadecyl, ω-phenyl-eicosyl or ω-phenyl-docosyl, preferably C₇-C₁₈aralkyl such as benzyl, 2-benzyl-2-propyl, β-phenyl-ethyl, α,α-dimethylbenzyl, ω-phenyl-butyl, ω,ω-dimethyl-ω-phenyl-butyl, ω-phenyl-dodecyl or ω-phenyl-octadecyl, and particularly preferred C₇-C₁₂aralkyl such as benzyl, 2-benzyl-2-propyl, β-phenyl-ethyl, α,α-dimethylbenzyl, ω-phenyl-butyl, or ω,ω-dimethyl-ω-phenyl-butyl, in which both the aliphatic hydrocarbon group and aromatic hydrocarbon group may be unsubstituted or substituted.

Examples of arylene radicals are phenylene, naphthylene, phenalenylene, antracylene and phenantrylene, which may optionally be substituted by one or more C₁-C₁₈alkyl groups. Preferred arylene radicals are 1,3-phenylene, 3,6-naphthylene, and 4,9-phenalenylene, which may optionally be substituted by one or more C₁-C₁₈alkyl groups.

Examples of heteroarylene radicals are 1,3,4-thiadiazol-2,5-ylene, 1,3-thiazol-2,4-ylene, 1,3-thiazol-2,5-ylene, 2,4-thiophenylene, 2,5-thiophenylene, 1,3-oxazol-2,4-ylene, 1,3-oxazol-2,5-ylene and 1,3,4-oxadiazol-2,5-ylene, 2,5-indenylene, 2,6-indenylene, especially pyrazinylene, pyridinylene, pyrimidinylene, and triazolylene, which may optionally be substituted by one or more C₁-C₁₈alkyl groups. Preferred heteroarylene radicals are 2,6-pyrazinylene, 2,6-pyridinylene, 4,6-pyrimidinylene, and 2,6-triazolylene, which may optionally be substituted by one or more C₁-C₁₈alkyl groups.

Possible substituents of the above-mentioned groups are C₁-C₈alkyl, a hydroxyl group, a mercapto group, C₁-C₈alkoxy, C₁-C₈alkylthio, halogen, halo-C₁-C₈alkyl, or a cyano group.

The term "haloalkyl" mean groups given by partially or wholly substituting the above-mentioned alkyl group with halogen, such as trifluoromethyl etc.

If a substituent, such as, for example R⁴¹ occurs more than one time in a group, it can be different in each occurrence.

The wording "substituted by G" means that one, or more, especially one to three substituents G might be present.

As described above, the aforementioned groups may be substituted by E and/or, if desired, interrupted by D. Interruptions are of course possible only in the case of groups containing at least 2 carbon atoms connected to one another by single bonds; C₆-C₁₈aryl is not interrupted; interrupted arylalkyl contains the unit D in the alkyl moiety. C₁-C₁₈alkyl substituted by one or more E and/or interrupted by one or more units D is, for example, (CH₂CH₂O)₁₋₉-R^{x}, where R^{x} is H or C₁-C₁₀alkyl or C₂-C₁₀alkanoyl (e.g. CO-CH(C₂H₅)C₄H₉), CH₂-CH(OR^{y'})-CH₂-O-R^{y}, where R^{y} is C₁-C₁₈alkyl, C₅-C₁₂cycloalkyl, phenyl, C₇-C₁₅phenylalkyl, and R^{y'} embraces the same definitions as R^{y} or is H;
C₁-C₈alkylene-COO-R^{z}, e.g. CH₂COOR_{z}, CH(CH₃)COOR^{z}, C(CH₃)₂COOR^{z}, where R^{z} is H, C₁-C₁₈alkyl, (CH₂CH₂O)₁₋₉-R^{x}, and R^{x} embraces the definitions indicated above; CH₂CH₂-O-CO-CH=CH₂; CH₂CH(OH)CH₂-O-CO-C(CH₃)=CH₂.

The organic electronic device of the present application is, for example, an organic solar cell (organic photovoltaics), a switching element. such as an organic transistors, for example organic FET and organic TFT, organic light emitting field effect transistor (OLEFET), or an organic light-emitting diode (OLED), preference being given to OLEDs.

The compounds of formula I are preferably used as electron transport materials, hole transport materials and/or hosts for phosphorescent materials.

In an especially preferred embodiment of the present application relates compounds of formula I are used in combination with a phosphorescent emitter preferably as host in an organic light emitting device.

### Phosphorescent Materials

Phosphorescent materials may be used alone or, in certain cases, in combination with each other, either in the same or different layers. Examples of phosphorescent and related materials are described in WO00/57676, WO00/70655, WO01/41512, WO02/15645, US2003/0017361, WO01/93642, WO01/39234, US6,458,475, WO02/071813, US6,573,651, US2002/0197511, WO02/074015, US6,451,455, US2003/0072964, US2003/0068528, US6,413,656, US6,515,298, US6,451,415, US6,097,147, US2003/0124381, US2003/0059646, US2003/0054198, EP1239526, EP1238981, EP1244155, US2002/0100906, US2003/0068526, US2003/0068535, JP2003073387, JP2003073388, US2003/0141809, US2003/0040627, JP2003059667, JP2003073665, US2002/0121638, WO06/000544, WO07/074093, WO07/115981, WO08/101842, WO09/100991, WO10/129323, WO2010056669, WO10086089, US2010/213834, US2011/089407, and WO11/073149.

The emission wavelengths of cyclometallated Ir(III) complexes of the type IrL₃ and IrL₂L', such as the green-emitting fac-tris(2-phenylpyridinato-N,C^{2'})iridium(III) and bis(2-phenylpyridinato-N,C^{2'})Iridium(III) (acetylacetonate) may be shifted by substitution of electron donating or withdrawing groups at appropriate positions on the cyclometallating ligand L, or by choice of different heterocycles for the cyclometallating ligand L. The emission wavelengths may also be shifted by choice of the ancillary ligand L'. Examples of red emitters are bis(1-(phenyl)isoquinoline) iridium (III) acetylanetonate, (acetylanetonato)bis(2,3,5-triphenylpyrazinato)iridium(III), bis(2-(2'-benzothienyl)pyridinato-N,C^{3'})iridium(III)-(acetylacetonate) and tris(1-phenylisoquinolinato-N,C)iridium(III). A blue-emitting example is bis(2-(4,6-diflourophenyl)-pyridinato-N,C^{2'})Iridium(III)(picolinate).

Red electrophosphorescence has been reported, using bis(2-(2'-benzo[4,5-a]thienyl)pyridinato-N, C³)iridium(acetylacetonate)[Btp₂Ir(acac)] as the phosphorescent material (Adachi, C., Lamansky, S., Baldo, M. A., Kwong, R. C., Thompson, M. E., and Forrest, S. R., App. Phys. Lett., 78, 1622 1624 (2001)).

Other important phosphorescent materials include cyclometallated Pt(II) complexes such as cis-bis(2-phenylpyridinato-N,C^{2'})platinum(II), cis-bis(2-(2'-thienyl)pyridinato-N,C^{3'}) platinum(II), cis-bis(2-(2'-thienyl)quinolinato-N,C^{5'}) platinum(II), or (2-(4,6-diflourophenyl)pyridinato-NC2') platinum(II)acetylacetonate. Pt(II)porphyrin complexes such as 2,3,7,8,12,13,17,18-octaethyl-21H, 23H-porphine platinum(H) are also useful phosphorescent materials.

Still other examples of useful phosphorescent materials include coordination complexes of the trivalent lanthanides such as Th³⁺ and Eu³⁺ (J. Kido et al, Appl. Phys. Lett., 65, 2124 (1994)).

Other important phosphorescent materials are described in WO06/000544 and WO2008/101842.

Examples of phosphorescent materials are compounds A-1 to A-234, B-1 to B-234, C-1 to C-44 and D-1 to D-234, which are described in WO2008/101842. Preferred examples are described in WO2009/100991 example 48-53 and 54-78.

Suitable structures of organic electronic devices are known to those skilled in the art and are specified below.

The organic transistor generally includes a semiconductor layer formed from an organic layer with hole transport capacity and/or electron transport capacity; a gate electrode formed from a conductive layer; and an insulating layer introduced between the semiconductor layer and the conductive layer. A source electrode and a drain electrode are mounted on this arrangement in order thus to produce the transistor element. In addition, further layers known to those skilled in the art may be present in the organic transistor.

The organic solar cell (photoelectric conversion element) generally comprises an organic layer present between two plate-type electrodes arranged in parallel. The organic layer may be configured on a comb-type electrode. There is no particular restriction regarding the site of the organic layer and there is no particular restriction regarding the material of the electrodes. When, however, plate-type electrodes arranged in parallel are used, at least one electrode is preferably formed from a transparent electrode, for example an ITO electrode or a fluorine-doped tin oxide electrode. The organic layer is formed from two sublayers, i.e. a layer with p-type semiconductor properties or hole transport capacity, and a layer formed with n-type semiconductor properties or electron transport capacity. In addition, it is possible for further layers known to those skilled in the art to be present in the organic solar cell.

The present invention further relates to an organic light-emitting diode comprising an anode An and a cathode Ka, a light-emitting layer E arranged between the anode An and the cathode Ka, an electron transport layer arranged between the cathode Ka and the light-emitting layer E, and if appropriate at least one further layer selected from the group consisting of at least one blocking layer for holes/excitons, at least one blocking layer for electrons/excitons, at least one hole injection layer and at least one hole transport layer.

### Structure of the inventive OLED

The inventive organic light-emitting diode (OLED) thus generally has the following structure:
an anode (An) and a cathode (Ka) and a light-emitting layer E arranged between the anode (An) and the cathode (Ka) and an electron transport layer arranged between the cathode Ka and the light-emitting layer E.

The inventive OLED may, for example - in a preferred embodiment - be formed from the following layers:
1. Anode
2. Hole transport layer
3. Light-emitting layer
4. Blocking layer for holes/excitons
5. Electron transport layer
6. Cathode

Layer sequences different than the aforementioned structure are also possible, and are known to those skilled in the art. For example, it is possible that the OLED does not have all of the layers mentioned; for example, OLEDs which have layers (1), (3), (4), (5) and (6), are likewise suitable. In addition, the OLEDs may have a blocking layer for electrons/excitons between the hole transport layer (2) and the light-emitting layer (3).

It is additionally possible that a plurality of the aforementioned functions (electron/exciton blocker, hole/exciton blocker, hole injection, hole transport, electron injection, electron transport) are combined in one layer and are assumed, for example, by a single material present in this layer. For example, a material used in the hole transport layer, in one embodiment, may simultaneously block excitons and/or electrons.

Furthermore, the individual layers of the OLED among those specified above may in turn be formed from two or more layers. For example, the hole transport layer may be formed from a layer into which holes are injected from the electrode, and a layer which transports the holes away from the hole-injecting layer into the light-emitting layer. The electron transport layer may likewise consist of a plurality of layers, for example a layer in which electrons are injected by the electrode, and a layer which receives electrons from the electron injection layer and transports them into the light-emitting layer. These layers mentioned are each selected according to factors such as energy level, thermal resistance and charge carrier mobility, and also energy difference of the layers specified with the organic layers or the metal electrodes. The person skilled in the art is capable of selecting the structure of the OLEDs such that it is matched optimally to the organic compounds used as emitter substances in accordance with the invention.

In order to obtain particularly efficient OLEDs, for example, the HOMO (highest occupied molecular orbital) of the hole transport layer should be matched to the work function of the anode, and the LUMO (lowest unoccupied molecular orbital) of the electron transport layer should be matched to the work function of the cathode, provided that the aforementioned layers are present in the inventive OLEDs.

The anode (1) is an electrode which provides positive charge carriers. It may be formed, for example, from materials which comprise a metal, a mixture of various metals, a metal alloy, a metal oxide or a mixture of various metal oxides. Alternatively, the anode may be a conductive polymer. Suitable metals comprise metals and alloys of the metals of the main groups, transition metals and of the lanthanoids, especially the metals of groups Ib, IVa, Va and VIa of the periodic table of the elements, and the transition metals of group VIIIa. When the anode is to be transparent, generally mixed metal oxides of groups IIb, IIIb and IVb of the periodic table of the elements (IUPAC version) are used, for example indium tin oxide (ITO). It is likewise possible that the anode (1) comprises an organic material, for example polyaniline, as described, for example, in Nature, Vol. 357, pages 477 to 479 (June 11, 1992). At least either the anode or the cathode should be at least partly transparent in order to be able to emit the light formed. The material used for the anode (1) is preferably ITO.

Suitable hole transport materials for layer (2) of the inventive OLEDs are disclosed, for example, in Kirk-Othmer Encyclopedia of Chemical Technology, 4th edition, Vol. 18, pages 837 to 860, 1996. Both hole-transport molecules and polymers can be used as the hole transport material. Hole-transport molecules typically used are selected from the group consisting of tris[N-(1-naphthyl)-N-(phenylamino)]triphenylamine (1-NaphDATA), 4,4'-bis[N-(1-naphthyl)-N-phenylamino]biphenyl (α-NPD), N,N`-diphenyl-N,N`-bis(3-methylphenyl)-[1,1'-biphenyl]-4,4'-diamine (TPD), 1,1-bis[(di-4-tolylamino)phenyl]cyclohexane (TAPC), N,N'-bis(4-methylphenyl)-N,N'-bis(4-ethylphenyl)-[1,1'-(3,3'-dimethyl)biphenyl]-4,4'-diamine (ETPD), tetrakis(3-methylphenyl)-N,N,N',N'-2,5-phenylenediamine (PDA), α,-phenyl-4-N,N-diphenylaminostyrene (TPS), p-(diethylamino)benzaldehyde diphenylhydrazone (DEH), triphenylamine (TPA), bis[4-(N,N-diethylamino)-2-methylphenyl)(4-methylphenyl)methane (MPMP), 1-phenyl-3-[p-(diethylamino)styryl]-5-[p-(diethylamino)phenyl]pyrazoline (PPR or DEASP), 1, 2-trans-bis(9H-carbazol-9-yl)cyclobutane (DCZB), N,N,N',N'-tetrakis(4-methylphenyl)-(1,1'-biphenyl)-4,4'-diamine (TTB), 4,4',4' '-tris(N,N-diphenylamino)triphenylamine (TDTA), 4,4',4"-tris(N-carbazolyl)triphenylamine (TCTA), N,N'-bis(naphthalen-2-yl)-N,N'-bis(phenyl)benzidine (β-NPB), N,N'-bis(3-methylphenyl)-N, N'-bis(phenyl)-9,9-spirobifluorene (Spiro-TPD), N,N'-bis(naphthalen-1-yl)-N,N'-bis(phenyl)-9,9-spirobifluorene (Spiro-NPB), N,N'-bis(3-methylphenyl)-N,N'-bis(phenyl)-9,9-dimethylfluorene (DMFL-TPD), di[4-(N,N-ditolylamino)phenyl]cyclohexane, N,N'-bis(naphthalen-1-yl)-N,N'-bis(phenyl)-9,9-dimethylfluorene, N,N'-bis(naphthalen-1-yl)-N,N'-bis(phenyl)-2,2-dimethylbenzidine, N,N'-bis(naphthalen-1-yl)-N,N'-bis(phenyl)benzidine, N,N'-bis(3-methylphenyl)-N,N'-bis(phenyl)benzidine, 2,3,5,6-tetrafluoro-7,7,8,8-tetracyanoquinodimethane (F4-TCNQ), 4,4',4"-tris(N-3-methylphenyl-N-phenylamino)triphenylamine, 4,4',4"-tris(N-(2-naphthyl)-N-phenyl-amino)triphenylamine, pyrazino[2,3-f][1,10]phenanthroline-2,3-d carbonitrile (PPDN), N,N,N',N '-tetrakis(4-methoxyphenyl)benzidine (MeO-TPD), 2,7-bis[N,N-bis(4-methoxyphenyl)amino]-9,9-spirobifluorene (MeO-Spiro-TPD), 2,2'-bis[N,N-bis(4-methoxyphenyl)amino]-9,9-spirobifluorene (2,2'-MeO-Spiro-TPD), N,N'-diphenyl-N,N'-di[4-(N,N-ditolylamino)phenyl]benzidine (NTNPB), N,N'-diphenyl-N,N'-di[4-(N,N-diphenylamino)phenyl]benzidine (NPNPB), N,N'-di(naphthalen-2-yl)-N,N'-diphenylbenzene-1,4-diamine (β-NPP), N,N'-bis(3-methylphenyl)-N,N'-bis(phenyl)-9,9-diphenylfluorene (DPFL-TPD), N,N'-bis(naphthalen-1-yl)-N,N'-bis(phenyl)-9,9-diphenylfluorene (DPFL-NPB), 2,2',7,7'-tetrakis(N,N-diphenylamino)-9,9'-spirobifluorene (Spiro-TAD), 9,9-bis[4-(N,N-bis(biphenyl-4-yl)amino)phenyl]-9H-fluorene (BPAPF), 9,9-bis[4-(N,N-bis(naphthalen-2-yl)amino)phenyl]-9H-fluorene (NPAPF), 9,9-bis[4-(N,N-bis(naphthalen-2-yl)-N,N'-bisphenylamino)phenyl]-9H-fluorene (N PBAPF), 2,2',7,7'-tetrakis[N-naphthalenyl(phenyl)amino]-9,9'-spirobifluorene (Spiro-2NPB), N,N'-bis(phenanthren-9-yl)-N,N'-bis(phenyl)benzidine (PAPB), 2,7-bis[N, N-bis(9,9-spirobifluoren-2-yl)amino]-9,9-spirobifluorene (Spiro-5), 2,2'-bis[N,N-bis(biphenyl-4-yl)amino]-9,9-spirobifluorene (2,2'-Spiro-DBP), 2,2'-bis(N,N-diphenylamino)-9.9-spirobifluorene (Spiro-BPA), 2,2',7,7'-tetra(N,N-ditolyl)aminospirobifluorene (Spiro-TTB), N,N,N',N'-tetranaphthalen-2-ylbenzidine (TNB), porphyrin compounds and phthalocyanines such as copper phthalocyanines and titanium oxide phthalocyanines. Hole-transporting polymers typically used are selected from the group consisting of polyvinylcarbazoles, (phenylmethyl)polysilanes and polyanilines. It is likewise possible to obtain hole-transporting polymers by doping hole-transporting molecules into polymers such as polystyrene and polycarbonate. Suitable hole-transporting molecules are the molecules already mentioned above.

In addition - in one embodiment - it is possible to use carbene complexes as hole transport materials, the band gap of the at least one hole transport material generally being greater than the band gap of the emitter material used. In the context of the present application, "band gap" is understood to mean the triplet energy. Suitable carbene complexes are, for example, carbene complexes as described in WO 2005/019373 A2, WO 2006/056418 A2, WO 2005/113704, WO 2007/115970, WO 2007/115981 and WO 2008/000727. One example of a suitable carbene complex is fac-Iridium-tris(1,3-diphenylbenzimidazolin-2-yliden-C,C^{2'}) (Ir(dpbic)₃), which is disclosed, for example, in WO2005/019373. Preferably, the hole transport layer comprises a compound of formula 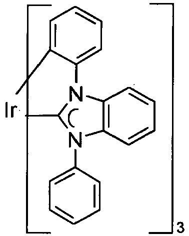 doped with molybdenum oxide (MoOₓ), especially MoO₃, or rhenium oxide (ReOₓ), especially ReO₃. The dopant is contained in an amount of from 0.1% by weight to 60 %, preferably 10 % to 50 % by weight, based on the amount of dopant and carbene complex.

In addition - in one embodiment - it is possible to use Group I substituted compounds of formula I; such as, for example, **A-1** to **A-9** and **B-1** to **B-9,** as hole transport materials.

The light-emitting layer (3) comprises at least one emitter material. In principle, it may be a fluorescence or phosphorescence emitter, suitable emitter materials being known to those skilled in the art. The at least one emitter material is preferably a phosphorescence emitter. The phosphorescence emitter compounds used with preference are based on metal complexes, and especially the complexes of the metals Ru, Rh, Ir, Pd and Pt, in particular the complexes of Ir, have gained significance.

Suitable metal complexes for use in the inventive OLEDs are described, for example, in documents W O 02/60910 A1, US 2001/0015432 A1, US 2001/0019782 A1, US2002/0055014A1, US 2002/0024293 A1, US 2002/0048689 A1, EP 1 191 612 A2, EP 1 191 613 A2, EP 1211 257 A2, US 2002/0094453 A1, WO 02/02714 A2, WO 00/70655 A2, WO 01/41512 A 1 ; W O 02/15645 A1, WO 2005/019373 A2, WO 2005/113704 A2 , WO 2006/115301 A1, WO 2006/067074 A1, WO 2006/056418, WO 2006121811 A1, WO 2007095118 A2, WO 2007/115970, WO 2007/115981 and WO 2008/000727, WO06/000544, WO07/074093, WO07/115981, WO08/101842, WO09/100991, WO10/129323, WO2010056669, WO10086089, US2010/213834, US2011/089407, and WO11/073149.

In one embodiment of the present invention, the compounds of the formula X are used in the light-emitting layer as matrix material together with carbene complexes as triplet emitters. 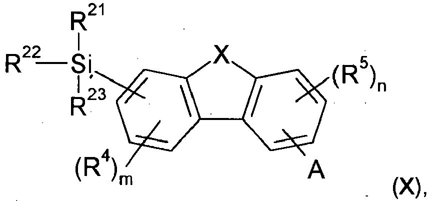 wherein
X is NR2²⁴, S, O or PR²⁴;
R²⁴ is aryl, heteroaryl, alkyl, cycloalkyl, or heterocycloalkyl;
A is -NR²⁶R²⁷, -P(O)R^{28'}R²⁹, -PR¹⁰R¹¹, -S(O)₂R¹², -S(O)R¹³, -SR¹⁴, or -OR¹⁵;
R¹, R² and R³ are independently of each other aryl, heteroaryl, alkyl, cycloalkyl, or heterocycloalkyl, wherein at least on of the groups R¹, R², or R³ is aryl, or heteroaryl;
R⁴ and R⁵ are independently of each other alkyl, cycloalkyl, heterocycloalkyl, aryl, heteroaryl, a group A, or a group having donor, or acceptor characteristics;
n and m are independently of each other 0, 1, 2, or 3;
R²⁶, R²⁷ form together with the nitrogen atom a cyclic residue having 3 to 10 ring atoms, which can be unsubstituted, or which can be substituted with one, or more substituents selected from alkyl, cycloalkyl, heterocycloalkyl, aryl, heteroaryl and a group having donor, or acceptor characteristics; and/or which can be annulated with one, or more further cyclic residues having 3 to 10 ring atoms, wherein the annulated residues can be unsubstituted, or can be substituted with one, or more substituents selected from alkyl, cycloalkyl, heterocycloalkyl, aryl, heteroaryl and a group having donor, or acceptor characteristics; and
R28', R²⁹, R¹⁰, R¹¹, R¹², R¹³, R¹⁴ und R¹⁵ are independently of each other aryl, heteroaryl, alkyl, cycloalkyl, or heterocycloalkyl. Compounds of formula X, such as, for example, 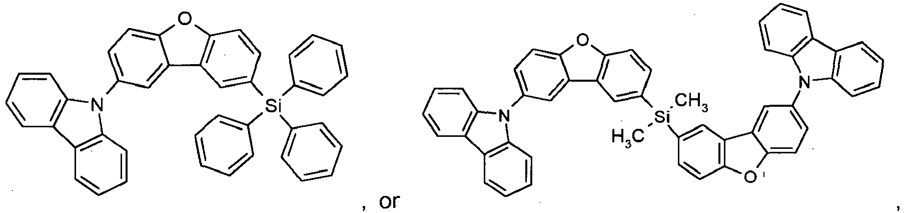 are described in WO2010079051 (PCT/EP2009/067120; in particular pages on 19 to 26 and in tables on pages 27 to 34, pages 35 to 37 and pages 42 to 43).

Additional matrix materials on basis of dibenzofurane are, for example, described in US2009066226, EP1885818B1, EP1970976, EP1998388 and EP2034538. Examples of particularly preferred matrix materials are shown below: 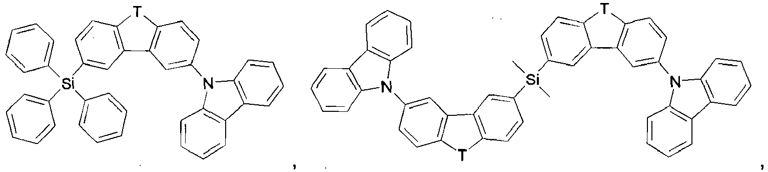 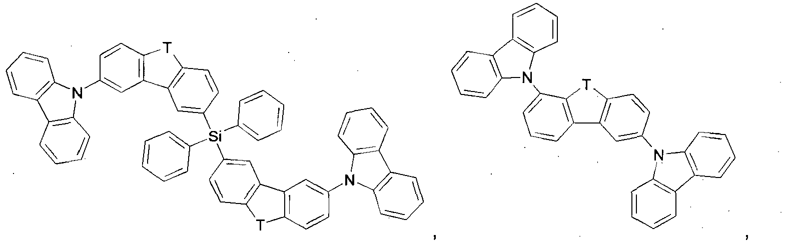 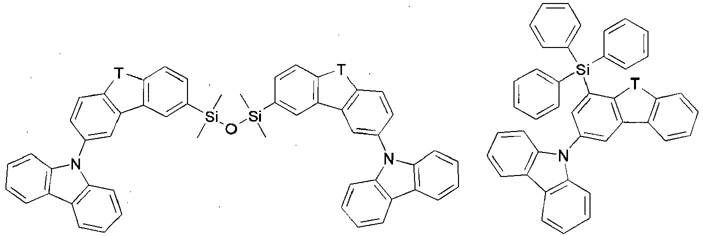 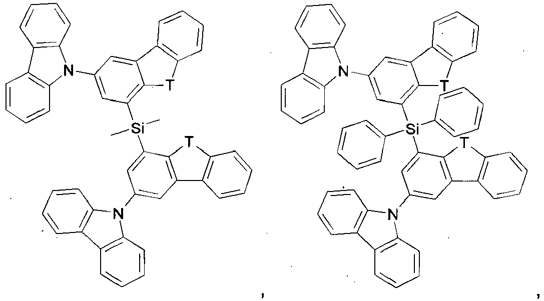 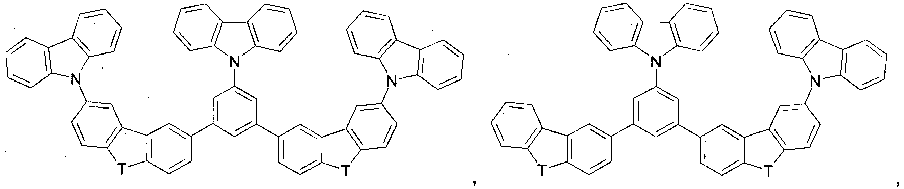 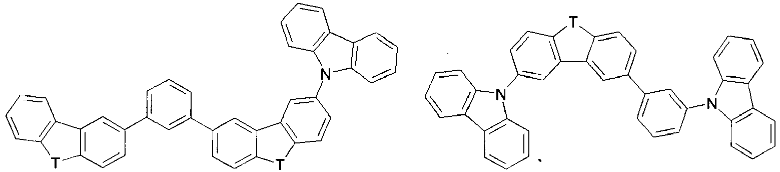 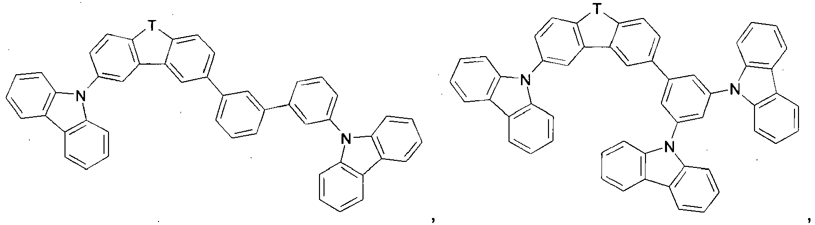 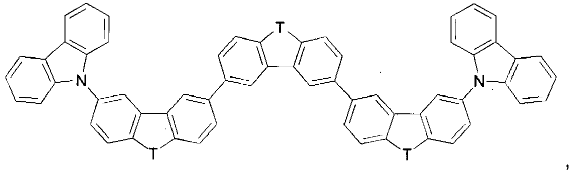 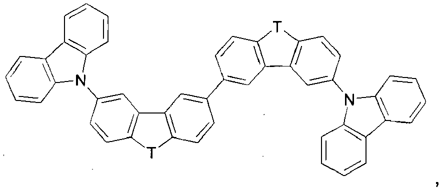 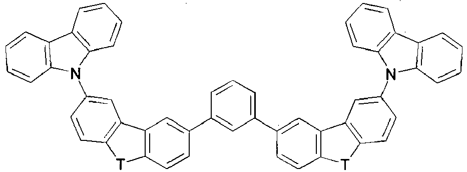 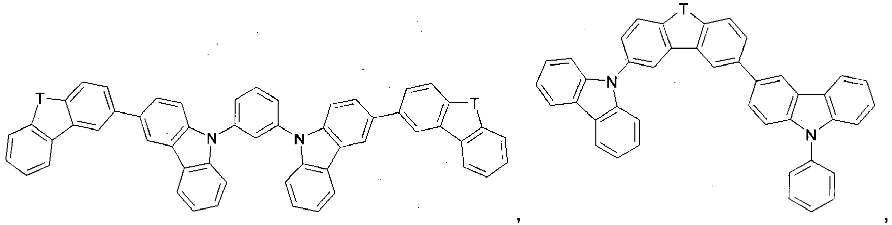 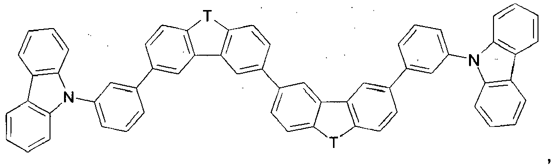 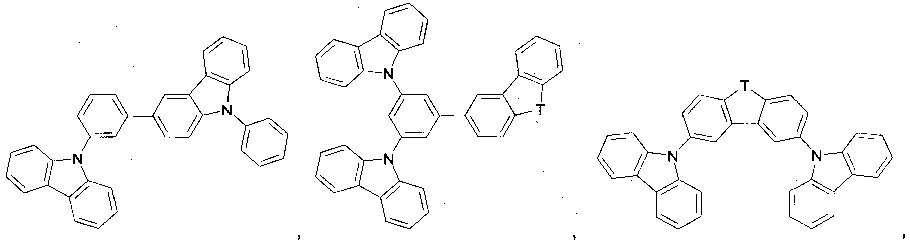 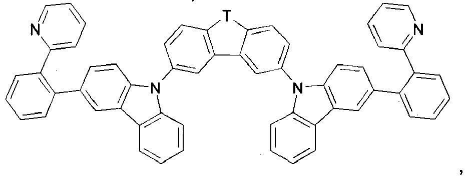 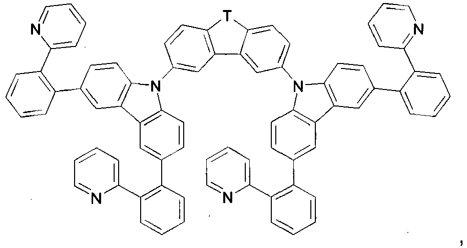 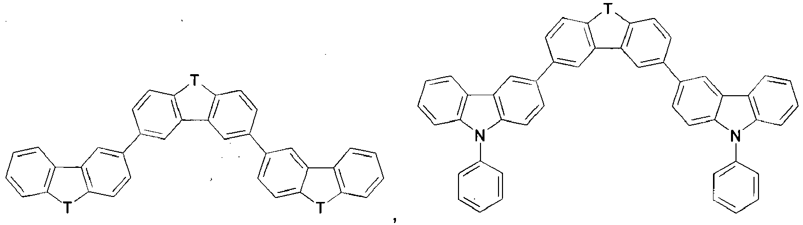 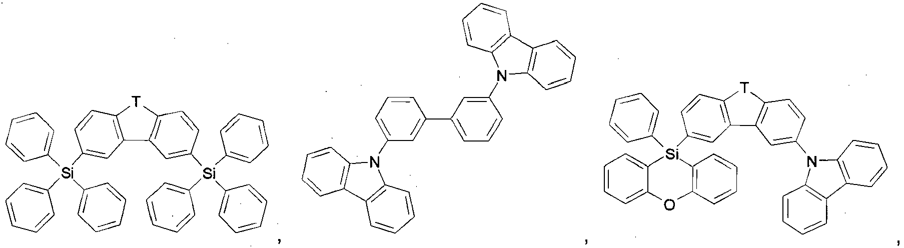 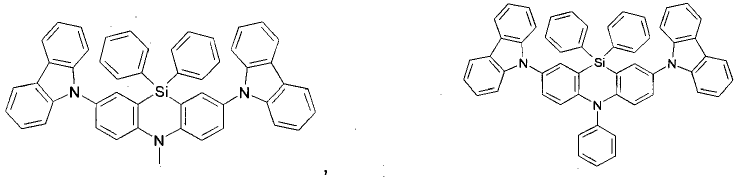 and

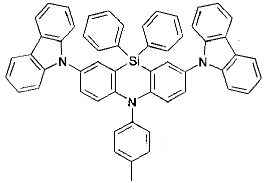

In the above-mentioned compounds T is O, or S, preferably O. If T occurs more than one time in a molecule, all groups T have the same meaning.

In a particularly preferred embodiment of the present invention compounds of the formula I, especially Group I substituted compounds of formula I, such as, for example, **A-1** to **A-9** and **B-1** to **B-9**, are used in the light-emitting layer as matrix material (host) together with phosphorescent materials (triplet emitters) described, for example, in WO06/000544, WO2008/101842, and WO2009100991. Examples of phosphorescent materials are compounds A-1 to B-234, B-1 to B-234, C-1 to C-44 and D-1 to D-234, which are described in WO2008/101842. Preferred examples are described in WO2009100991 example 48-53, 54-78.

In a further embodiment, the compounds of the formula **X** are used as hole/exciton blocker material, preferably together with a triplet emitter describe above. The compounds of the formula **X** may be used as matrix materials or both as matrix materials and as hole/exciton blocker materials together with a triplet emitter described above. Suitable metal complexes for use together with the compounds of the formula **X** as matrix material and/or hole/exciton blocker material, in OLEDs are thus, for example, also carbene complexes as described in WO 2005/019373 A 2, W O 2006/056418 A2, WO 2005/113704, WO 2007/115970, WO 2007/115961 and WO 2008/00727. Explicit reference is made here to the disclosure of the WO applications cited.

Hole blocker materials typically used in OLEDs are compounds of formula **X,** 2,6-bis(N-carbazolyl)pyridine (mCPy), 2,9-dimethyl-4,7-diphenyl-1,10-phenanthroline (bathocuproin, (BCP)), bis(2-methyl-8-quinolinato)-4-phenylphenylato)aluminum(III) (BAIq), phenothiazine S,S-dioxide derivates and 1,3,5-tris(N-phenyl-2-benzylimidazolyl)benzene) (TPBI), TPBI also being suitable as electron-conducting material. Further suitable hole blockers and/or electron transport materials are 2,2',2"-(1,3,5-benzenetriyl)tris(1-phenyl-1-H-benzimidazole), 2-(4-biphenylyl)-5-(4-tert-butylphenyl)-1,3,4-oxadiazole, 8-hydroxyquinolinolatolithium, 4-(naphthalen-1-yl)-3,5-diphenyl-4H-1,2,4-triazole, 1,3-bis[2-(2,2'-bipyridin-6-yl)-1,3,4-oxadiazo-5-yl]benzene, 4,7-diphenyl-1,10-phenanthroline, 3-(4-biphenylyl)-4-phenyl-5-tert-butylphenyl-1,2,4-triazole, 6,6'-bis[5-(biphenyl-4-yl)-1,3,4-oxadiazo-2-yl]-2,2'-bipyridyl, 2-phenyl-9,10-di(naphthalene-2-yl)anthracene, 2,7-bis[2-(2,2'bipyridin-6-yl)-1,3,4-oxadiazo-5-yl]-9,9-d dimethylfluorene, 1,3-bis[2-(4-tert-butylphenyl)-1,3,4-oxadiazo-5-yl]benzene, 2-(naphthalene-2-yl)-4,7-diphenyl-1,10-phenanthroline, tris-(2,4,6-trimethyl-3-(pyridin-3-yl)phenyl)borane, 2,9-bis(naphthalene-2-yl)-4,7-diphenyl-1,10-phenanthroline, 1-methyl-2-(4-(naphthalene-2-yl)phenyl)-1H-imidazo[4,5-f][1,10]-phenanthroline. In a further embodiment, it is possible to use compounds which comprise aromatic or heteroaromatic rings joined via groups comprising carbonyl groups, as disclosed in WO2006/100298, disilyl compounds selected from the group consisting of disilylcarbazoles, disilylbenzofurans, disilylbenzothiophenes, disilylbenzophospholes, disilylbenzothiophene S-oxides and disilylbenzothiophene S,S-dioxides, as specified, for example, in WO2009003919 (PCT/EP2008/058207) and WO2009003898 (PCT/EP2008/058106) and disilyl compounds as disclosed in WO2008/034758, as a blocking layer for holes/excitons (4) or as matrix materials in the light-emitting layer (3).

In a preferred embodiment, the present invention relates to an inventive OLED comprising the layers (1) anode, (2) hole transport layer, (3) light-emitting layer, (4) blocking layer for holes/excitons, (5) electron transport layer and (6) cathode, and if appropriate further layers.

Suitable electron transport materials for the layer (5) of the inventive OLEDs comprise metals chelated to oxinoid compounds, such as 2,2',2"-(1,3,5-phenylene)tris[1-phenyl-1Hbenzimidazole] (TPBI), tris(8-quinolinolato)aluminum (Alq₃), compounds based on phenanthroline, such as 2,9-dimethyl-4,7-diphenyl-1,10-phenanthroline (DDPA = BCP) or 4,7-diphenyl-1,10-phenanthroline (DPA), and azole compounds such as 2-(4-biphenylyl)-5-(4-t-butylphenyl)-1,3,4-oxadiazole (PBD) and 3-(4-biphenylyl)-4-phenyl-5-(4-t-butylphenyl)-1,2,4-triazole (TAZ), 8-hydroxyquinolinolatolithium (Liq), 4,7-diphenyl-1,10-phenanthroline (BPhen), bis(2-methyl-8-quinolinolato)-4-(phenylphenolato)aluminum (BAIq), 1,3-bis[2-(2,2'-bipyridin-6-yl)-1,3,4-oxadiazo-5-yl]benzene (Bpy-OXD), 6,6'-bis[5-(biphenyl-4-yl)-1,3,4-oxadiazo-2-yl]-2,2'-bipyridyl (BP-OXD-Bpy), 4-(naphthalen-1-yl)-3,5-diphenyl-4H-1,2,4-triazole (NTAZ), 2,9-bis(naphthalen-2-yl)-4,7-diphenyl-1,10-phenanthroline (NBphen), 2,7-bis[2-(2,2'-bipyridin-6-yl)-1,3,4-oxadiazo-5-yl]-9,9-dimethylfluorene (Bby-FOXD), 1,3-bis[2-(4-tert-butylphenyl)-1,3,4-oxadiazo-5-yl]benzene (OXD-7), tris(2,4,6-trimethyl-3-(pyridin-3-yl)phenyl)borane (3TPYMB), 1-methyl-2-(4-(naphthalen-2-yl)phenyl)-1H-imidazo[4,5-f][1,10]phenanthroline (2-NPIP), 2-phenyl-9,10-di(naphthalen-2-yl)anthracene (PADN), 2-(naphthalen-2-yl)-4,7-diphenyl-1,10-phenanthroline (HNBphen). The layer (5) may serve both to facilitate electron transport and as a buffer layer or barrier layer in order to prevent quenching of the exciton at the interfaces of the layers of the OLED. The layer (5) preferably improves the mobility of the electrons and reduces quenching of the exciton. In a preferred embodiment, BCP doped with CsCO₃ is used as the electron transport material. In principle, it is possible that the electron transport (conductor) layer comprises at least one compound of the formula (I) as electron transport material.

In addition - in one embodiment - it is possible to use Group II and III substituted compounds of formula I, such as, for example, **C-1** to **C-6, D-1** to **D-6, E-1** to **E-20** and **F-1** to **F-20** as electron transport materials.

Among the materials mentioned above as hole transport materials and electron transport materials, some may fulfil several functions. For example, some of the electron-transporting materials are simultaneously hole-blocking-materials when they have a low-lying HOMO. These can be used, for example, in the blocking layer for holes/excitons (4).

The charge transport layers can also be electronically doped in order to improve the transport properties of the materials used, in order firstly to make the layer thicknesses more generous (avoidance of pinholes/short circuits) and in order secondly to minimize the operating voltage of the device. For example, the hole transport materials can be doped with electron acceptors; for example, phthalocyanines or arylamines such as TPD or TDTA can be doped with tetrafluorotetracyanquinodimethane (F4-TCNQ) or with MoO₃ or WO₃. Electronic doping is known to those skilled in the art and is disclosed, for example, in W. Gao, A. Kahn, J. Appl. Phys., Vol. 94, No. 1, 1 July 2003 (p-doped organic layers); A. G. Wemer, F. Li, K. Harada, M. Pfeiffer, T. Fritz, K. Leo. Appl. Phys. Lett., Vol. 82, No. 25, 23 June 2003 and Pfeiffer et al., Organic Electronics 2003, 4, 89 - 103. For example, the hole transport layer may, in addition to a carbene complex, e.g. Ir(dpbic)₃, be doped with MoO₃, ReO₃, or WO₃.

The cathode (6) is an electrode which serves to introduce electrons or negative charge carriers. Suitable materials for the cathode are selected from the group consisting of alkali metals of group la, for example Li, Cs, alkaline earth metals of group IIa, for example calcium, barium or magnesium, metals of group IIb of the periodic table of the elements (old IUPAC version), comprising the lanthanides and actinides, for example samarium. In addition, it is also possible to use metals such as aluminum or indium, and combinations of all metals mentioned. In addition, lithium-comprising organometallic compounds or potassium fluoride (KF) can be applied between the organic layer and the cathode in order to reduce the operating voltage.

The OLED according to the present invention may additionally comprise further layers which are known to those skilled in the art. For example, a layer which facilitates the transport of the positive charge and/or matches the band gaps of the layers to one another may be applied between the layer (2) and the light-emitting layer (3). Alternatively, this further layer may serve as a protective layer. In an analogous manner, additional layers may be present between the light-emitting layer (3) and the layer (4) in order to facilitate the transport of negative charge and/or to match the band gaps between the layers to one another. Alternatively, this layer may serve as a protective layer.

In a preferred embodiment, the inventive OLED, in addition to layers (1) to (6), comprises at least one of the following layers mentioned below:
- a hole injection layer between the anode (1) and the hole-transport layer (2);
- a blocking layer for electrons between the hole-transport layer (2) and the light-emitting layer (3);
- an electron injection layer between the electron-transport layer (5) and the cathode (6).

Materials for a hole injection layer may be selected from copper phthalocyanine, 4,4',4"-tris(N-3-methylphenyl-N-phenylamino)triphenylamine (m-MTDATA), 4,4',4"-tris(N-(2-naphthyl)-N-phenylamino)triphenylamine (2T-NATA), 4,4',4"-tris(N-(1-naphthyl)-N-phenylamino)triphenylamine (1T-NATA), 4,4',4"-tris(N,N-diphenylamino)triphenylamine (NATA), titanium oxide phthalocyanine, 2,3,5,6-tetrafluoro-7,7,8,8-tetracyanoquino-dimethane (F4-TCNQ), pyrazino[2,3-f][1,10]phenanthroline-2,3-dicarbonitrile (PPDN), N,N,N',N'-tetrakis(4-methoxyphenyl)benzidine (MeO-TPD), 2,7-bis[N,N-bis(4-methoxyphenyl)amino]-9,9-spirobifluorene (MeO-Spiro-TPD), 2,2'-bis[N,N-bis(4-methoxyphenyl)amino]-9,9-spirobifluorene (2,2'-MeO-Spiro-TPD), N,N'-diphenyl-N,N'-di-[4-(N,N-ditolylamino)phenyl]benzidine (NTNPB), N,N'-diphenyl-N,N'-di-[4-(N,N-diphenyl-amino)phenyl]benzidine (NPNPB), N,N'-di(naphthalen-2-yl)-N,N'-diphenylbenzene-1,4-diamine (α-NPP). In principle, it is possible that the hole injection layer comprises at least one compound of the formula X as hole injection material. In addition, polymeric hole-injection materials can be used such as poly(N-vinylcarbazole) (PVK), polythiophenes, polypyrrole, polyaniline, self-doping polymers, such as, for example, sulfonated poly(thiophene-3-[2[(2-methoxyethoxy)ethoxy]-2,5-diyl) (Plexcore^{®} OC Conducting Inks commercially available from Plextronics), and copolymers such as poly(3,4-ethylenedioxythiophene)/poly(4-styrenesulfonate) also called PEDOT/PSS.

As a material for the electron injection layer, KF, or 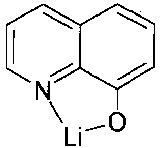 (8-hydroxyquinolinolato-lithium (**Liq**)), for example, can be selected. KF is more preferred than Liq.

The person skilled in the art is aware (for example on the basis of electrochemical studies) of how suitable materials have to be selected. Suitable materials for the individual layers are known to those skilled in the art and are disclosed, for example, in WO 00/70655.

In addition, it is possible that some of the layers used in the inventive OLED have been surface-treated in order to increase the efficiency of charge carrier transport. The selection of the materials for each of the layers mentioned is preferably determined by obtaining an OLED with a high efficiency and lifetime.

The inventive OLED can be produced by methods known to those skilled in the art. In general, the inventive OLED is produced by successive vapor deposition of the individual layers onto a suitable substrate. Suitable substrates are, for example, glass, inorganic semi-transports, typically ITO, or IZO, or polymer films. For vapor deposition, it is possible to use customary techniques, such as thermal evaporation, chemical vapor deposition (CVD), physical vapor deposition (PVD) and others. In an alternative process, the organic layers of the OLED can be applied from solutions or dispersions in suitable solvents, employing coating techniques known to those skilled in the art.

In general, the different layers have the following thicknesses: anode (1) 50 to 500 nm, preferably 100 to 200 nm; hole-conducting layer (2) 5 to 100 nm, preferably 20 to 80 nm, light-emitting layer (3) 1 to 100 nm, preferably 10 to 80 nm, blocking layer for holes/excitons (4) 2 to 100 nm, preferably 5 to 50 nm, electron-conducting layer (5) 5 to 100 nm, preferably 20 to 80 nm, cathode (6) 20 to 1000 nm, preferably 30 to 500 nm. The relative position of the recombination zone of holes and electrons in the inventive OLED in relation to the cathode and hence the emission spectrum of the OLED can be influenced, among other factors, by the relative thickness of each layer. This means that the thickness of the electron transport layer should preferably be selected such that the position of the recombination zone is matched to the optical resonator property of the diode and hence to the emission wavelength of the emitter. The ratio of the layer thicknesses of the individual layers in the OLED depends on the materials used. The layer thicknesses of any additional layers used are known to those skilled in the art. It is possible that the electron-conducting layer and/or the hole-conducting layer have greater thicknesses than the layer thicknesses specified when they are electrically doped.

Use of the electron transport layer of the present application makes it possible to obtain OLEDs with high efficiency and with low operating voltage. Frequently, the OLEDs obtained by the use of the electron transport layer of the present application additionally have high lifetimes. The efficiency of the OLEDs can additionally be improved by optimizing the other layers of the OLEDs. Shaped substrates and novel hole-transport materials which bring about a reduction in the operating voltage or an increase in the quantum efficiency are likewise usable in the inventive OLEDs. Moreover, additional layers may be present in the OLEDs in order to adjust the energy level of the different layers and to facilitate electroluminescence.

The OLEDs may further comprise at least one second light-emitting layer. The overall emission of the OLEDs may be composed of the emission of the at least two light-emitting layers and may also comprise white light.

The OLEDs can be used in all apparatus in which electroluminescence is useful Suitable devices are preferably selected from stationary and mobile visual display units and illumination units. Stationary visual display units are, for example, visual display units of computers, televisions, visual display units in printers, kitchen appliances and advertising panels, illuminations and information panels. Mobile visual display units are, for example, visual display units in cell phones, laptops, digital cameras, MP3 players, vehicles and destination displays on buses and trains. Further devices in which the inventive OLEDs can be used are, for example, keyboards; items of clothing; furniture; wallpaper.

In addition, the compounds of the present application can be used in OLEDs with inverse structure. The structure of inverse OLEDs and the materials typically used therein are known to those skilled in the art.

In addition, the present invention relates to an apparatus selected from the group consisting of stationary visual display units such as visual display units of computers, televisions, vis-ual display units in printers, kitchen appliances and advertising panels, illuminations, information panels, and mobile visual display units such as visual display units in cellphones, laptops, digital cameras, MP3 players, vehicles and destination displays on buses and trains; illumination units; keyboards; items of clothing; furniture; wallpaper, comprising the inventive organic electronic device, or the inventive the hole transport, or electron transport layer.

The following examples are included for illustrative purposes only. Unless otherwise stated, all parts and percentages are by weight.

### Examples

### Example 1

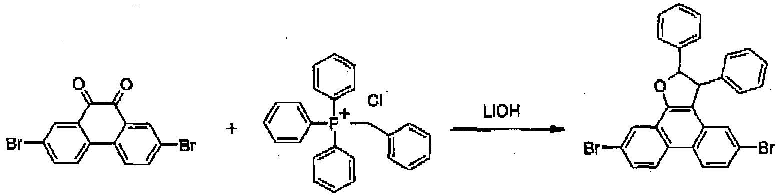

a) 6.88 g (164 mmol) lithium hydroxide monohydrate in 50 ml water are added to 20 g ( 54.6 mmol) 2,7-dibromophenanthrene-9,10-dione and 43.6 g (112 mmol) benzyl(triphenyl)phosphonium chloride in 200 ml methylen chloride. The reaction mixture is stirred at 25 °C for 4 h. The organic phase is separated and dried with magnesium sulphate. The solvent is distilled off and the product is decocted in ethanol (yield: 20 g (69 %)). ¹H NMR (300 MHz, CDCl₃, δ): 8.53 (d, J= 8.9 Hz, 1H), 8.42-8.46 (m, 2H), 7.83 (dd, J= 8.9 Hz, J= 2.1 Hz, 1 H), 7.53 (dd, J= 2.1 Hz, J= 8.9 Hz, 1 H), 7.25-7.45 (m, 11 H), 5.84 (d, J= 5.8 Hz, 1 H), 4.95 (d, J=5.8 Hz, 1 H). 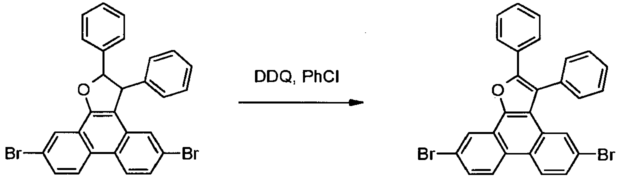
b) 2.50 g (11.0 mmol) 2,3-dichloro-5,6-dicyanobenzoquinone (DDQ) are added to 5.6 g (10.0 mmol) of 5,10-dibromo-2,3-diphenyl-2,3-dihydrophenanthro[9,10-b]furan in 25 ml chlorobenzene. The reaction mixture is refluxed for 2 h under nitrogen, dichloromethane is added and the reaction mixture is washed with a sodium hydrogen carbonate solution. The organic phase is dried with magnesium sulphate. The solvent is distilled off and the product is decocted in dibutylether (yield: 4.80 g (91 %)). ¹H NMR (300 MHz, CDCl₃, δ): 8.61 (d, J= 2 Hz, 1 H), 8.45-8.49 (m, 2H), 7.73 (dd, J= 8.9 Hz, J= 2.1 Hz, 1 H), 7.54-7.64 (m, 8 H), 7.27-7.37 (m, 4H). 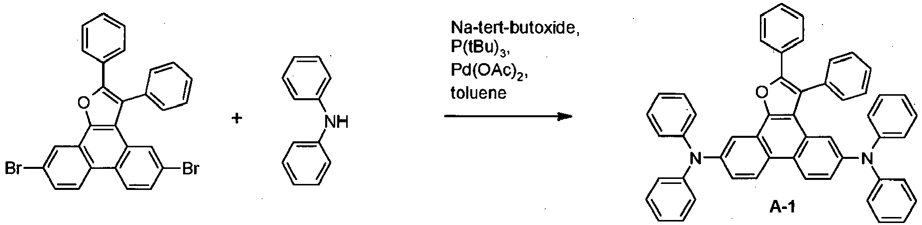
c) 2.01g g (20.9 mmol) sodium tert-butoxide are added to 4.80 g (9.08 mmol) of the product of example 1b in 100 ml toluene. 3.38 g (20.0 mmol) N-phenylaniline are added. The reaction mixture is degassed with argon. 102 mg (0.45 mmol) palladium(II) acetate are added. The reaction mixture is degassed with argon. 184 mg (91 mmol) tri-tert-butylphosphine are added. The reaction mixture is degassed with argon. The reaction mixture is stirred for 18 h at 90 °C under argon. A 1 % sodium cyanide solution is added and the mixture is extracted with dichloromethane. The organic phase is dried with magnesium sulphate. The solvent is distilled off. Column chromatography on silica gel with toluene cyclohexane 1/4 results in the product (yield: 3.18 g (50 %))
   ¹H NMR (300 MHz, THF-d₈, δ): 8.58 (d, J= 9.1 Hz, 1 H), 8.54 (d, J= 9.9 Hz, 1 H), 8.11 (d, J= 2.4 Hz, 1H), 6.95-7.45 (m, 33H).

### Example 2

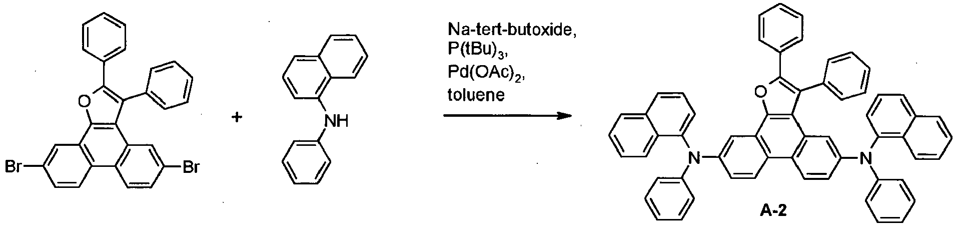

Example **1c**) is repeated, except that 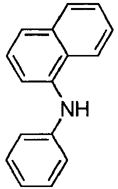 is used instead of N-phenylaniline.
Compound **A-2** is obtained in a yield of 54%.
¹H NMR (300 MHz, Benzene-d₆, δ): 8.60 (d, J= 2.4 Hz, 1H); 8.41-8.45 (m, 1H), 8.34- 8.27 (m, 2H), 8.12 (d, J= 8.4 Hz, 1 H), 6.86-7.79 (m, 35H).

### Application Example 1

The ITO substrate used as the anode is first cleaned with commercial detergents for LCD production (Deconex^{®} 20NS, and 250RGAN-ACID^{®} neutralizing agent) and then in an acetone/isopropanol mixture in an ultrasound bath. To eliminate any possible organic residues, the substrate is exposed to a continuous ozone flow in an ozone oven for a further 25 minutes. This treatment also improves the hole injection properties of the ITO. Then Plexcore^{®} OC AJ20-1000 (commercially available from Plextronics Inc.) is spin-coated and dried to form a hole injection layer (∼40 nm).

Thereafter, the organic materials specified below are applied by vapor deposition to the clean substrate at a rate of approx. 0.5-5 nm/min at about 10⁻⁷ -10⁻⁹ mbar. As a hole transport and exciton blocker, Ir(dpbic)₃ (for preparation, see Ir complex (7) in the application WO 2005/019373) is applied to the substrate with a thickness of 45 nm, wherein the first 35 nm are doped with MoOₓ (∼10%) to improve the conductivity.

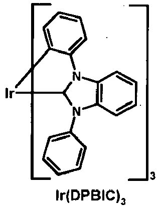

Subsequently, a mixture of 10% by weight of compound 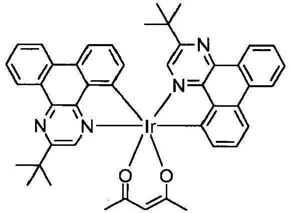 and 90% by weight of compound A-1 (matrix material) is applied by vapor deposition in a thickness of 20 nm.

Subsequently, the material bis(2-methyl-8-quinolinolato)(4-phenylphenolato)aluminum(III) (BAlq) is applied by vapor deposition with a thickness of 10 nm as exciton and hole blocker.

Next, a mixture of 93 % by weight of 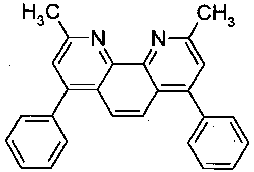 (BCP) and 7 % by weight of Cs₂CO₃ is applied as electron transport layer by vapor deposition in a thickness of 50 nm and finally a 100 nm-thick Al electrode is applied by vapor deposition.

All prefabricated parts are sealed with a glass lid in an inert nitrogen atmosphere.

### Comparative Application Example 1

Production and construction of an OLED as in the application example 1, except α-NPD is used host instead of compound **A-1.**

To characterize the OLED, electroluminescence spectra are recorded at various currents and voltages. In addition, the current-voltage characteristic is measured in combination with the light output emitted. The light output can be converted to photometric parameters by calibration with a photometer. To determine the lifetime, the OLED is operated at a constant current density and the decrease in the light output is recorded. The lifetime is defined as that time which lapses until the luminance decreases to half of the initial luminance.

| | Host | EQE¹⁾ @ 1000nits |
|---|---|---|
| Application Example 1 | Compound A-1 | 12.4% |
| Comp. Appl. Ex. 1 | α-NPD | 11.6% |

| | | |
|---|---|---|
| ¹⁾ External quantum efficiency (EQE) is # a of generated photons escaped from a substance or a device / # of electrons flowing through it. | | |

The device of application example 1, where compound **A-1** is used as host, shows a better external quantum efficiency as the device of comparative application example 1, where α-NPD is used as host.

### Application Example 2

After the hole injection layer the organic materials specified below are applied by vapor deposition to the clean substrate at a rate of approx. 0.5-5 nm/min at about 10⁻⁷ -10⁻⁹ mbar. As a hole transport and exciton blocker α-NPD is applied to the substrate with a thickness of 20 nm, wherein the first 10 nm are doped with MoOx (∼10%) to improve the conductivity.

Subsequently, a mixture of 10% by weight of compound 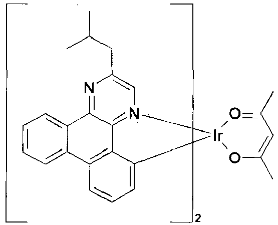 and 90% by weight of compound **A-2** (matrix material) is applied by vapor deposition in a thickness of 20 nm.

Subsequently, the material BAIq is applied by vapor deposition with a thickness of 10 nm as exciton and hole blocker. Next, a mixture of 93 % by weight of BCP and 7 % by weight of Cs₂CO₃ is applied as electron transport layer by vapor deposition in a thickness of 50 nm and finally a 100 nm-thick Al electrode is applied by vapor deposition.

### Comparative Application Example 2

Production and construction of an OLED as in the application example 2, except 4,4'-bis[N-(1-naphthyl)-N-phenylamino]biphenyl (α-NPD) is used host instead of compound **A-2.**

| | HTL¹⁾ | Host | Lifetime [h] @ 6000nits |
|---|---|---|---|
| Application Example 2 | α-NPD | Compound A-2 | 893 h |
| Comp. Appl. Ex. 2 | α-NPD | α-NPD | 607 h |

| | | | |
|---|---|---|---|
| ¹⁾HTL = Hole Transport Layer. | | | |

The device of application example 2, where compound **A-1** is used as host, shows a better life time than the device of comparative application example 2, where α-NPD is used as host.

### Application Example 3

After the hole injection layer the organic materials specified below are applied by vapor deposition to the clean substrate at a rate of approx. 0.5-5 nm/min at about 10⁻⁷ -10⁻⁹ mbar. As a hole transport and exciton blocker compound **A-2** is applied to the substrate with a thickness of 20 nm, wherein the first 10 nm are doped with MoOₓ (∼10%) to improve the conductivity.

Subsequently, a mixture of 10% by weight of compound 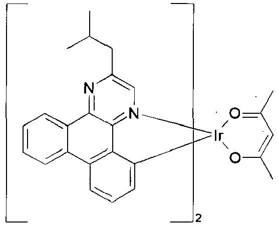 and 90% by weight of α-NPD (matrix material) is applied by vapor deposition in a thickness of 20 nm.

Subsequently, the material BAIq is applied by vapor deposition with a thickness of 10 nm as exciton and hole blocker. Next, a mixture of 93 % by weight of 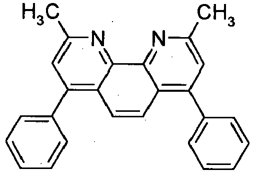 (BCP) and 7 % by weight of Cs₂CO₃ is applied as electron transport layer by vapor deposition in a thickness of 50 nm and finally a 100 nm-thick Al electrode is applied by vapor deposition. **HTL Host Lifetime [h] @ 6000nits Application Example 3** Cpd**. A-2** α-NPD 1349 h **Comp. Appl. Ex. 2** α-NPD α-NPD 607 h

The device of application example 3, where compound **A-1** is used as hole transport material (HTM), shows a better life time than the device of comparative application example 2, where α-NPD is used as HTM.

### Example 3

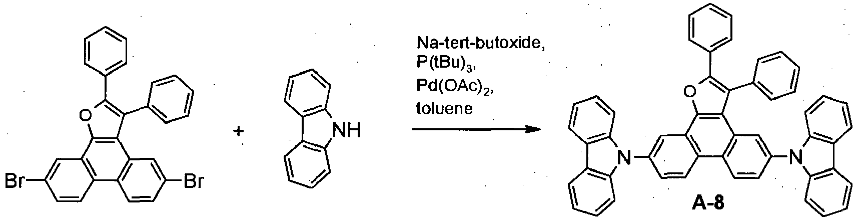

0.84 g (8.74 mmol) sodium tert-butoxide are added to 2.10 g (3.98 mmol) of the product of example **1b** in 60 ml toluene. 0.70 g (8.37 mmol) carbazole are added. The reaction mixture is degassed with argon. 43 mg (0.19 mmol) palladium(II) acetate are added. 77 mg (0.38 mmol) tri-tert-butylphosphine are added. The reaction mixture is stirred for 24 h at 100 °C under argon. The reaction mixture is filtered through a plug of silica gel, and the solvent of the filtrate is distilled off. Column chromatography on silica gel with toluene cyclohexane 1/4 results in the product (compound **A-8**) (yield: 1.31 g (47 %))
¹H NMR (500 MHz, THF-d₈, δ): 8.96 (t, J = 8.1 Hz, 2H), 8.75 (s, 1H), 8.24 (d, J = 7.8 Hz, 2H), 8.12 (d, J = 7.7 Hz, 2H), 7.89-7.92 (m, 2H), 7.84 (d, J = 8.7 Hz, 1H), 7.63 (d, J = 8.1 Hz, 2H), 7.55-7.60 (m, 4H), 7.50 (t, J = 7.6 Hz, 2H), 7.35-7.44 (m, 8H), 7.30 (t, J = 7.2 Hz, 2H), 7.22-7.28 (m, 4H).

### Example 4

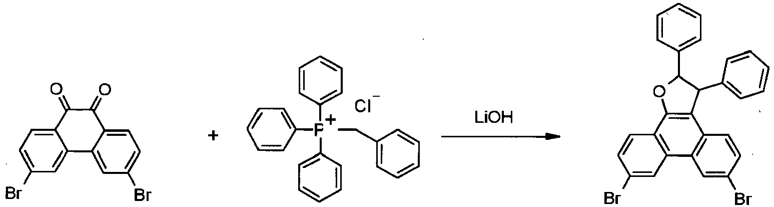

a) 1.96 g (81.9 mmol) lithium hydroxide in 25 ml water are added to 10.0 g (27.3 mmol) 3,6-dibromophenanthrene-9,10-dione (Brunner, Klemens; Dijken, Addy van; Boerner, Herbert; Bastiaansen, Jolanda J. A. M.; Kiggen, Nicole M. M.; Langeveld, Bea M. W.; J. Am. Chem. Soc. 126 (2004) 6035-6042) and 21.7 g (55.8 mmol) benzyl(friphenyl)phosphonium chloride in 230 ml methylene chloride. The reaction mixture is stirred at 25 °C for 20 h. 100 ml water are added and the organic layer is separated. The aqueous layer is extracted with methylene chloride and the combined organic phases are dried with sodium sulphate. The solvent is distilled off and the product is decocted in ethanol (yield: 10.4 g (72 %)).
   ¹H NMR (400 MHz, CDCl₃, δ): 8.76 (d, J = 1.6 Hz, 1H), 8.67 (d, J = 1.7 Hz, 1H), 8.14 (d, J = 8.6 Hz, 1H), 7.80 (dd, J = 8.5 Hz and 1.7 Hz, 1H), 7.43 (dd, J = 8.7 Hz and 1.8 Hz, 1H), 7.30-7.40 (m, 8H), 7.22-7.25 (m, 2H), 7.15 (d, J = 8.6 Hz, 1 H), 5.80 (d, J = 6.2 Hz, 1 H), 4.94 (d, J = 6.2 Hz, 1 H). 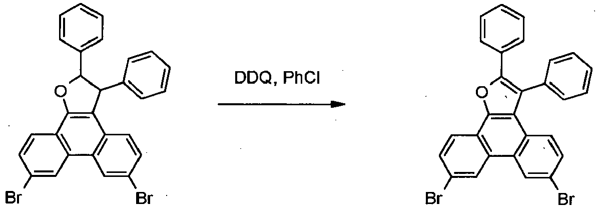
b) 4.70 g (20.7 mmol) 2,3-dichloro-5,6-dicyanobenzoquinone (DDQ) are added to 10.0 g (18.8 mmol) of 6,9-dibromo-2,3-diphenyl-2,3-dihydrophenanthro[9,10-b]furan in 50 ml chlorobenzene. The reaction mixture is refluxed for 4 h under nitrogen, dichloromethane is added and the reaction mixture is washed with a sodium hydrogen carbonate solution. The organic phase is dried with magnesium sulphate. The solvent is distilled off and the product is decocted in dibutylether. Column chromatography on silica gel with toluene cyclohexane 4/1 results in the product (yield: 5.40 g (54 %)).
   ¹H NMR (400 MHz, CDCl₃, δ): 8.65 (d, J = 10.9 Hz, 2H), 8.29 (d, J = 8.6 Hz, 1H), 7.79 (d, J = 8.6 Hz, 1H), 7.49-7.59 (m, 7 H), 7.34-7.43 (m, 2H), 7.26-7.32 (m, 3H). 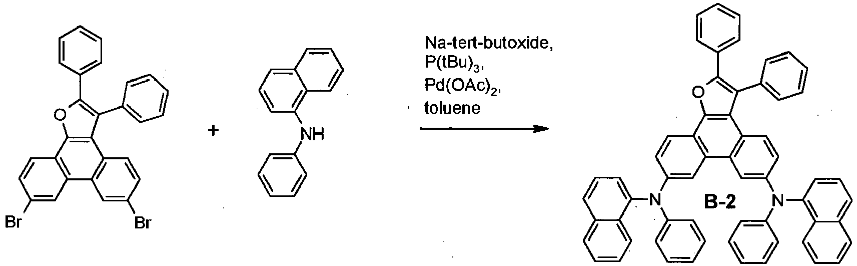
c) 0.42 g (4.35 mmol) sodium tert-butoxide are added to 1.00 g (1.89 mmol) of the product of example **4b** in 15 ml toluene. 0.91 g (4.16 mmol) N-phenyl-N-naphthylamine are added.
   The reaction mixture is degassed with argon. 21 mg (0.09 mmol) palladium(II) acetate are added. 38 mg (0.19 mmbl) tri-tert-butylphosphine are added. The reaction mixture is stirred for 18 h at 100 °C under argon. The reaction mixture is filtered through a plug of silica gel, and the solvent of the filtrate is distilled off. Column chromatography on silica gel with toluene cyclohexane 1/4 results in the product (B-2) (yield: 540 mg (35 %))
   ¹H NMR (360 MHz, THF-d₈, δ): 8.58 (d, J = 9.0 Hz, 1H), 7.84-7.94 (m, 4H), 7.73-7.82 (m, 4H), 6.88-7.57 (m, 31 H).

### Example 5

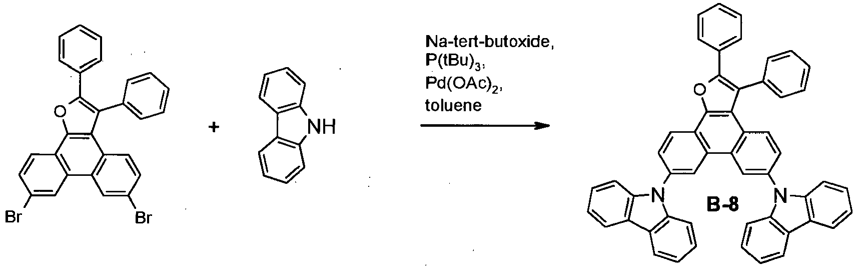

0.42 g (4.35 mmol) sodium tert-butoxide are added to 1.00 g (1.89 mmol) of the product of example **4b** in 30 ml toluene. 0.70 g (4.19 mmol) carbazole are added. The reaction mixture is degassed with argon. 21 mg (0.09 mmol) palladium(II) acetate are added. 38 mg (0.19 mmol) tri-tert-butylphosphine are added. The reaction mixture is stirred for 24 h at 100 °C under argon. The reaction mixture is filtered through a plug of silica gel, and the solvent of the filtrate is distilled off. Column chromatography on silica gel with toluene cyclohexane 1/4 results in the product (**B-8**; yield: 720 mg (54 %))
¹H NMR (400 MHz, THF-d₈, δ): 9.13 (d, J = 9.8 Hz, 2H), 8.65 (d, J = 8.6 Hz, 1H), 8.07-8.15 (m, 4H), 8.03 (d, J = 8.5 Hz, 1 H), 7.91 (d, J = 8.7 Hz, 1 H), 7.60-7.74 (m, 8H), 7.49 (d, J = 8.2 Hz, 2H), 7.14-7.42 (m, 13H).

### Example 6

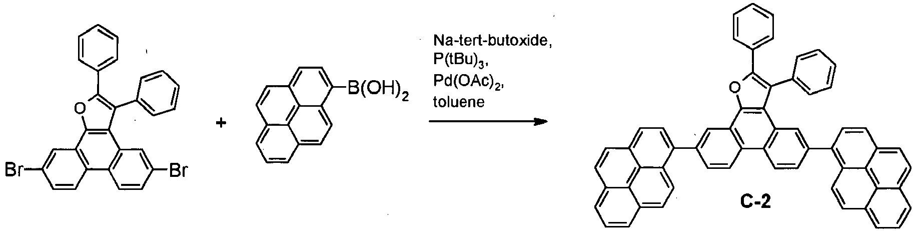

0.84 g (8.74 mmol) sodium tert-butoxide are added to 2.10 g (3.98 mmol) of the product of example **1b** in 60 ml toluene. 2.10 g (8.53 mmol) pyrene-1-boronic acid are added. The reaction mixture is degassed with argon. 43 mg (0.19 mmol) palladium(II) acetate are added. 77 mg (0.38 mmol) tri-tert-butylphosphine are added. The reaction mixture is stirred for 24 h at 100 °C under argon. Methylene chloride is added to the reaction mixture, and the undissolved residue is filtered off and discarded. The solvent of the filtrate is distilled off. Column chromatography on silica gel with n-hexane ethyl acetate 9/1 results in the product (yield: 290 mg (10 %)). MS (MALDI (pos): m/z(%)): 770 (M⁺, 100%).

### Example 7

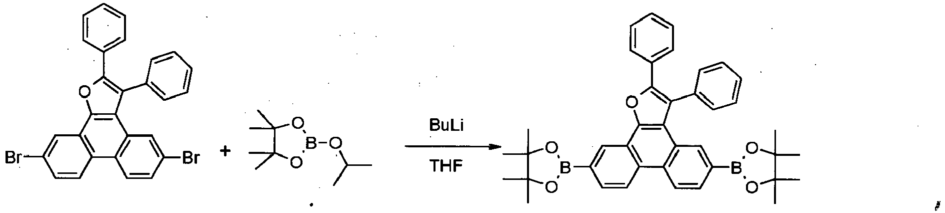

a) 7.2 ml (18.0 mmol) of a 2.5 M n-butyl lithium solution in hexane are added to 3.95 g (7.48 mmol) of the product of example 1 b) in water free tetrahydrofuran (THF) under argon at -78 °C. The reaction mixture is stirred for 30 minutes at -78 °C. 3.90 g (20.9 mmol) 2-isopropoxy-4,4',5,5'-tetramethyl-1,3,2-dioxaborolan are added. The reaction mixture is stirred for 30 minutes and is then warmed up to 25 °C, poured into water and the water phase is extracted with diethyl ether and dichloromethane. The organic phase is dried with magnesium sulfate and the solvent is distilled off. The product is used without purification for the next reaction step. 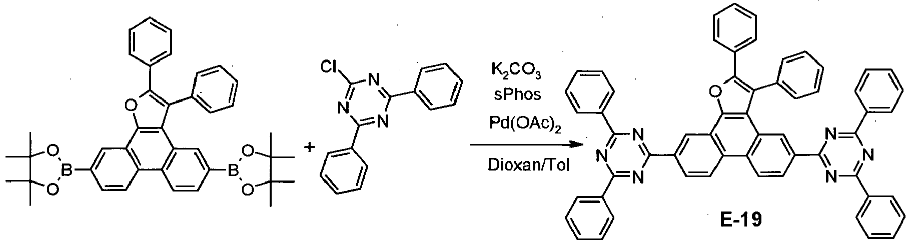
b) 10 ml of a 2M solution of potassium carbonate in water are added to 1.80 g (2.89 mmol) of the product of example 7a) and 1.70 g (6.36 mmol) 2-chlor-4,6-diphenyl-1,3,5-triazine in a mixture of 10 ml dioxane and 50 ml toluene. Then the reaction mixture is degassed with argon. 143 mg (0.35 mmol) 2-dicyclohexylphosphino-2',6'-dimethoxybiphenyl (sPhos) and 6.5 mg (0.029 mmol) palladium(II) acetate are added and the reaction mixture is stirred for 24 h at 120 °C under argon. 40 ml of a 1 % solution of sodium cyanide water are added and the reaction mixture is stirred at 100 °C for 1 h. The solvent is destilled off and the product is filtered off. The product is washed with water and ethanol. Column chromatography on silica gel with toluene, toluene/ethylacetate 10/1, toluene/ethylacetate 1/1 and toluene/ethanol 10/1 results in the ,product (**E-19**; yield: 41 mg (2 %)). MS (APCI (pos): m/z(%): 833 (M⁺¹, 100%).

## Claims

1. A compound of the formula 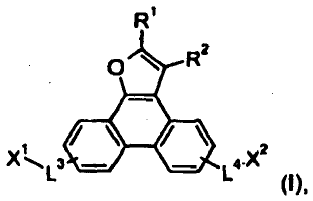 wherein
R¹ and R² are independently of each other H, F, C₁-C₁₈alkyl, C₁-C₁₈alkyl which is substituted by E and/or interrupted by D, C₆-C₂₄aryl, C₈-C₂₄aryl which is substituted by G, C₂-C₂₀heteroaryl, C₂-C₂₀heteroaryl which is substituted by G, or
R¹ and R² form together a group 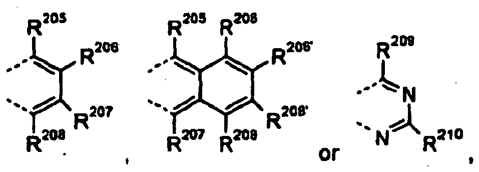 wherein R^{206'}, R^{208'}, R²⁰⁵, R²⁰⁸, R²⁰⁷, R²⁰⁸, R²⁰⁹ and R²¹⁰ are independently of each other H, C₁-C₁₈alkyl, C₁-C₁₈alkyl which is substituted by E and/or interrupted by D, C₁-C₁₈alkoxy, or C₁-C₁₈alkoxy which is substituted by E and/or interrupted by D, C₁-C₁₈fluoroalkyl, C₆-C₂₄aryl, C₆-C₂₄aryl which is substituted by G, C₂-C₂₀heteroaryl, C₂-C₂₀heteroaryl which is substituted by G, C₂-C₁₈alkenyl, C₂-C₁₈alkynyl, C₇-C₂₅aralkyl, C₇-C₂₅aralkyl which is substituted by G; CN, or -CO-R²⁸,
X¹ and X² are independently of each other a group -NA¹A^{1'},
A¹ and A^{1'} are independently of each other a C₆-C₂₄aryl group, a C₆-C₂₄aryl group, which is substituted by G; a C₂-C₂₀heteroaryl group, or a C₂-C₂₀heteroaryl group which is substituted by G; or
A¹ and A^{1'} together with the nitrogen atom to which they are bonded form a heteroaromatic ring, or ring system a C₁₀-C₂₈aryl group, which can optionally be substituted by G; and/or -L³-X¹ and -L⁴-X² are independently of each other a group of formula 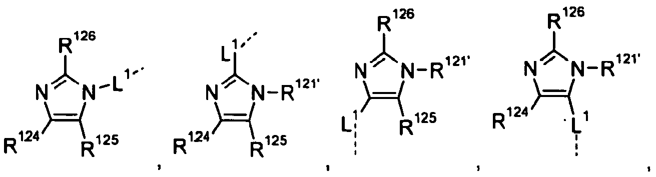 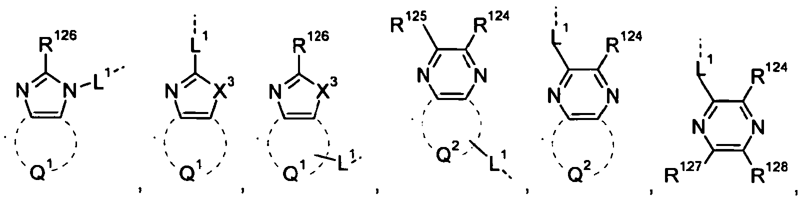 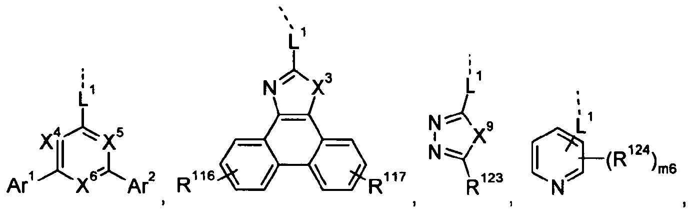 or 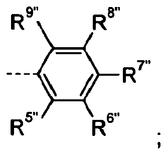 wherein
R^{5"}, R^{6"}, R^{7"}, and R^{8"} are independently of each other C₆-C₁₈aryl; which may optionally be substituted by G; or C₂-C₂₀heteroaryl, which may optionally be substituted by G, R^{9"} is hydrogen, or has the meaning of R^{5"}, m6 is an integer of 1 to 4,
X³ represents O, S or N-R^{121'},
X⁹ represents O, S or N-R¹²¹,
Q¹ and Q² represents atoms necessary for forming a carbocyclic aromatic, or heterocyclic aromatic ring, which can optionally be condensed with other ring(s) to form a condensed ring, and/or can optionally be substituted by G,
R¹¹⁶ and R¹¹⁷ are independently of each other H, halogen, -CN, C₁-C₁₈alkyl, C₁-C₁₈alkyl which is substituted by E and/or interrupted by D, C₆-C₂₄aryl, C₆-C₂₄aryl which is substituted by G, C₂-C₂₀heteroaryl, C₂-C₂₀heteroaryl which is substituted by G, C₂-C₁₈alkenyl, C₂-C₁₈alkynyl, C₁-C₁₈alkoxy, C₁-C₁₈alkoxy which is substituted by E and/or interrupted by D, C₇-C₂₅aralkyl, C₇-C₂₅aralkyl, which is substituted by G; -C(=O)-R⁷⁸, -C(=O)OR⁷⁷, or -C(=O)NR⁷⁵R⁷⁶, or
substituents R¹¹⁶ and R¹¹⁷, which are adjacent to each other, can form a ring, R⁷⁵, R⁷⁶ and R⁷⁸ are independently of each other H; C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -O-,
R⁷⁷ is C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -0-,
R^{121'} is C₆-C₁₈aryl; or C₂-C₂₀heteroaryl; which can optionally be substituted by C₁-C₁₈alkyl, C₁-C₁₈fluoroalkyl, or C₁-C₁₈alkoxy; C₁-C₁₈alkyl; or C₁-C₁₈alkyl which is interrupted by -O-;
R¹²³, R¹²⁴, R¹²⁵ and R¹²⁶ are independently of each other H, C₁-C₁₈alkyl, C₁-C₁₈alkyl which is substituted by E and/or interrupted by D, C₁-C₁₈fluoroalkyl, C₆-C₂₄aryl, which can optionally be substituted by G, C₂-C₂₀heteroaryl, which can optionally be substituted by G, C₂-C₁₈alkenyl, C₂-C₁₈alkynyl, C₁-C₁₈alkoxy, C₁-C₁₈alkoxy which is substituted by E and/or interrupted by D, or C₇-C₂₅aralkyl,
R¹²⁷ and R¹²⁸ are independently of each other H, CN , C₁-C₁₈alkyl, C₁-C₁₈alkyl which is substituted by E and/or interrupted by D, C₁-C₁₈fluoroalkyl, C₆-C₂₄aryl, which can optionally be substituted by G, C₂-C₂₀heteroaryl, which can optionally be substituted by G, C₂-C₁₈alkenyl, C₂-C₁₈alkynyl, C₁-C₁₈alkoxy, C₁-C₁₈alkoxy which is substituted by E and/or interrupted by D, or C₇-C₂₅aralkyl,
L¹ is a single bond, -(CR⁴⁷=CR⁴⁸)ₘ₂-, -(Ar³)ₘ₃-, -[Ar³(Y¹)ₘ₅]ₘ₄-, -[(Y¹)ₘ₅Ar³]ₘ₄-,
or -[Ar³(Y²)ₘ₅Ar⁴]ₘ₄-, wherein
Y¹ is -(CR⁴⁷=CR⁴⁸)-,
Y² is NR⁴⁹, O, S, C=O, C(=O)O, wherein R⁴⁹ is H; C₆-C₁₈aryl which can optionally be substituted by C₁-C₁₈alkyl, or C₁-C₁₈alkoxy; C₁-C₁₈alkyl; or C₁-C₁₈alkyl which is interrupted by -O-;
R⁴⁷ and R⁴⁸ are independently of each other hydrogen, fluorine, C₁-C₂₅alkyl, or C₆-C₂₄aryl, which can optionally be substituted by G,
m2 is an integer of 1 to 10, m3 is an integer of 1 to 5, m4 is an integer of 1 to 5, m5 is an integer of 1 to 10,
Ar¹ and Ar² are independently of each other C₆-C₂₄aryl, which can optionally be substituted by G, or C₂-C₂₀heteroaryl, which can optionally be substituted by G,
Ar³ and Ar⁴ are independently of each other arylen, or heteroarylen, which can optionally be substituted.
X⁴, X⁵ and X⁶ are independently of each other N, or CH, with the proviso that at least one of the substituents X⁴, X⁵ and X⁶ is N, and
L³ and L⁴ are independently of each other a single bond, or a bridging unit BU,
D is -CO- -COO-, -S- -SO-, -SO₂-, -O- -NR⁶⁵-, -SiR⁷⁰R⁷¹-, -POR⁷²-, -CR⁶³=CR⁶⁴-, or - C≡C-, and
E is -OR⁸⁹, -SR⁸⁹, -NR⁶⁵R⁸⁸, -COR⁶⁸, -COOR⁶⁷, -CONR⁶⁵R⁶⁶, -CN, or halogen, G is E, or C₁-C₁₈alkyl,
R⁶³ and R⁶⁴ are independently of each other C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -0-; R⁶⁵ and R⁶⁶ are independently of each other C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -0-; or
R⁶⁵ and R⁶⁶ together form a five or six membered ring,
R⁶⁷ is C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -O-,
R⁶⁸ is H; C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -0-,
R⁶⁹ is C₆-C₁₈aryl; C₆-C₁₈aryl, which is substituted by C₁-C₁₈alkyl, or C₁-C₁₈alkoxy; C₁-C₁₈alkyl; or C₁-C₁₈alkyl which is interrupted by -O-,
R⁷⁰ and R⁷¹ are independently of each other C₁-C₁₈alkyl, C₆-C₁₈aryl, or C₆-C₁₈aryl, which is substituted by C₁-C₁₈alkyl, and
R⁷² is C₁-C₁₈alkyl, C₆-C₁₈aryl, or C₆-C₁₈aryl, which is substituted by C₁-C₁₈alkyl; R⁴⁵ and R^{45'} are independently of each other a C₁-C₂₅alkyl group, a C₁-C₁₈cycloalkyl group, in which one or more carbon atoms which are not in neighbourhood to each other could be replaced by -NR^{45"}-, -O-, -S-, -C(=O)-O-, or, -O-C(=O)-O-, and/or wherein one or more hydrogen atoms can be replaced by F, a C₆-C₂₄aryl group, or a C₆-C₂₄aryloxy group, wherein one or more carbon atoms can be replaced by O, S, or N, and/or which can be substituted by one or more non-aromatic groups R⁴¹,
R^{45"} is a C₁-C₂₅alkyl group, or a C₄-C₁₈cycloalkyl group, and
m1 can be the same or different at each occurence and is 0, 1, 2, 3, or 4.

2. The compound according to claim 1, which is a compound of formula 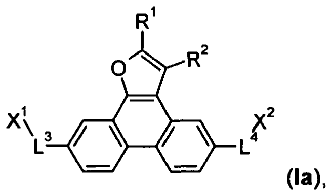 or 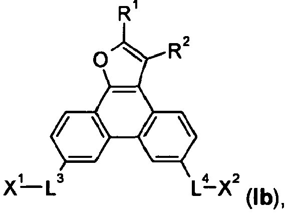 wherein X¹, X², L³, L⁴, R¹ and R² are as defined in claim 1.

3. The compound according to claim 1, or 2, wherein R¹ and R² are a group of formula 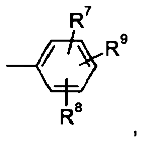 or 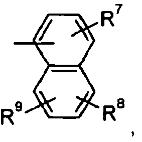 wherein R⁷, R⁸ and R⁹ are independently of each other H, C₁-C₁₈alkyl, C₁-C₁₈alkoxy, or C₁-C₁₈alkyl which is interrupted by O; or R¹ and R² form together a group 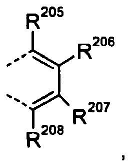 wherein R²⁰⁵, R²⁰⁶, R²⁰⁷, and R²⁰⁸ are independently of each other H, C₁-C₁₈alkyl, C₁-C₁₈alkyl which is interrupted by O, C₁-C₁₈alkoxy, or C₁-C₁₈alkoxy which is interrupted by O, or C₁-C₁₈fluoroalkyl.

4. The compound according to any of claims 1 to 3, wherein -L³-X¹ and -L ⁴-X² are independently of each other a group of formula -NA¹A^{1'}, or a group 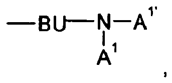 wherein
A¹ and A^{1'} are independently of each other 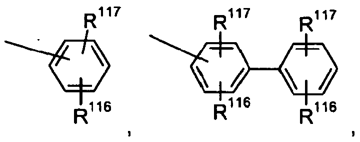 or 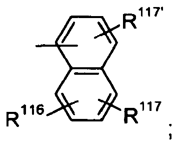 or
A¹ and A^{1'} together with the nitrogen atom to which they are bonded form a heteroaromatic ring, or ring system 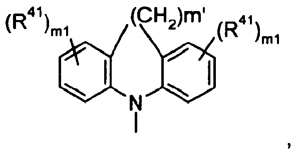 or 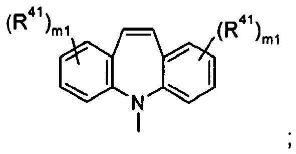 m' is 0, 1, or 2;
m1 can be the same or different at each occurence and is 0, 1, 2, 3, or 4,
R¹¹⁶, R¹¹⁷ and R^{117'} are independently of each other H, halogen -CN, C₁-C₁₈alkyl, C₁-C₁₈alkyl which is substituted by E and/or interrupted by D, C₆-C₂₄aryl, C₆-C₂₄aryl which is substituted by G, C₂-C₂₀heteroaryl, C₂-C₂₀heteroaryl which is substituted by G, C₂-C₁₈alkenyl, C₂-C₁₈alkynyl, C₁-C₁₈alkoxy, C₁-C₁₈alkoxy which is substituted by E and/or interrupted by D, C₇-C₂₅aralkyl, -C(=O)-R⁷⁷, -C(=O)OR⁷⁸, or - C(=O)NR⁷⁵R⁷⁶, or
substituents R¹¹⁶, R¹¹⁷ and R^{117'}, which are adjacent to each other, can form a ring, R⁷⁵, R⁷⁶ and R⁷⁸ are independently of each other H; C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -O-,
R⁷⁷ is C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -O-, BU is 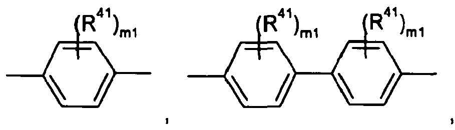 or 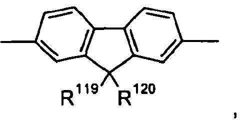 wherein m1 is as defined above;
R ⁴¹ can be the same or different at each occurence and is Cl, F, CN, NR⁴⁵R^{45'}, a C₁-C₂₅alkyl group, a C₄-C₁₈cycloalkyl group, a C₁-C₂₅alkoxy group, in which one or more carbon atoms which are not in neighbourhood to each other could be replaced by -NR⁴⁵-, -O-, -S-, -C(=O)-O-, or -O-C(=O)-O-, and/or wherein one or more hydro-gen atoms can be replaced by F, a C₆-C₂₄aryl group, or a C₆-C₂₄aryloxy group, wherein one or more carbon atoms can be replaced by O, S, or N, and/or which can be substituted by one or more non-aromatic groups R⁴¹, or
two or more groups R⁴¹ form a ring system:
R⁴⁷ and R⁴⁸ are independently of each other H, C₁-C₁₈alkyl, or C₆-C₁₀aryl, which may optionally be substituted by one, or more C₁-C₈alkyl groups, or C₁-C₈alkoxy groups;
R¹¹⁹ and R¹²⁰ are independently of each other C₁-C₁₈alkyl, C₁-C₁₈alkyl which is substituted by E and/or interrupted by D, C₆-C₂₄aryl, C₆-C₂₄aryl which is substituted by G, C₂-C₂₀heteroarvl. C₂-C₂₀heteroaryl which is substituted by G, C₂-C₁₈alkenyl, C₂-C₁₈alkynyl, C₁-C₁₈alkoxy, C₁-C₁₈alkoxy which is substituted by E and/or interrupted by D, or C₇-C₂₅aralkyl, or
R¹¹⁹ and R¹²⁰ together form a group of formula =CR¹²¹R¹²², wherein
R¹²¹ and R¹²² are independently of each other H, C₁-C₁₈alkyl, C₁-C₁₆alkyl which is substituted by E and/or interrupted by D, C₆-C₂₄aryl, C₆-C₂₄aryl which is substituted by G, or C₂-C₂₀heteroaryl, or C₂-C₂₀heteroaryl which is substituted by G, or
R¹¹⁹ and R¹²⁰ together form a five or six membered ring, which optionally can be substituted by C₁-C₁₈alkyl, C₁-C₁₈alkyl which is substituted by E and/or interrupted by D C₆-C₂₄aryl, C₆-C₂₄aryl which is substituted by G, C₂-C₂₀heteroaryl, C₂-C₂₀heteroaryl which is substituted by G, C₂-C₁₈galkenyl, C₂-C₁₈alkynyl, C₁-C₁₈alkoxy, C₁-C₁₈alkoxy which is substituted by E and/or interrupted by D, C₇-C₂₅aralkyl, or -C(=O)-R²⁸,
R²⁸ is H; C₆-C₁₈aryl;_C₆-C₁₈aryl which is substituted by C₁-C₁₈alkyl, or C₁-C₁₈alkoxy; C₁-C₁₈alkyl; or C₁-C₁₈alkyl which is interrupted by -O-, or
a group of formula 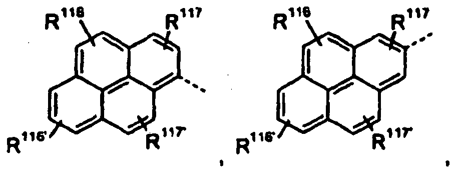 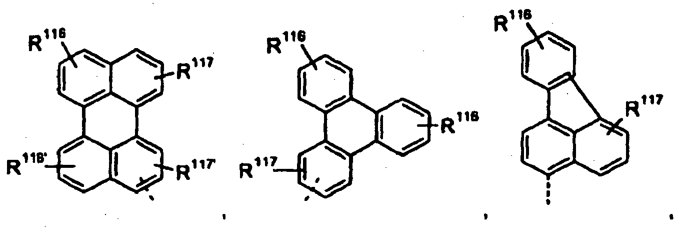 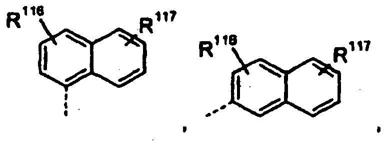 or 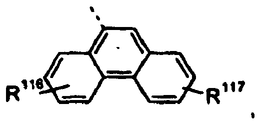 wherein
R^{116'} has the meaning of R¹¹⁶, R¹¹⁸, R¹¹⁷ and R^{117'} are as defined above, D, E and G are as defined in claim 1; or
a group of formula 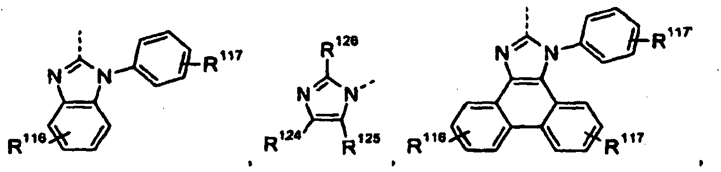 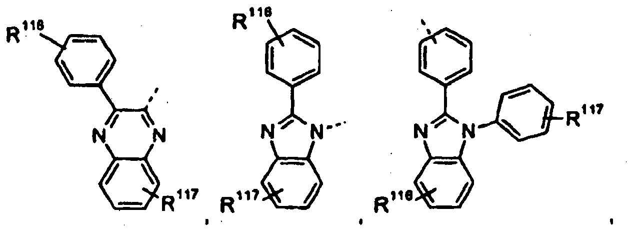 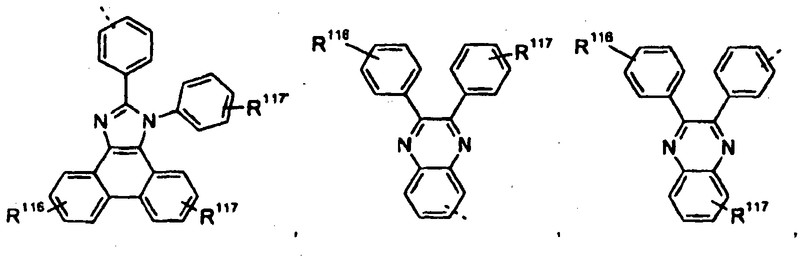 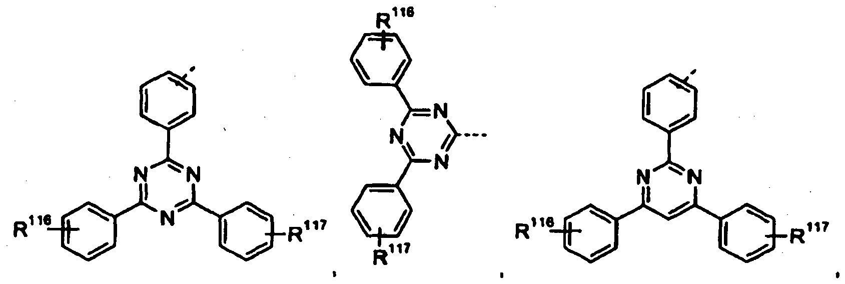 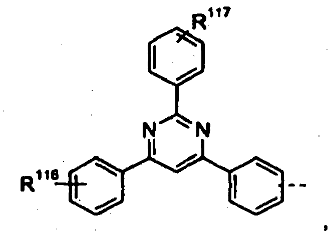 or 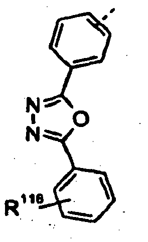 wherein R¹¹⁸, R¹¹⁷ and R^{117'} are as defined above and R¹²⁴, R¹²⁵ and R¹²⁶ are as defined in claim 1.

5. The compound according to claim 4, wherein -L³-X¹ and -L⁴-X² are independently of each other a group of formula -NA¹A^{1'}, or a group 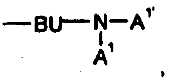 wherein
A¹ and A^{1'} are independently of each other 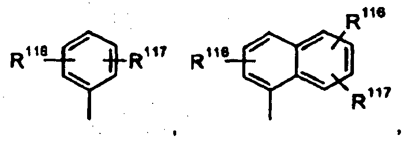 or 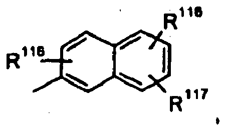 or A¹ and A^{1'} together with the nitrogen atom to which they are bonded form a group of formula 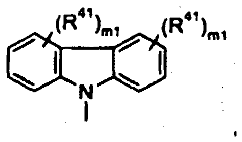 R¹¹⁶ and R¹¹⁷ are independently of each other C₁-C₁₈alkyl, which may optionally be interrupted by -O-, or C₁-C₁₈alkoxy;
Bu is 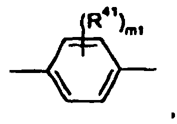 or 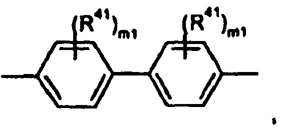 wherein R⁴¹ can be the same or different at each occurence and is C₁-C₁₈alkyl, which may optionally be interrupted by - O-, or C₁-C₁₆alkoxy; m1 is 0, 1, or 2.

6. The compound according to claim 4, wherein -L³-X¹ and -L⁴-X² are independently of each other a group of formula 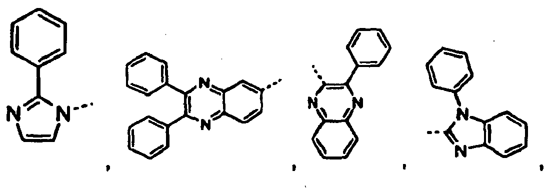 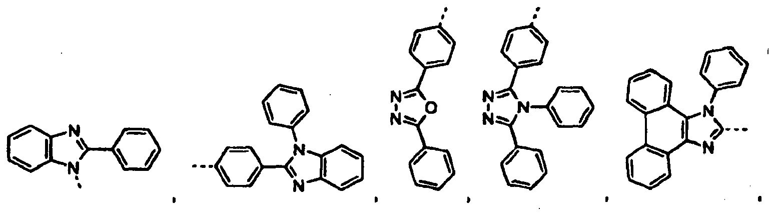 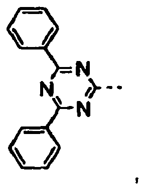 or

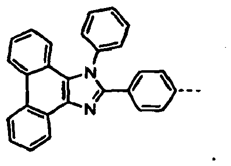

7. The compound according to claim 4, wherein -L³-X¹ and -L⁴-X² are independently of each other a group of formula 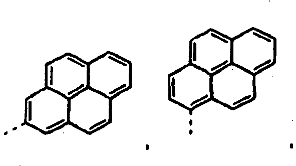 or

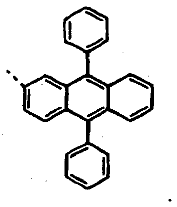

8. The compound according to any of claims 1 to 7, which is selected from
| | | | |
|---|---|---|---|
| 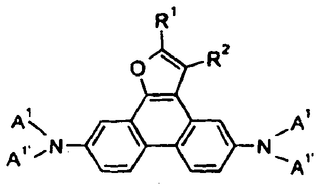 | | | |
| **Cpd.** | **R¹ = R²** | **A¹** | **A^{1'}** |
|---|---|---|---|
| **A-1** | 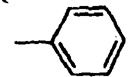 | 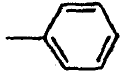 | 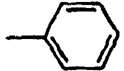 |
| **A-2** | 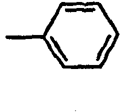 | 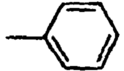 | 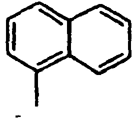 |
| **A-3** | 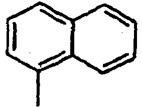 | 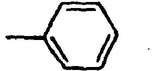 | 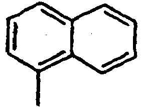 |
| **A-4** | 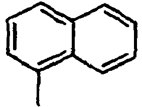 | 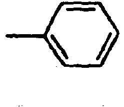 | 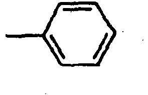 |
| **A-5** | 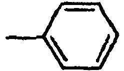 | 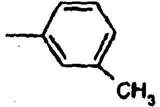 | 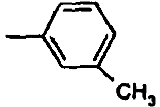 |
| **A-6** | ¹⁾ | 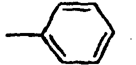 | 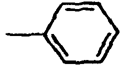 |
| **A-7** | ¹⁾ | ²⁾ | ²⁾ |
| **A-8** | 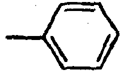 | ²⁾ | ²⁾ |
| **A-9** | 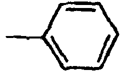 | 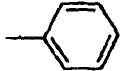 | 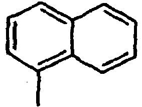 |
| | | | |
|---|---|---|---|
| 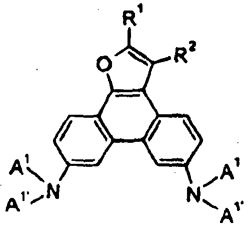 | | | |
| **Cpd.** | **R¹ = R²** | **A¹** | **A^{1'}** |
|---|---|---|---|
| **B-1** | 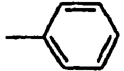 | 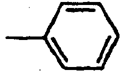 | 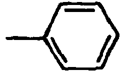 |
| **B-2** | 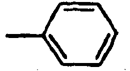 | 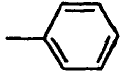 | 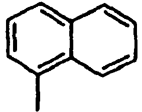 |
| **B-3** | 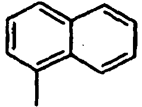 | 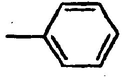 | 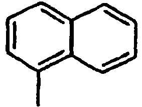 |
| **B-4** | 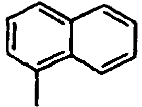 | 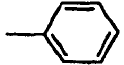 | 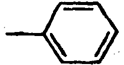 |
| **B-5** | 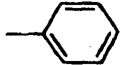 | 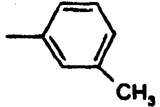 | 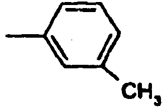 |
| **B-6** | ¹⁾ | 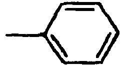 | 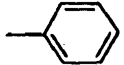 |
| **B-7** | ¹⁾ | ²⁾ | ²⁾ |
| **B-8** | 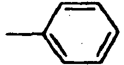 | ²⁾ | ²⁾ |
| **B-9** | 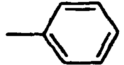 | 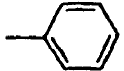 | 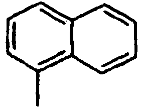 |
| | | | |
|---|---|---|---|
| 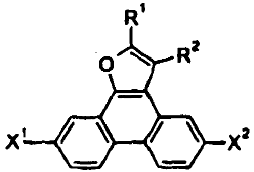 | | | |
| **Cpd.** | **R¹ = R²** | **X¹** | **X²** |
|---|---|---|---|
| **C-1** | 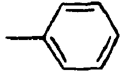 | **AR-1** | **AR-1** |
| **C-2** | 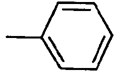 | **AR-2** | **AR-2** |
| **C-3** | 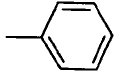 | **AR-3** | **AR-3** |
| **C-4** | ¹⁾ | **AR-1** | **AR-1** |
| **C-5** | ¹⁾ | **AR-2** | **AR-2** |
| **C-6** | ¹⁾ | **AR-3** | **AR-3** |
| | | | |
|---|---|---|---|
| 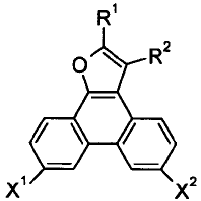 | | | |
| **Cpd.** | **R¹ = R²** | **X¹** | **X²** |
|---|---|---|---|
| **D-1** | 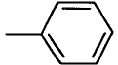 | **AR-1** | **AR-1** |
| **D-2** | 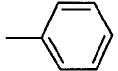 | **AR-2** | **AR-2** |
| **D-3** | 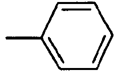 | **AR-3** | **AR-3** |
| **D-4** | ¹⁾ | **AR-1** | **AR-1** |
| **D-5** | ¹⁾ | **AR-2** | **AR-2** |
| **D-6** | ¹⁾ | **AR-3** | **AR-3** |
| | | | |
|---|---|---|---|
| 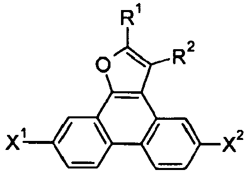 | | | |
| **Cpd.** | **R¹ = R²** | **X¹** | **X²** |
|---|---|---|---|
| **E-1** | 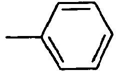 | **HE-1** | **HE-1** |
| **E-2** | 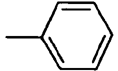 | **HE-2** | **HE-2** |
| **E-3** | 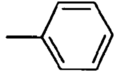 | **HE-3** | **HE-3** |
| **E-4** | 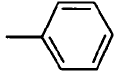 | **HE-4** | **HE-4** |
| **E-5** | 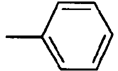 | **HE-5** | **HE-5** |
| **E-6** | 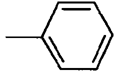 | **HE-6** | **HE-6** |
| **E-7** | 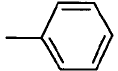 | **HE-7** | **HE-7** |
| **E-8** | 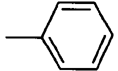 | **HE-8** | **HE-8** |
| **E-9** | 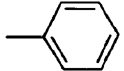 | **HE-9** | **HE-9** |
| **E-10** | ¹⁾ | **HE-1** | **HE-1** |
| **E-11** | ¹⁾ | **HE-2** | **HE-2** |
| **E-12** | ¹⁾ | **HE-3** | **HE-3** |
| **E-13** | ¹⁾ | **HE-4** | **HE-4** |
| **E-14** | ¹⁾ | **HE-5** | **HE-5** |
| **E-15** | ¹⁾ | **HE-6** | **HE-6** |
| **E-16** | ¹⁾ | **HE-7** | **HE-7** |
| **E-17** | ¹⁾ | **HE-8** | **HE-8** |
| **E-18** | ¹⁾ | **HE-9** | **HE-9** |
| **E-19** | 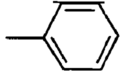 | **HE-10** | **HE-10** |
| **E-20** | ¹⁾ | **HE-10** | **HE-10** |
| | | | |
|---|---|---|---|
| 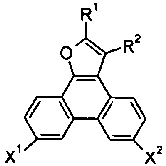 | | | |
| **Cpd.** | **R¹ = R²** | **X¹** | **X²** |
|---|---|---|---|
| **F-1** | 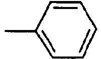 | **HE-1** | **HE-1** |
| **F-2** | 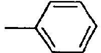 | **HE-2** | **HE-2** |
| **F-3** | 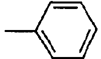 | **HE-3** | **HE-3** |
| **F-4** | 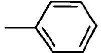 | **HE-4** | **HE-4** |
| **F-5** | 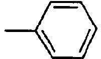 | **HE-5** | **HE-5** |
| **F-6** | 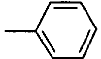 | **HE-6** | **HE-6** |
| **F-7** | 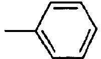 | **HE-7** | **HE-7** |
| **F-8** | 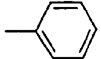 | **HE-8** | **HE-8** |
| **F-9** | 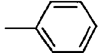 | **HE-9** | **HE-9** |
| **F-10** | ¹⁾ | **HE-1** | **HE-1** |
| **F-11** | ¹⁾ | **HE-2** | **HE-2** |
| **F-12** | ¹⁾ | **HE-3** | **HE-3** |
| **F-13** | ¹⁾ | **HE-4** | **HE-4** |
| **F-14** | ¹⁾ | **HE-5** | **HE-5** |
| **F-15** | ¹⁾ | **HE-6** | **HE-6** |
| **F-16** | ¹⁾ | **HE-7** | **HE-7** |
| **F-17** | ¹⁾ | **HE-8** | **HE-8** |
| **F-18** | ¹⁾ | **HE-9** | **HE-9** |
| **F-19** | 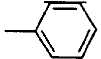 | **HE-10** | **HE-10** |
| **F-20** | ¹⁾ | **HE-10** | **HE-10** |
| | | | |
|---|---|---|---|
| ¹⁾ R¹ and R² together form a group 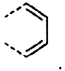 ²⁾ A¹ and A^{1'} together form a group 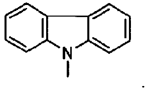 **AR-1 is** 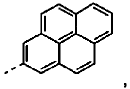 **AR-2 is** 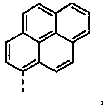 **AR-3 is** 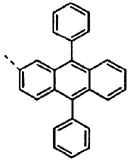 **HE-1 is** 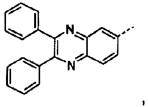 **HE-2 is** 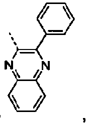 **HE-3 is** 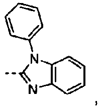 **HE-4** is 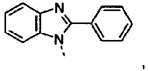 **HE-5 is** 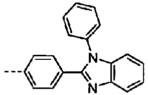 **HE-6 is** 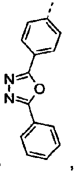 **HE-7 is** 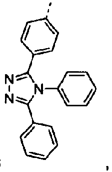 **HE-8 is** 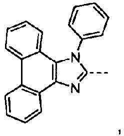 **HE-9 is** 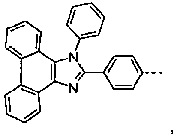 **HE-10 is** 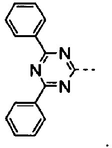 | | | |

9. An electronic device, comprising a compound according to any of claims 1 to 8.

10. The electronic device according to claim 9, which is an electroluminescent device.

11. A hole transport layer, or an electron transport layer, comprising a compound according to any of claims 1 to 8.

12. An apparatus selected from the group consisting of stationary visual display units, and mobile visual display units; illumination units; keyboards; items of clothing; furniture; wallpaper, comprising the organic electronic device according to claim 9, or 10, or the hole transport, or electron transport layer according to claim 11.

13. Use of the compounds of formula **I** according to claim 1 for electrophotographic photoreceptors, photoelectric converters, organic solar cells, switching elements, organic light emitting field effect transistors, image sensors, dye lasers and electroluminescent devices.

14. A process for the preparation of compounds of the formula I, wherein X¹ and X² are independently of each other -NA¹A^{1'}, 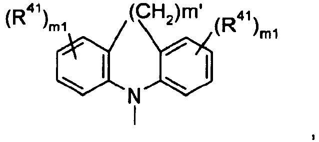 or 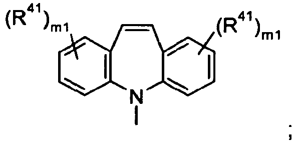 m' is 0, 1, or 2; which comprises reacting a compound of formula 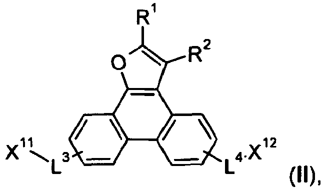 wherein X¹¹ and X¹² stand for halogen, with a compound of formula HNA¹A^{1'}, 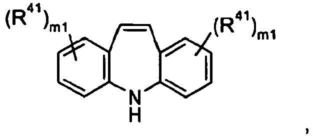 or 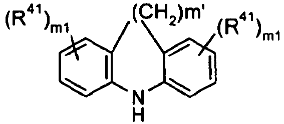 in the presence of a base and a catalyst in a solvent, wherein A¹, A^{1'}, L³, L⁴, R¹, R², R⁴¹ and m1 are as defined in claim 1.

15. A compound of the formula 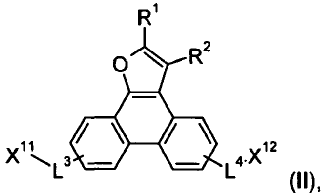 wherein L³, L⁴, R¹ and R² are as defined in claim 1; X¹¹ and X¹² are independently in each occurrence a halogen atom, or -OS(O)₂CF₃, -OS(O)₂-aryl, -OS(O)₂CH₃, -B(OH)₂, -B(OY¹)₂, 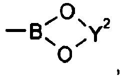 -BF₄Na, or -BF₄K, wherein Y¹ is independently in each occurrence a C₁-C₁₀alkyl group and Y² is independently in each occurrence a C₂-C₁₀alkylene group.

## Patentansprüche

1. Verbindung der Formel 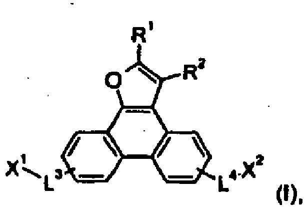 wobei
R¹ und R² unabhängig voneinander H, F, C₁-C₁₈Alkyl, C₁-C₁₈Alkyl, das mit E substituiert und/oder von D unterbrochen ist, C₆-C₂₄Aryl, C₆-C₂₄Aryl, das mit G substituiert ist, C₂-C₂₀Heteroaryl, C₂-C₂₀Heteroaryl, das mit G substituiert ist, sind oder
R¹ und R² zusammen eine Gruppe 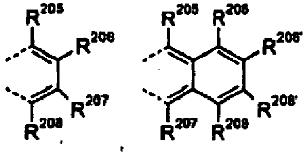 oder 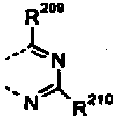 bilden, wobei R^{206'}, R^{208'}, R²⁰⁵, R²⁰⁶, R²⁰⁷, R²⁰⁸, R²⁰⁹ und R²¹⁰ unabhängig voneinander H, C₁-C₁₈Alkyl, C₁-C₁₈Alkyl, das mit E substituiert und/oder von D unterbrochen ist, C₁-C₁₈Alkoxy oder C₁-C₁₈Alkoxy, das mit E substituiert und/oder von D unterbrochen ist, C₁-C₁₈Fluoralkyl, C₆-C₂₄Aryl, C₆-C₂₄Aryl, das mit G substituiert ist, C₂-C₂₀Heteroaryl, C₂-C₂₀Heteroaryl, das mit G substituiert ist, C₂-C₁₈Alkenyl, C₂-C₁₈Alkinyl, C₇-C₂₅Aralkyl, C₇-C₂₅Aralkyl, das mit G substituiert ist; CN oder -CO-R²⁸ sind,
X¹ und X² unabhängig voneinander eine Gruppe -NA¹A^{1'} sind,
A¹ und A^{1'} unabhängig voneinander eine C₆-C₂₄Arylgruppe, eine C₆-C₂₄Arylgruppe, die mit G substituiert ist; eine C₂-C₂₀Heteroarylgruppe oder eine C₂-C₂₀Heteroarylgruppe, die mit G substituiert ist, sind; oder
A¹ und A^{1'} zusammen mit dem Stickstoffatom, an das sie gebunden sind, einen heteroaromatischen Ring oder ein heteroaromatisches Ringsystem; eine C₁₀-C₂₈Arylgruppe, die gegebenenfalls mit G substituiert sein kann, bilden; und/oder -L³-X¹ und - L⁴-X² unabhängig voneinander eine Gruppe der Formel 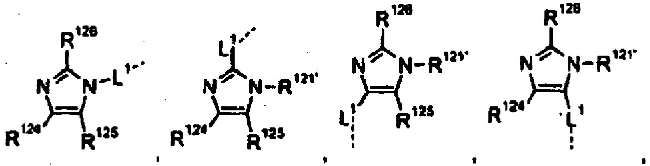 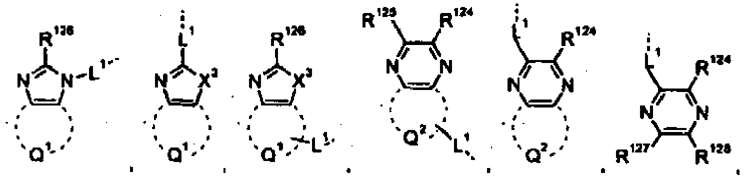 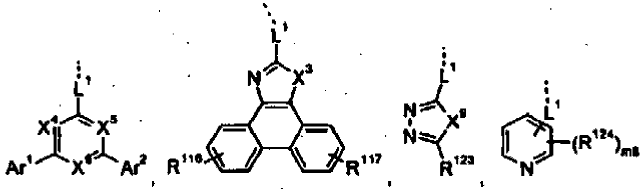 oder 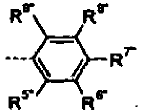 sind;
wobei
R^{5"}, R^{6"}, R^{7"} und R^{8"} unabhängig voneinander C₆-C₁₈Aryl; das gegebenenfalls mit G substituiert sein kann; oder C₂-C₂₀Heteroaryl, das gegebenenfalls mit G substituiert sein kann, sind,
R^{9"} Wasserstoff ist oder die Bedeutung von R^{5"} hat, m6 eine ganze Zahl von 1 bis 4 ist,
X³ O, S oder N-R^{121'} darstellt,
X⁹ O, S oder N-R^{121'} darstellt,
Q¹ und Q² Atome darstellen, die zum Bilden eines carbocyclischen aromatischen oder heterocyclischen aromatischen Rings erforderlich sind, der gegebenenfalls mit anderen Ringen kondensiert sein kann, um einen kondensierten Ring zu bilden, und/oder gegebenenfalls mit G substituiert sein kann,
R¹¹⁶ und R¹¹⁷ unabhängig voneinander H, Halogen, -CN, C₁-C₁₈Alkyl, C₁-C₁₈Alkyl, das mit E substituiert und/oder von D unterbrochen ist, C₆-C₂₄Aryl, C₆-C₂₄Aryl, das mit G substituiert ist, C₂-C₂₀Heteroaryl, C₂-C₂₀Heteroaryl, das mit G substituiert ist, C₂-C₁₈Alkenyl, C₂-C₁₈Alkinyl, C₁-C₁₈Alkoxy, C₁-C₁₈Alkoxy, das mit E substituiert und/oder von D unterbrochen ist, C₇-C₂₅Aralkyl, C₇-C₂₅Aralkl, das mit G substituiert ist; -C (=O) -R⁷⁸, -C (=O) OR⁷⁷ oder -C (=O) NR⁷⁵R⁷⁶ sind oder
Substituenten R¹¹⁶ und R¹¹⁷, die einander benachbart sind, einen Ring bilden können,
R⁷⁵, R⁷⁶ und R⁷⁸ unabhängig voneinander H; C₆-C₁₈Aryl; C₆-C₁₈Aryl, das mit C₁-C₁₈Alkyl oder C₁-C₁₈Alkoxy substituiert ist; C₁-C₁₈Alkyl; oder C₁-C₁₈Alkyl, das von -0- unterbrochen ist, sind,
R⁷⁷ C₆-C₁₈Aryl; C₆-C₁₈Aryl, das mit C₁-C₁₈Alkyl oder C₁-C₁₈Alkoxy substituiert ist; C₁-C₁₈Alkyl; oder C₁-C₁₈Alkyl, das von -0- unterbrochen ist, ist,
R^{121'} C₆-C₁₈Aryl; oder C₂-C₂₀Heteroaryl, das gegebenenfalls mit C₁-C₁₈Alkyl, C₁-C₁₈Fluoralkyl oder C₁-C₁₈Alkoxy substituiert sein kann; C₁-C₁₈Alkyl; oder C₁-C₁₈Alkyl, das von -O- unterbrochen ist, ist;
R¹²³, R¹²⁴, R¹²⁵ und R¹²⁶ unabhängig voneinander H, C₁-C₁₈Alkyl, C₁-C₁₈Alkyl, das mit E substituiert und/oder von D unterbrochen ist, C₁-C₁₈Fluoralkyl, C₆-C₂₄Aryl, das gegebenenfalls mit G substituiert sein kann, C₂-C₂₀Heteroaryl, das gegebenenfalls mit G substituiert sein kann, C₂-C₁₈Alkenyl, C₂-C₁₈Alkinyl, C₁-C₁₈Alkoxy, C₁-C₁₈Alkoxy, das mit E substituiert und/oder von D unterbrochen ist, oder C₇-C₂₅Aralkyl sind,
R¹²⁷ und R¹²⁸ unabhängig voneinander H, CN, C₁-C₁₈Alkyl, C₁-C₁₈Alkyl, das mit E substituiert und/oder von D unterbrochen ist, C₁-C₁₈Fluoralkyl, C₆-C₂₄Aryl, das gegebenenfalls mit G substituiert sein kann, C₂-C₂₀Heteroaryl, das gegebenenfalls mit G substituiert sein kann, C₂-C₁₈Alkenyl, C₂-C₁₈Alkinyl, C₁-C₁₈Alkoxy, C₁-C₁₈Alkoxy, das mit E substituiert und/oder von D unterbrochen ist, oder C₇-C₂₅Aralkyl sind,
L¹ eine Einfachbindung, - (CR⁴⁷=CR⁴⁸)ₘ₂-, - (Ar³)ₘ₃-, -[AR³(Y¹)]ₘ₅]ₘ₄-, -[(Y¹)ₘ₅Ar³]ₘ₄- oder - [AR³ (Y²)ₘ₅Ar⁴]ₘ₄-ist, wobei
Y¹ - (CR⁴⁷=CR⁴⁸) - ist,
Y² NR⁴⁹, 0, S, C=O, C(=O)O ist, wobei R⁴⁹ H; C₆-C₁₈Aryl, das gegebenenfalls mit C₁-C₁₈Alkyl oder C₁-C₁₈Alkoxy substituiert sein kann; C₁-C₁₈Alkyl; oder C₁-C₁₈Alkyl, das von -O- unterbrochen ist, ist;
R⁴⁷ und R⁴⁸ unabhängig voneinander Wasserstoff, Fluor, C₁-C₂₅Alkyl oder C₆-C₂₄Aryl, das gegebenenfalls mit G substituiert sein kann, sind,
m2 eine ganze Zahl von 1 bis 10 ist, m3 eine ganze Zahl von 1 bis 5 ist, m4 eine ganze Zahl von 1 bis 5 ist, m5 eine ganze Zahl von 1 bis 10 ist,
Ar¹ und Ar² unabhängig voneinander C₆-C₂₄Aryl, das gegebenenfalls mit G substituiert sein kann, oder C₂-C₂₀Heteroaryl, das gegebenenfalls mit G substituiert sein kann, sind,
Ar³ und Ar⁴ unabhängig voneinander Arylen oder Heteroarylen sind, die gegebenenfalls substituiert sein können,
X⁴, X⁵ und X⁶ unabhängig voneinander N oder CH sind, mit der Maßgabe, dass wenigstens einer der Substituenten X⁴, X⁵ und X⁶ N ist, und
L³ und L⁴ unabhängig voneinander eine Einfachbindung oder eine verbrückende Einheit BU sind,
D -CO-, -COO-, -S-, -SO-, -SO₂-, -O-, -NR⁶⁵-, -SiR⁷⁰R⁷¹, -POR⁷²-, -CR⁶³=CR⁶⁴- oder -C=C- ist und
E -OR⁶⁹, -SR⁶⁹, -NR⁶⁵R⁶⁶, -COR⁶⁸, -COOR⁶⁷, -CONR⁶⁵R⁶⁶, -CN oder Halogen ist,
G E oder C₁-C₁₈Alkyl ist,
R⁸³ und R⁶⁴ unabhängig voneinander C₆-C₁₈Aryl; C₆-C₁₈Aryl, das mit C₁-C₁₈Alkyl oder C₁-C₁₈Alkoxy substituiert ist; C₁-C₁₈Alkyl; oder C₁-C₁₈Alkyl, das von -0- unterbrochen ist, sind;
R⁶⁵ und R⁶⁸ unabhängig voneinander C₆-C₁₈Aryl; C₆-C₁₈Aryl, das mit C₁-C₁₈Alkyl oder C₁C₁₈Alkoxy substituiert ist; C₁-C₁₈Alkyl; oder C₁-C₁₈Alkyl, das von -O- unterbrochen ist, sind; oder
R⁶⁵ und R⁶⁸ zusammen einen fünf- oder sechsgliedrigen Ring bilden,
R⁶⁷ C₆-C₁₈Aryl; C₆-C₁₈Aryl, das mit C₁-C₁₈Alkyl oder C₁-C₁₈Alkoxy substituiert ist; C₁-C₁₈Alkyl; oder C₁-C₁₈Alkyl, das von -O- unterbrochen ist, ist,
R⁶⁸ H, C₆-C₁₈Aryl; C₆-C₁₈Aryl, das mit C₁-C₁₈Alkyl oder C₁-C₁₈Alkoxy substituiert ist; C₁-C₁₈Alkyl; oder C₁-C₁₈Alkyl, das von -O- unterbrochen ist, ist,
R⁶⁹ C₆-C₁₈Aryl; C₆-C₁₈Aryl, das mit C₁-C₁₈Alkyl oder C₁-C₁₈Alkoxy substituiert ist; C₁-C₁₈Alkyl; oder C₁-C₁₈Alkyl, das von -O- unterbrochen ist, ist,
R⁷⁰ und R⁷¹ unabhängig voneinander C₁-C₁₈Akyl, C₆-C₁₈Aryl oder C₆-C₁₈Aryl, das mit C₁-C₁₈Alkyl substituiert ist, sind, und
R⁷² C₁-C₁₈Alkyl, C₆-C₁₈Aryl oder C₆-C₁₈Aryl, das mit C₁-C₁₈Alkyl substituiert ist, ist,
R⁴⁵ und R^{45'} unabhängig voneinander eine C₁-C₂₅Alkylgruppe, eine C₄-C₁₈Cycloalkylgruppe, bei der ein oder mehrere Kohlenstoffatome, die einander nicht benachbart sind, durch -NR^{45"}-, -O-, -S-, -C(=O)-O- oder -O-C(=O)-O- ersetzt sein könnten und/oder bei der ein oder mehrere Wasserstoffatome durch F ersetzt sein können, eine C₆-C₂₄Arylgruppe oder eine C₆-C₂₄Aryloxygruppe, bei der ein oder mehrere Kohlenstoffatome durch O, S oder N ersetzt sein können und/oder die mit einer oder mehreren nichtaromatischen Gruppen R⁴¹ substituiert sein können,
R^{45"} eine C₁-C₂₅Alkylgruppe oder eine C₄-C₁₈Cycloalkylgruppe ist und
m1 bei jedem Auftreten gleich oder verschieden sein kann und 0, 1, 2, 3 oder 4 ist.

2. Verbindung gemäß Anspruch 1, die eine Verbindung der Formel 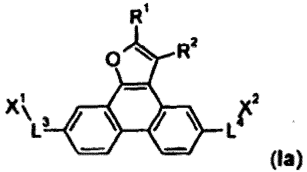 oder 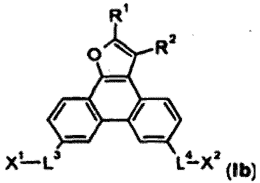 ist, wobei X¹, X², L³, L⁴, R¹ und R² wie in Anspruch 1 definiert sind.

3. Verbindung gemäß Anspruch 1 oder 2, wobei R¹ und R² eine Gruppe der Formel 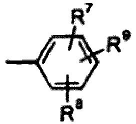 oder 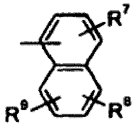 sind, wobei R⁷, R⁸ und R⁹ unabhängig voneinander H, C₁-C₁₈Alkyl, C₁-C₁₈Alkoxy oder C₁-C₁₈Alkyl, das von O unterbrochen ist, sind; oder R¹ und R² zusammen eine Gruppe 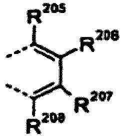 bilden, wobei R²⁰⁵, R²⁰⁶, R²⁰⁷ und R²⁰⁸ unabhängig voneinander H, C₁-C₁₈Alkyl, C₁-C₁₈Alkyl, das von O unterbrochen ist, C₁-C₁₈Alkoxy oder C₁-C₁₈Alkoxy, das von O unterbrochen ist, oder C₁-C₁₈Fluoralkyl sind.

4. Verbindung gemäß einem der Ansprüche 1 bis 3, wobei -L³-X¹ und -L⁴-X² unabhängig voneinander eine Gruppe der Formel -NA¹A^{1'} oder eine Gruppe 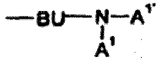 sind, wobei A¹ und A^{1'} unabhängig voneinander 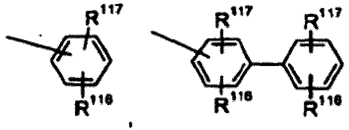 oder 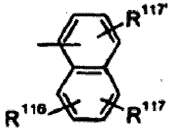 sind;
oder
A¹ und A^{1'} zusammen mit dem Stickstoffatom, an das sie gebunden sind, einen heteroaromatischen Ring oder das Ringsystem 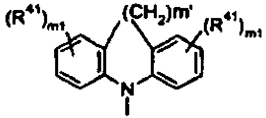 oder 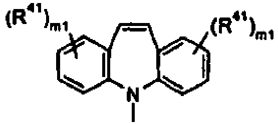 bilden; m' 0, 1 oder 2 ist;
m1 bei jedem Auftreten gleich oder verschieden sein kann und 0, 1, 2, 3 oder 4 ist,
R¹¹⁶, R¹¹⁷ und R^{117'} unabhängig voneinander H, Halogen, -CN, C₁-C₁₈Alkyl, C₁-C₁₈Alkyl, das mit E substituiert und/oder von D unterbrochen ist, C₆-C₂₄Aryl, C₆-C₂₄Aryl, das mit G substituiert ist, C₂-C₂₀Heteroaryl, C₂-C₂₀Heteroaryl, das mit G substituiert ist, C₂-C₁₈Alkenyl, C₂-C₁₈Alkinyl, C₁-C₁₈Alkoxy, C₁-C₁₈Alkoxy, das mit E substituiert und/oder von D unterbrochen ist, C₇-C₂₅Aralkyl, -C((=O)-R⁷⁷, -C (=O)OR⁷⁸ oder -C(=O)NR⁷⁵R⁷⁶ sind oder
Substituenten R¹¹⁶, R¹¹⁷ und R^{117'}, die einander benachbart sind, einen Ring bilden können,
R⁷⁵, R⁷⁶ und R⁷⁸ unabhängig voneinander H; ; C₆-C₁₈Aryl; C₆-C₁₈Aryl, das mit C₁-C₁₈Alkyl oder C₁-C₁₈Alkoxy substituiert ist; C₁-C₁₈Alkyl; oder C₁-C₁₈Alkyl, das von -0- unterbrochen ist, sind,
R⁷⁷ C₆-C₁₈Aryl; C₆-C₁₈Aryl, das mit C₁-C₁₈Alkyl oder C₁-C₁₈Alkoxy substituiert ist; C₁-C₁₈Alkyl; oder C₁-C₁₈Alkyl, das von -O- unterbrochen ist, ist,
BU 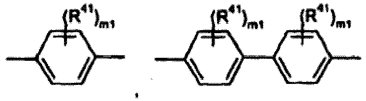 oder 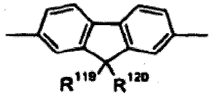 ist, wobei m1 wie vorstehend definiert ist;
R⁴¹ bei jedem Auftreten gleich oder verschieden sein kann und Cl, F, CN, NR⁴⁵R^{45'}, eine C₁-C₂₅Alkylgruppe, eine C₄-C₁₈Cycloalkylgruppe, eine C₁-C₂₅Alkoxygruppe, bei der ein oder mehrere Kohlenstoffatome, die einander nicht benachbart sind, durch -NR⁴⁵-, -O-, -S-, -C(=O)-O- oder -O-C(=O)-O- ersetzt sein könnten und/oder bei der ein oder mehrere Wasserstoffatome durch F ersetzt sein können, eine C₆-C₂₄Arylgruppe oder eine C₆-C₂₄Aryloxygruppe, bei der ein oder mehrere Kohlenstoffatome durch 0, S oder N ersetzt sein können und/oder die mit einer oder mehreren nichtaromatischen Gruppen R⁴¹ substituiert sein können, oder
zwei oder mehrere Gruppen R⁴¹ ein Ringsystem bilden;
R⁴⁷ und R⁴⁸ unabhängig voneinander H, C₁-C₁₈Alkyl oder C₆-C₁₀Aryl, das gegebenenfalls mit einer oder mehreren C₁-C₈Alkylgruppen oder C₁-C₈Alkoxygruppen substituiert sein kann, sind;
R¹¹⁹ und R¹²⁰ unabhängig voneinander C₁-C₁₈Alkyl, C₁-C₁₈Alkyl, das mit E substituiert und/oder von D unterbrochen ist, C₆-C₂₄Aryl, C₆-C₂₄Aryl, das mit G substituiert ist, C₂-C₂₀Heteroaryl, C₂-C₂₀Heteroaryl, das mit G substituiert ist, C₂-C₁₈Alkenyl, C₂-C₁₈Akinyl, C₁-C₁₈Alkoxy, C₁-C₁₈Alkoxy, das mit E substituiert und/oder von D unterbrochen ist, oder C₇-C₂₅Aralkyl sind oder
R¹¹⁹ und R¹²⁰ zusammen eine Gruppe der Formel =CR¹²¹R¹²² bilden, wobei
R¹²¹ und R¹²² unabhängig voneinander H, C₁-C₁₈Alkyl, C₁-C₁₈Alkyl, das mit E substituiert und/oder von D unterbrochen ist, C₆-C₂₄Aryl, C₆-C₂₄Aryl, das mit G substituiert ist, C₂-C₂₀Heteroaryl, C₂-C₂₀Heteroaryl, das mit G substituiert ist, sind oder
R¹¹⁹ und R¹²⁰ zusammen einen fünf- oder sechsgliedrigen Ring bilden, der gegebenenfalls substituiert sein kann mit C₁-C₁₈Alkyl, C₁-C₁₈Alkyl, das mit E substituiert und/oder von D unterbrochen ist, C₆-C₂₄Aryl, C₆-C₂₄Aryl, das mit G substituiert ist, C₂-C₂₀Heteroaryl, C₂-C₂₀Heteroaryl, das mit G substituiert ist, C₂-C₁₈Alkenyl, C₂-C₁₈Alkinyl, C₁-C₁₈Alkoxy, C₁-C₁₈Alkoxy, das mit E substituiert und/oder von D unterbrochen ist, C₇-C₂₅Aralkyl oder -C (=O) -R²⁸,
R²⁸ H; C₆-C₁₈Aryl; C₆-C₁₈Aryl, das mit C₁-C₁₈Alkyl oder C₁-C₁₈Alkoxy substituiert ist; C₁-C₁₈Alkyl; oder C₁-C₁₈Alkyl, das von -O- unterbrochen ist, ist; oder
eine Gruppe der Formel 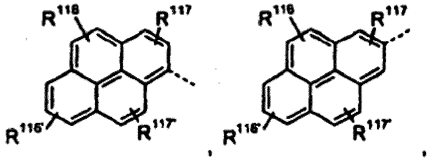 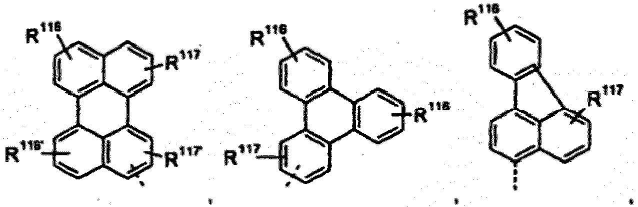 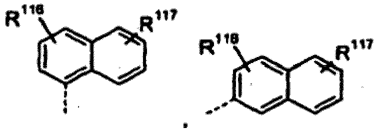 oder 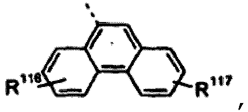 wobei
R^{116'} die vorstehend definierte Bedeutung von R¹¹⁶, R¹¹⁸, R¹¹⁷ und R^{117'} hat, D, E und G wie in Anspruch 1 definiert sind; oder
eine Gruppe der Formel 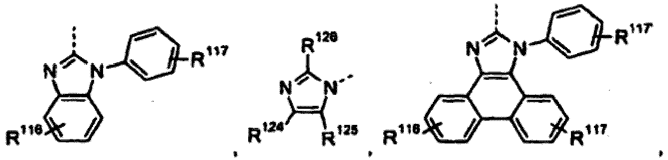 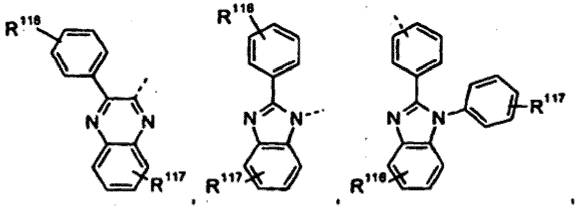 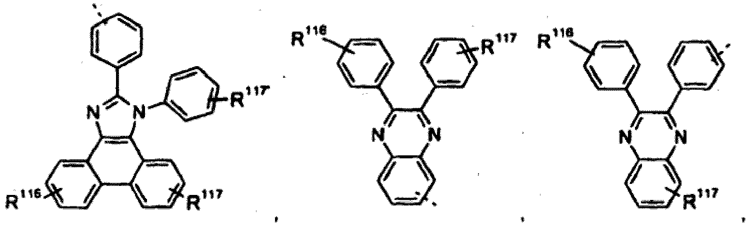 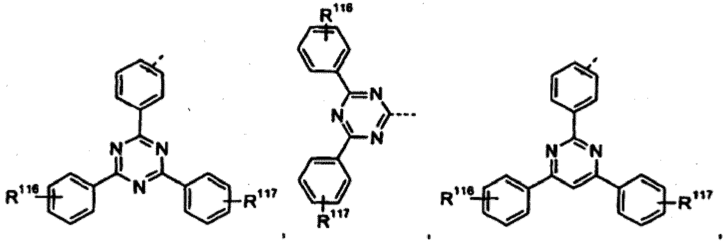 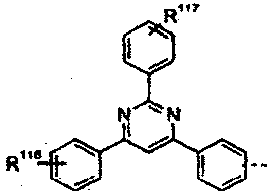 oder 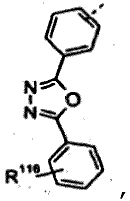 wobei R¹¹⁸, R¹¹⁷ und R^{117'} wie vorstehend definiert sind und R¹²⁴, R¹²⁵ und R¹²⁶ wie in Anspruch 1 definiert sind.

5. Verbindung gemäß Anspruch 4, wobei -L³-X¹ und -L⁴-X² unabhängig voneinander eine Gruppe der Formel -NA¹A^{1'} oder eine Gruppe 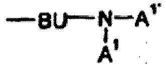 sind, wobei A¹ und A^{1'} unabhängig voneinander 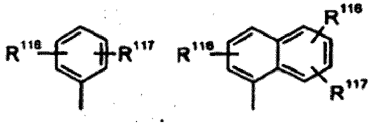 oder 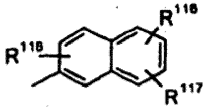 sind oder A¹ und A^{1'} zusammen mit dem Stickstoffatom, an das sie gebunden sind, eine Gruppe der Formel 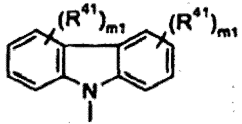 bilden,
R¹¹⁸ und R¹¹⁷ unabhängig voneinander C₁-C₁₈Alkyl, das gegebenenfalls von -0- unterbrochen sein kann, oder C₁-C₁₈Alkoxy sind;
Bu 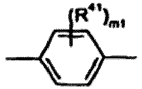 oder 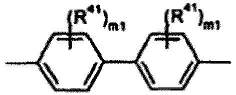 ist, wobei R⁴¹ bei jedem Auftreten gleich oder verschieden sein kann und C₁-C₁₈Alkyl, das gegebenenfalls von -0-unterbrochen sein kann, oder C₁-C₁₈Alkoxy ist; m1 0, 1 oder 2 ist.

6. Verbindung gemäß Anspruch 4, wobei -L³-X¹ und -L⁴-X² unabhängig voneinander eine Gruppe der Formel 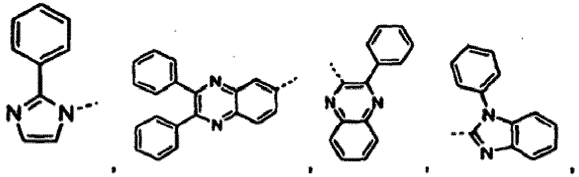 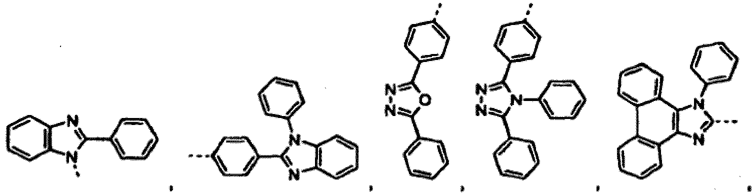 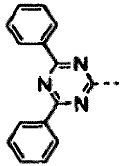 oder 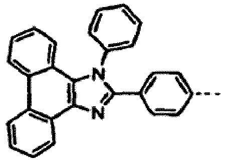 sind.

7. Verbindung gemäß Anspruch 4, wobei -L³-X¹ und -L⁴-X² unabhängig voneinander eine Gruppe der Formel 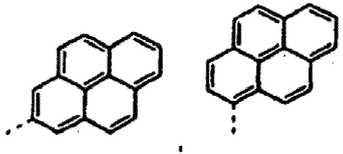 oder 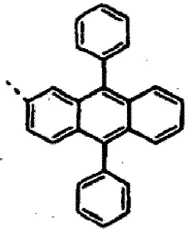 sind.

8. Verbindung gemäß einem der Ansprüche 1 bis 7, die ausgewählt ist aus
| | | | |
|---|---|---|---|
| 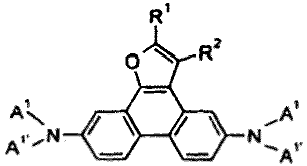 | | | |
| Verbindg. | **R¹=R²** | **A¹** | **A¹** |
|---|---|---|---|
| **A-1** | 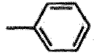 | 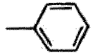 | 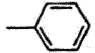 |
| **A.2** | 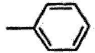 | 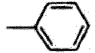 | 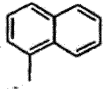 |
| **A-3** | 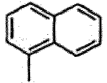 | 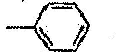 | 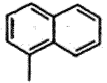 |
| **A-4** | 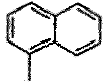 | 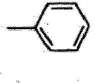 | 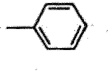 |
| **A-5** | 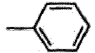 | 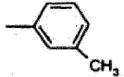 | 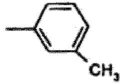 |
| **A-6** | | 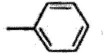 | 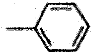 |
| **A-7** | ¹⁾ | ²⁾ | ²⁾ |
| **A-8** | 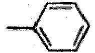 | ²⁾ | ²⁾ |
| **A-9** | 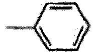 | 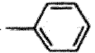 | 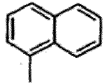 |
| | | | |
|---|---|---|---|
| 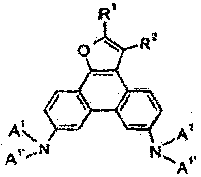 | | | |
| **Verbindg.** | **R¹=R²** | **A¹** | **A^{1'}** |
|---|---|---|---|
| **B-1** | 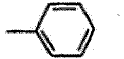 | 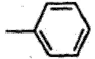 | 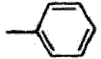 |
| **B-2** | 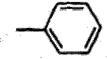 | 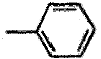 | 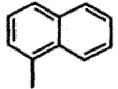 |
| **B-3** | 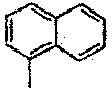 | 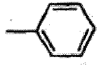 | 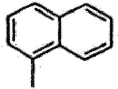 |
| **B-4** | 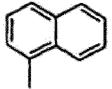 | 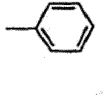 | 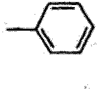 |
| **B-5** | 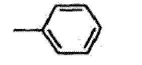 | 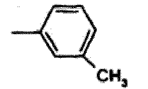 | 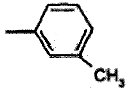 |
| **B-6** | ¹⁾ | 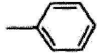 | 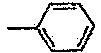 |
| **B-7** | ¹⁾ | ²⁾ | ²⁾ |
| **B-8** | 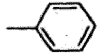 | ²⁾ | ²⁾ |
| **B-9** | 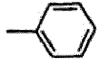 | 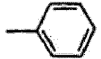 | 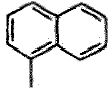 |
| | | | |
|---|---|---|---|
| 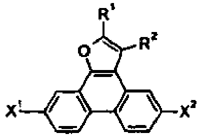 | | | |
| **Verbindg.** | **R¹ = R²** | **X¹** | **X²** |
|---|---|---|---|
| **C-1** | 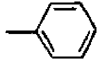 | **AR-1** | **AR-1** |
| **C-2** | 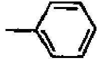 | **AR-2** | **AR-2** |
| **C-3** | 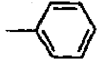 | **AR-3** | **AR-3** |
| **C-4** | ¹⁾ | **AR-1** | **AR-1** |
| **C-5** | ¹⁾ | **AR-2** | **AR-2** |
| **C-6** | ¹⁾ | **AR-3** | **AR-3** |
| | | | |
|---|---|---|---|
| 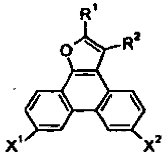 | | | |
| **Verbindg.** | **R¹ = R²** | **X¹** | **X²** |
|---|---|---|---|
| **D-1** | 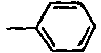 | **AR-1** | **AR-1** |
| **D-2** | 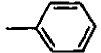 | **AR-2** | **AR-2** |
| **D-3** | 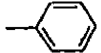 | **AR-3** | **AR-3** |
| **D-4** | ¹⁾ | **AR-1** | **AR-1** |
| **D-5** | | **AR-2** | **AR-2** |
| **D-6** | ¹⁾ | **AR-3** | **AR-3** |
| | | | |
|---|---|---|---|
| 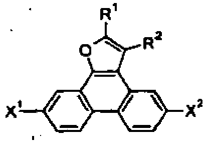 | | | |
| **Verbindg.** | **R¹ = R²** | **X¹** | **X²** |
|---|---|---|---|
| **E-1** | 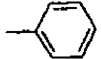 | **HE-1** | **HE-1** |
| **E-2** | 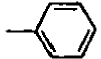 | **HE-2** | **HE-2** |
| **E-3** | 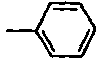 | **HE-3** | **HE-3** |
| **E-4** | 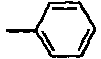 | **HE-4** | **HE-4** |
| **E-5** | 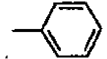 | **HE-5** | **HE-5** |
| **E-6** | 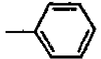 | **HE-6** | **HE-6** |
| **E-7** | 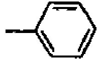 | **HE-7** | **HE-7** |
| **E-8** | 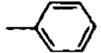 | **HE-8** | **HE-8** |
| **E-9** | 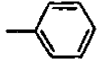 | **HE-9** | **HE-9** |
| **E-10** | ¹⁾ | **HE-1** | **HE-1** |
| **E-1** | ¹⁾ | **HE-2** | **HE-2** |
| **E-12** | ¹⁾ | **HE-3** | **HE-3** |
| **E-13** | ¹⁾ | **HE-4** | **HE-4** |
| **E-14** | ¹⁾ | **HE-5** | **HE-5** |
| **E-15** | ¹⁾ | **HE-6** | **HE-6** |
| **E-16** | ¹⁾ | **HE-7** | **HE-7** |
| **E-17** | ¹⁾ | **HE-8** | **HE-8** |
| **E-18** | ¹⁾ | **HE-9** | **HE-9** |
| **E-19** | 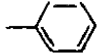 | **HE-10** | **HE-10** |
| **E-20** | ¹⁾ | **HE-10** | **HE-10** |
| | | | |
|---|---|---|---|
| 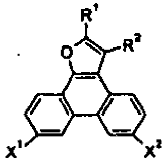 | | | |
| **Verbindg.** | **R¹ = R²** | **X¹** | **X²** |
|---|---|---|---|
| **F-1** | 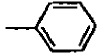 | **HE-1** | **HE-1** |
| **F-2** | 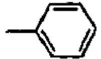 | **HE-2** | **HE-2** |
| **F-3** | 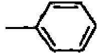 | **HE-3** | **HE-3** |
| **F-4** | 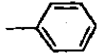 | **HE-4** | **HE-4** |
| **F-5** | 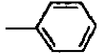 | **HE-5** | **HE-5** |
| **F-6** | 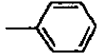 | **HE-6** | **HE-6** |
| **F-7** | 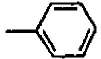 | **HE-7** | **HE-7** |
| **F-8** | 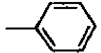 | **HE-8** | **HE-8** |
| **F-9** | 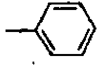 | **HE-9** | **HE-9** |
| **F-10** | ¹⁾ | **HE-1** | **HE-1** |
| **F-11** | ¹⁾ | **HE-2** | **HE-2** |
| **F-12** | ¹⁾ | **HE-3** | **HE-3** |
| **F-13** | ¹⁾ | **HE-4** | **HE-4** |
| **F-14** | ¹⁾ | **HE-5** | **HE-5** |
| **F-15** | ¹⁾ | **HE-6** | **HE-6** |
| **F-16** | ¹⁾ | **HE-7** | **HE-7** |
| **F-17** | ¹⁾ | **HE-8** | **HE-8** |
| **F-18** | ¹⁾ | **HE-9** | **HE-9** |
| **F-19** | 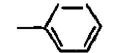 | **HE-10** | **HE-10** |
| **F-20** | ¹⁾ | **HE-10** | **HE-10** |
| | | | |
|---|---|---|---|
| ¹⁾ R¹ und R² bilden zusammeneine Gruppe 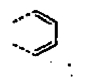 ²⁾ A¹ und A^{1'} bilden zusammen eine Gruppe 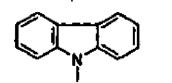 AR-1 ist 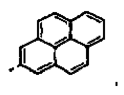 AR-2 ist 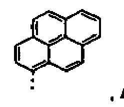 AR-3 ist 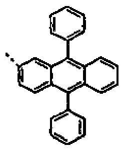 HE-1 ist 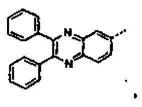 HE-2 ist 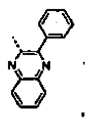 HE-3 ist 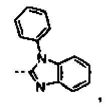 HE-4 ist 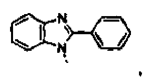 HE-5 ist 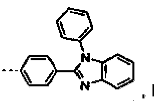 HE-6 ist 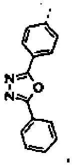 HE-7 ist 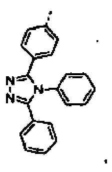 HE-8 ist 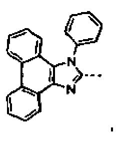 HE-9 ist 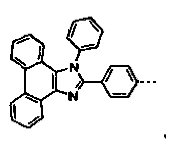 H-10 ist 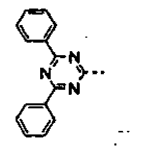 | | | |

9. Elektronische Einheit, umfassend eine Verbindung gemäß einem der Ansprüche 1 bis 8.

10. Elektronische Einheit gemäß Anspruch 9, die eine elektrolumineszierende Einheit ist.

11. Löchertransportschicht oder Elektronentransportschicht, umfassend eine Verbindung gemäß einem der Ansprüche 1 bis 8.

12. Vorrichtung, ausgewählt aus der Gruppe bestehend aus stationären visuellen Anzeigeeinheiten und mobilen visuellen Anzeigeeinheiten; Beleuchtungseinheiten; Tastaturen; Kleidungsgegenständen; Möbeln; Tapeten, umfassend die organische elektronische Einheit gemäß Anspruch 9 oder 10 oder die Löchertransport- oder Elektronentransportschicht gemäß Anspruch 11.

13. Verwendung der Verbindungen der Formel I gemäß Anspruch 1 für elektrophotographische Photorezeptoren, photoelektrische Wandler, organische Solarzellen, Schalterelemente, organische lichtemittierende Feldeffekttransistoren, Bildsensoren, Farbstofflaser und elektrolumineszierende Einheiten.

14. Verfahren zum Herstellen von Verbindungen der Formel I, wobei X¹ und X² unabhängig voneinander -NA¹A^{1'}, 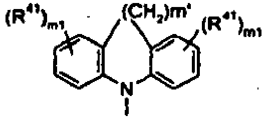 oder 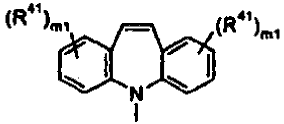 ist; m' 0, 1 oder 2 ist; umfassend Umsetzen einer Verbindung der Formel 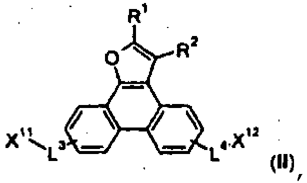 wobei
X¹¹ und X¹² für Halogen stehen, mit einer Verbindung der Formel HNA¹A^{1'}, 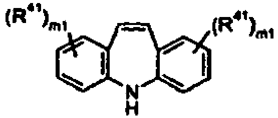 oder 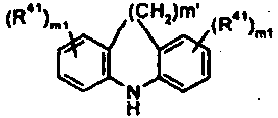 in Gegenwart einer Base und eines Katalysators in einem Lösungsmittel, wobei A¹, A^{1'}, L³, L⁴, R¹, R², R⁴¹ und m1 wie in Anspruch 1 definiert sind.

15. Verbindung der Formel 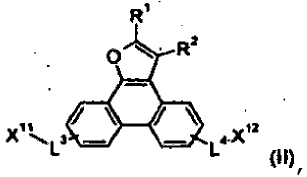 wobei L³, L⁴, R¹ und R² wie in Anspruch 1 definiert sind; X¹¹ und X¹² bei jedem Auftreten unabhängig ein Halogenatom oder -OS (O)₂CF₃, -OS(O)₂-Aryl, -OS (O)₂CH₃, -B(OH)₂, -B(OY¹)₂, 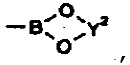 -BF₄Na oder -BF₄K sind, wobei Y¹ bei jedem Auftreten unabhängig eine C₁-C₁₀Alkylgruppe ist und Y² bei jedem Auftreten unabhängig eine C₁-C₁₀Alkylengruppe ist.

## Revendications

1. Composé de formule 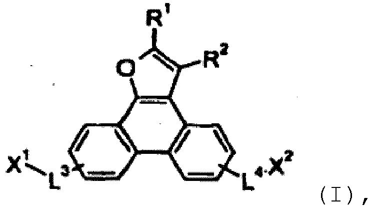 dans laquelle R¹ et R² représentent indépendamment l'un de l'autre H, F, un alkyle en C₁-C₁₈, un alkyle en C₁-C₁₈ qui est substitué par E et/ou interrompu par D, un aryle en C₆-C₂₄, un aryle en C₆-C₂₄ qui est substitué par G, un hétéroaryle en C₂-C₂₀, ou un hétéroaryle en C₂-C₂₀ qui est substitué par G, ou
R¹ et R² forment ensemble un groupe 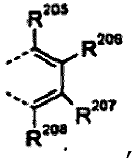 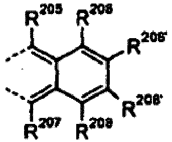 ou 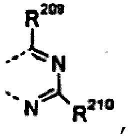 dans lesquels R^{206'}, R^{208'}, R²⁰⁵, R²⁰⁶, R²⁰⁷, R²⁰⁸, R²⁰⁹ et R²¹⁰ représentent indépendamment les uns des autres H, un alkyle en C₁-C₁₈, un alkyle en C₁-C₁₈ qui est substitué par E et/ou interrompu par D, un alcoxy en C₁-C₁₈, un alcoxy en C₁-C₁₈ qui est substitué par E et/ou interrompu par D, un fluoroalkyle en C₁-C₁₈, un aryle en C₆-C₂₄, un aryle en C₆-C₂₄ qui est substitué par G, un hétéroaryle en C₂-C₂₀, un hétéroaryle en C₂-C₂₀ qui est substitué par G, un alcényle en C₂-C₁₈, un alcynyle en C₂-C₁₈, un aralkyle en C₇-C₂₅, un aralkyle en C₇-C₂₅ qui est substitué par G, CN, ou -CO-R²⁸ ;
X¹ et X² représentent indépendamment l'un de l'autre un groupe -NA¹A^{1'},
A¹ et A^{1'} représentent indépendamment l'un de l'autre un groupe aryle en C₆-C₂₄, un groupe aryle en C₆-C₂₄ qui est substitué par G, un groupe hétéroaryle en C₂-C₂₀, ou un groupe hétéroaryle en C₂-C₂₀ qui est substitué par G, ou A¹ et A^{1'}, conjointement avec l'atome d'azote auquel ils sont liés, forment un cycle ou système cyclique hétéroaromatique, un groupe aryle en C₁₀-C₂₈ qui peut éventuellement être substitué par G ; et/ou
-L³-X¹ et -L⁴-X² représentent indépendamment l'un de l'autre un groupe de formule 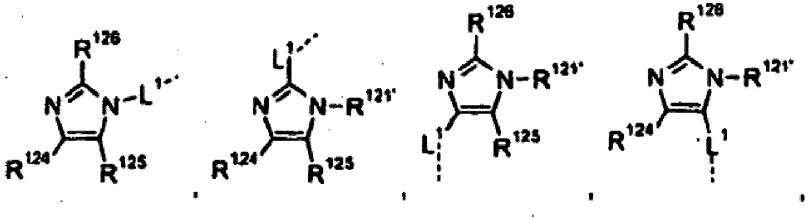 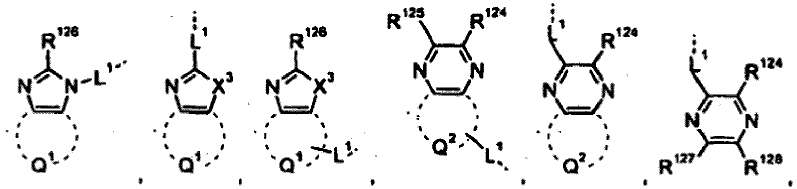 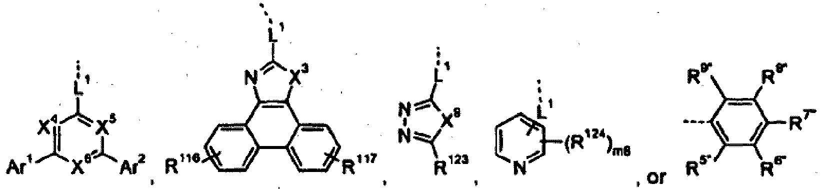 dans lesquels
R^{5"}, R^{6"}, R^{7"} et R^{8"} représentent indépendamment les uns des autres un aryle en en C₆-C₁₈ qui peut éventuellement être substitué par G, ou un hétéroaryle en C₂-C₂₀ qui peut éventuellement être substitué par G,
R^{9"} représente un hydrogène, ou a la signification de R^{5"}, m6 est un entier de 1 à 4,
X³ représente O, S ou N-R^{121'},
X⁹ représente O, S ou N-R^{121'},
Q¹ et Q² représentent des atomes nécessaires pour former un noyau aromatique carbocyclique ou aromatique hétérocyclique, qui peut éventuellement être condensé à un ou plusieurs autres cycles pour former un noyau condensé, et/ou qui peut éventuellement être substitué par G,
R¹¹⁶ et R¹¹⁷ représentent indépendamment l'un de l'autre H, un halogène, -CN, un alkyle en C₁-C₁₈, un alkyle en C₁-C₁₈ qui est substitué par E et/ou interrompu par D, un aryle en C₆-C₂₄, un aryle en C₆-C₂₄ qui est substitué par G, un hétéroaryle en C₂-C₂₀, un hétéroaryle en C₂-C₂₀ qui est substitué par G, un alcényle en C₂-C₁₈, un alcynyle en C₂-C₁₈, un alcoxy en C₁-C₁₈, un alcoxy en C₁-C₁₈ qui est substitué par E et/ou interrompu par D, un aralkyle en C₇-C₂₅, un aralkyle en C₇-C₂₅ qui est substitué par G, -C(=O)-R⁷⁸, -C(=O)OR⁷⁷, ou -C(=O)NR⁷⁵R⁷⁶,
ou
les substituants R¹¹⁶ et R¹¹⁷ qui sont adjacents l'un de l'autre peuvent former un cycle,
R⁷⁵, R⁷⁶ et R⁷⁸ représentent indépendamment les uns des autres H, un aryle en C₆-C₁₈, un aryle en C₆-C₁₈ qui est substitué par un groupe alkyle en C₁-C₁₈ ou alcoxy en C₁-C₁₈, un alkyle en C₁-C₁₈, ou un alkyle en C₁-C₁₈ qui est interrompu par -O-,
R⁷⁷ représente un aryle en C₆-C₁₈, un aryle en C₆-C₁₈ qui est substitué par un groupe alkyle en C₁-C₁₈ ou alcoxy en C₁-C₁₈, un alkyle en C₁-C₁₈, ou un alkyle en C₁-C₁₈ qui est interrompu par -O-,
R^{121'} représente un aryle en C₆-C₁₈ ou un hétéroaryle en C₂-C₂₀ qui peut éventuellement être substitué par un groupe alkyle en C₁-C₁₈, fluoroalkyle en C₁-C₁₈ ou alcoxy en C₁-C₁₈, un alkyle en C₁-C₁₈, ou un alkyle en C₁-C₁₈ qui est interrompu par -O-,
R¹²³, R¹²⁴, R¹²⁵ et R¹²⁶ représentent indépendamment les uns des autres H, un alkyle en C₁-C₁₈, un alkyle en C₁-C₁₈ qui est substitué par E et/ou interrompu par D, un fluoroalkyle en C₁-C₁₈, un aryle en C₆-C₂₄ qui peut éventuellement être substitué par G, un hétéroaryle en C₂-C₂₀ qui peut éventuellement être substitué par G, un alcényle en C₂-C₁₈, un alcynyle en C₂-C₁₈, un alcoxy en C₁-C₁₈, un alcoxy en C₁-C₁₈ qui est substitué par E et/ou interrompu par D, ou un aralkyle en C₇-C₂₅, et
R¹²⁷ et R¹²⁸ représentent indépendamment l'un de l'autre H, CN, un alkyle en C₁-C₁₈, un alkyle en C₁-C₁₈ qui est substitué par E et/ou interrompu par D, un fluoroalkyle en C₁-C₁₈, un aryle en C₆-C₂₄ qui peut éventuellement être substitué par G, un hétéroaryle en C₂-C₂₀ qui peut éventuellement être substitué par G, un alcényle en C₂-C₁₈, un alcynyle en C₂-C₁₈, un alcoxy en C₁-C₁₈, un alcoxy en C₁-C₁₈ qui est substitué par E et/ou interrompu par D, ou un aralkyle en C₇-C₂₅, et
L¹ représente un simple liaison, -(CR⁴⁷=CR⁴⁸)ₘ₂-, -(Ar³)ₘ₃-, -[Ar³(Y¹)ₘ₅]ₘ₄-, -[(Y¹)ₘ₅Ar³]ₘ₄-, ou -[Ar³(Y²)ₘ₅Ar⁴]ₘ₄-, dans lesquels
Y¹ représente -(CR⁴⁷=CR⁴⁸)-,
Y² représente NR⁴⁹, O, S, C=O, C(=O)O, R⁴⁹ représentant H, un aryle en C₆-C₁₈ qui peut éventuellement être substitué par un groupe alkyle en C₁-C₁₈ ou alcoxy en C₁-C₁₈, un alkyle en C₁-C₁₈, ou un alkyle en C₁-C₁₈ qui est substitué par -O-,
R⁴⁷ et R⁴⁸ représentent indépendamment l'un de l'autre un hydrogène, un fluor, un alkyle en C₁-C₂₅, ou un aryle en C₆-C₂₄ qui peut éventuellement être substitué par G,
m2 est un entier de 1 à 10, m3 est un entier de 1 à 5, m4 est un entier de 1 à 5, m5 est un entier de 1 à 10, Ar¹ et Ar² représentent indépendamment l'un de l'autre un aryle en C₆-C₂₄ qui peut éventuellement être substitué par G, ou un hétéroaryle en C₂-C₂₀ qui peut éventuellement être substitué par G,
Ar³ et Ar⁴ représentent indépendamment l'un de l'autre un arylène ou un hétéroarylène qui peut éventuellement être substitué, et
X⁴, X⁵ et X⁶ représentent indépendamment les uns des autres N ou CH, à condition qu'au moins un des substituants X⁴, X⁵ et X⁶ représente N ; et
L³ et L⁴ représentent indépendamment l'un de l'autre une simple liaison, ou une unité de pontage BU ;
D représente -CO-, -COO-, -S-, -SO-, -SO₂-, -O-, -NR⁶⁵-, -SiR⁷⁰R⁷¹-, -POR⁷²-, -CR⁶³=CR⁶⁴-, ou -C≡C-,
E représente -OR⁶⁹, -SR⁶⁹, -NR⁶⁵R⁶⁶, -COR⁶⁸, -COOR⁶⁷, -CONR⁶⁵R⁶⁶, -CN, ou un halogène,
G représente E ou un alkyle en C₁-C₁₈,
R⁶³ et R⁶⁴ représentent indépendamment l'un de l'autre un aryle en C₆-C₁₈, un aryle en C₆-C₁₈ qui est substitué par un groupe alkyle en C₁-C₁₈ ou alcoxy en C₁-C₁₈, un alkyle en C₁-C₁₈, ou un alkyle en C₁-C₁₈ qui est interrompu par -O-,
R⁶⁵ et R⁶⁶ représentent indépendamment l'un de l'autre un aryle en C₆-C₁₈, un aryle en C₆-C₁₈ qui est substitué par un groupe alkyle en C₁-C₁₈ ou alcoxy en C₁-C₁₈, un alkyle en C₁-C₁₈, ou un alkyle en C₁-C₁₈ qui est interrompu par -O-, ou
R⁶⁵ et R⁶⁶ forment ensemble un cycle à cing ou six chaînons,
R⁶⁷ représente un aryle en C₆-C₁₈, un aryle en C₆-C₁₈ qui est substitué par un groupe alkyle en C₁-C₁₈ ou alcoxy en C₁-C₁₈, un alkyle en C₁-C₁₈, ou un alkyle en C₁-C₁₈ qui est interrompu par -O-,
R⁶⁸ représente H, un aryle en C₆-C₁₈, un aryle en C₆-C₁₈ qui est substitué par un groupe alkyle en C₁-C₁₈ ou alcoxy en C₁-C₁₈, un alkyle en C₁-C₁₈, ou un alkyle en C₁-C₁₈ qui est interrompu par -O-,
R⁶⁹ représente un aryle en C₆-C₁₈, un aryle en C₆-C₁₈ qui est substitué par un groupe alkyle en C₁-C₁₈ ou alcoxy en C₁-C₁₈, un alkyle en C₁-C₁₈, ou un alkyle en C₁-C₁₈ qui est interrompu par -O-,
R⁷⁰ et R⁷¹ représentent indépendamment l'un de l'autre un alkyle en C₁-C₁₈, un aryle en C₆-C₁₈, ou un aryle en C₆-C₁₈ qui est substitué par un groupe alkyle en C₁-C₁₈, et R⁷² représente un alkyle en C₁-C₁₈, un aryle en C₆-C₁₈, ou un aryle en C₆-C₁₈ qui est substitué par un groupe alkyle en C₁-C₁₈,
R⁴⁵ et R^{45'} représentent indépendamment l'un de l'autre un groupe alkyle en C₁-C₂₅, un groupe cycloalkyle en C₄-C₁₈ dans lequel un ou plusieurs atomes de carbone qui ne sont pas voisins les uns des autres pourraient être remplacés par -NR^{45"}-, -O-, -S-, -C(=O)-O- ou -O-C(=O)-O-, et/ou dans lequel un ou plusieurs atomes d'hydrogène peuvent être remplacés par F, un groupe aryle en C₆-C₂₄, ou un groupe aryloxy en C₆-C₂₄ dans lequel un ou plusieurs atomes de carbone peuvent être remplacés par 0, S ou N, et/ou qui peut être substitué par un ou plusieurs groupes non aromatiques R⁴¹,
R^{45"} représente un groupe alkyle en C₁-C₂₅ ou un groupe cycloalkyle en C₄-C₁₈, et
m1 peut être identique ou différent à chaque occurrence et vaut 0, 1, 2, 3 ou 4.

2. Composé selon la revendication 1, qui est un composé de formule 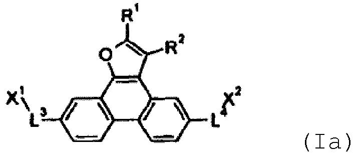 ou 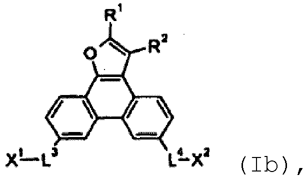 dans lesquelles X¹, X², L³, L⁴, R¹ et R² sont tels que définis dans la revendication 1.

3. Composé selon la revendication 1 ou 2, dans lequel R¹ et R² représentent un groupe de formule 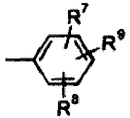 ou 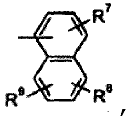 dans lesquelles R⁷, R⁸ et R⁹ représentent indépendamment les uns des autres H, un alkyle en C₁-C₁₈, un alcoxy en C₁-C₁₈, ou un alkyle en C₁-C₁₈ qui est interrompu par O ; ou R¹ et R² forment ensemble un groupe 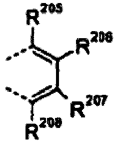 dans lequel R²⁰⁵, R²⁰⁶, R²⁰⁷ et R²⁰⁸ représentent indépendamment les uns des autres H, un alkyle en C₁-C₁₈, un alkyle en C₁-C₁₈ qui est interrompu par 0, un alcoxy en C₁-C₁₈, un alcoxy en C₁-C₁₈ qui est interrompu par 0, ou un fluoroalkyle en C₁-C₁₈.

4. Composé selon l'une quelconque des revendications 1 à 3, dans lequel -L³-X¹ et -L⁴-X² représentent indépendamment l'un de l'autre un groupe de formule -NA¹A^{1'} ou un groupe 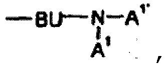 dans lesquels A¹ et A^{1'} représentent indépendamment l'un de l'autre 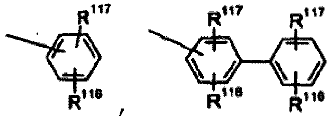 ou 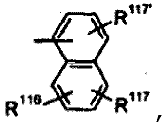 ou
A¹ et A^{1'}, conjointement avec l'atome d'azote auquel ils sont liés, forment un cycle ou système cyclique
hétéroaromatique 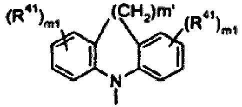 ou 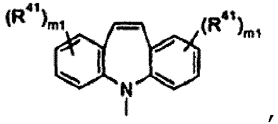 m' vaut 0, 1 ou 2,
m1 peut être identique ou différent à chaque occurrence et vaut 0, 1, 2, 3 ou 4,
R¹¹⁶, R¹¹⁷ et R^{117'} représentent indépendamment les uns des autres H, un halogène, -CN, un alkyle en C₁-C₁₈, un alkyle en C₁-C₁₈ qui est substitué par E et/ou interrompu par D, un aryle en C₆-C₂₄, un aryle en C₆-C₂₄ qui est substitué par G, un hétéroaryle en C₂-C₂₀, un hétéroaryle en C₂-C₂₀ qui est substitué par G, un alcényle en C₂-C₁₈, un alcynyle en C₂-C₁₈, un alcoxy en C₁-C₁₈, un alcoxy en C₁-C₁₈ qui est substitué par E et/ou interrompu par D, un aralkyle en C₇-C₂₅, -C(=O)-R⁷⁷, -C(=O)OR⁷⁸, ou -C(=O)NR⁷⁵R⁷⁶, ou
les substituants R¹¹⁶, R¹¹⁷ et R^{117'} qui sont adjacents les uns des autres peuvent former un cycle,
R⁷⁵, R⁷⁶ et R⁷⁸ représentent indépendamment les uns des autres H, un aryle en C₆-C₁₈, un aryle en C₆-C₁₈ qui est substitué par un groupe alkyle en C₁-C₁₈ ou alcoxy en C₁-C₁₈, un alkyle en C₁-C₁₈, ou un alkyle en C₁-C₁₈ qui est interrompu par -O-,
R⁷⁷ représente un aryle en C₆-C₁₈, un aryle en C₆-C₁₈ qui est substitué par un groupe alkyle en C₁-C₁₈ ou alcoxy en C₁-C₁₈, un alkyle en C₁-C₁₈, ou un alkyle en C₁-C₁₈ qui est interrompu par -O-, et
BU représente 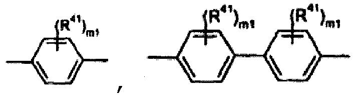 ou 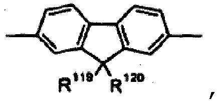 dans lesquels
m1 est tel que défini ci-dessus,
R⁴¹ peut être identique ou différent à chaque occurrence et représente Cl, F, CN, NR⁴⁵R⁴⁵', un groupe alkyle en C₁-C₂₅, un groupe cycloalkyle en C₄-C₁₈, un groupe alcoxy en C₁-C₂₅ dans lequel un ou plusieurs atomes de carbone qui ne sont pas voisins les uns des autres pourraient être remplacés par -NR⁴⁵-, -O-, -S-, -C(=O)-O- ou -O-C(=O)-O-, et/ou dans lequel un ou plusieurs atomes d'hydrogène peuvent être remplacés par F, un groupe aryle en C₆-C₂₄, ou un groupe aryloxy en C₆-C₂₄ dans lequel un ou plusieurs atomes de carbone peuvent être remplacés par 0, S ou N, et/ou qui peut être substitué par un ou plusieurs groupes non aromatiques R⁴¹, ou
au moins deux groupes R⁴¹ forment un système cyclique, R⁴⁷ et R⁴⁸ représentent indépendamment l'un de l'autre H, un alkyle en C₁-C₁₈, ou un aryle en C₆-C₁₀ qui peut éventuellement être substitué par un ou plusieurs groupes alkyle en C₁-C₈ ou groupes alcoxy en C₁-C₈,
R¹¹⁹ et R¹²⁰ représentent indépendamment l'un de l'autre un alkyle en C₁-C₁₈, un alkyle en C₁-C₁₈ qui est substitué par E et/ou interrompu par D, un aryle en C₆-C₂₄, un aryle en C₆-C₂₄ qui est substitué par G, un hétéroaryle en C₂-C₂₀, un hétéroaryle en C₂-C₂₀ qui est substitué par G, un alcényle en C₂-C₁₈, un alcynyle en C₂-C₁₈, un alcoxy en C₁-C₁₈, un alcoxy en C₁-C₁₈ qui est substitué par E et/ou interrompu par D, ou un aralkyle en C₇-C₂₅, ou
R¹¹⁹ et R¹²⁰ forment ensemble un groupe de formule =CR¹²¹R¹²² dans laquelle
R¹²¹ et R¹²² représentent indépendamment l'un de l'autre H, un alkyle en C₁-C₁₈, un alkyle en C₁-C₁₈ qui est substitué par E et/ou interrompu par D, un aryle en C₆-C₂₄, un aryle en C₆-C₂₄ qui est substitué par G, un hétéroaryle en C₂-C₂₀, ou un hétéroaryle en C₂-C₂₀ qui est substitué par G, ou
R¹¹⁹ et R¹²⁰ forment ensemble un cycle à cinq ou six chaînons qui peut éventuellement être substitué par un alkyle en C₁-C₁₈, un alkyle en C₁-C₁₈ qui est substitué par E et/ou interrompu par D, un aryle en C₆-C₂₄, un aryle en C₆-C₂₄ qui est substitué par G, un hétéroaryle en C₂-C₂₀, un hétéroaryle en C₂-C₂₀ qui est substitué par G, un alcényle en C₂-C₁₈, un alcynyle en C₂-C₁₈, un alcoxy en C₁-C₁₈, un alcoxy en C₁-C₁₈ qui est substitué par E et/ou interrompu par D, un aralkyle en C₇-C₂₅, ou -C(=O)-R²⁸, et
R²⁸ représente H, un aryle en C₆-C₁₈, un aryle en C₆-C₁₈ qui est substitué par un groupe alkyle en C₁-C₁₈ ou alcoxy en C₁-C₁₈, un alkyle en C₁-C₁₈, ou un alkyle en C₁-C₁₈ qui est interrompu par -O-, ou
un groupe de formule 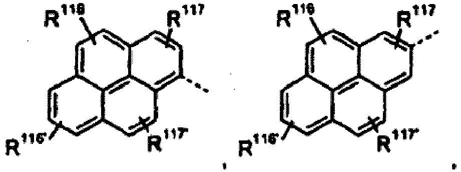 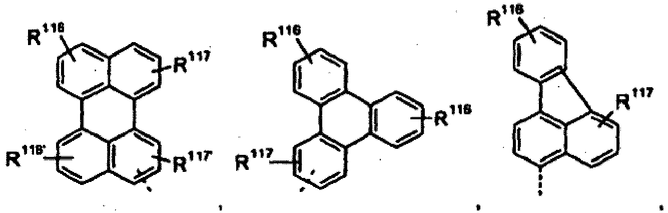 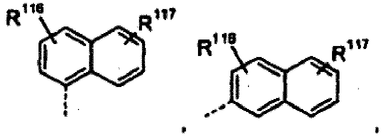 or 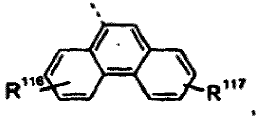 dans lesquelles R^{116'} a la signification de R¹¹⁶, R¹¹⁶, R¹¹⁷ et R^{117'} sont tels que définis ci-dessus, et D, E et G sont tels que définis dans la revendication 1, ou
un groupe de formule 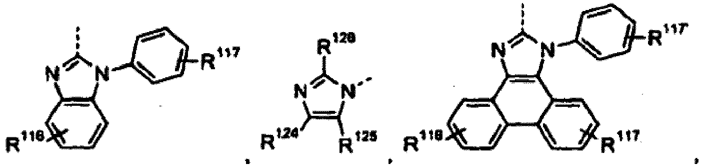 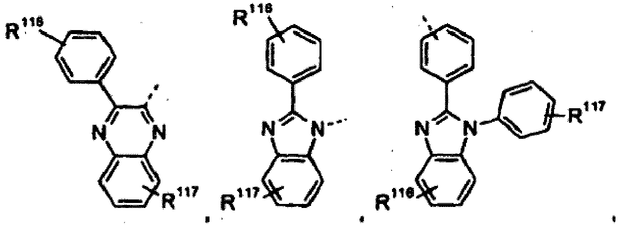 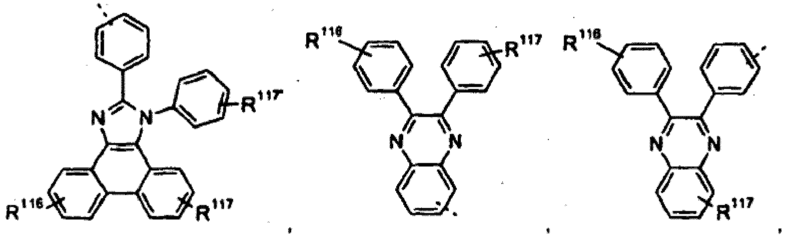 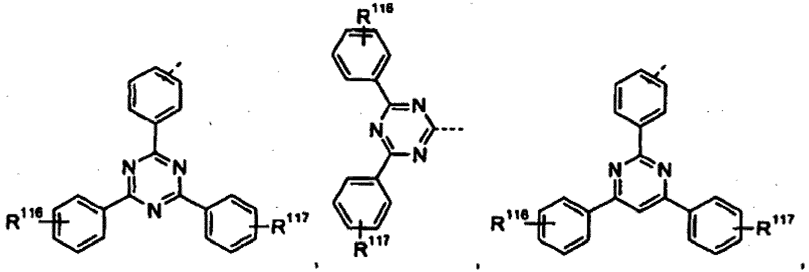 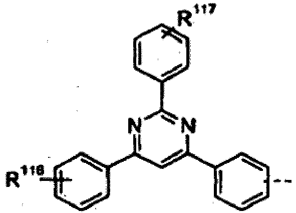 or 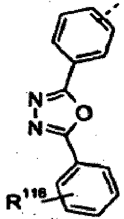 dans lesquelles R¹¹⁶, R¹¹⁷ et R^{117'} sont tels que définis ci-dessus, et R¹²⁴, R¹²⁵ et R¹²⁶ sont tels que définis dans la revendication 1.

5. Composé selon la revendication 4, dans lequel -L³-X¹ et -L⁴-X² représentent indépendamment l'un de l'autre un groupe de formule -NA¹A^{1'} ou un groupe 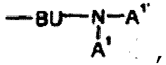 dans lesquels
A¹ et A^{1'} représentent indépendamment l'un de l'autre 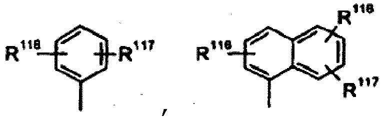 ou 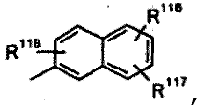 ou
A¹ et A^{1'}, conjointement avec l'atome d'azote auquel ils sont liés, forment un groupe de formule 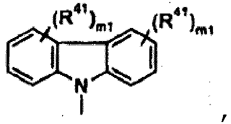 R¹¹⁶ et R¹¹⁷ représentent indépendamment l'un de l'autre un alkyle en C₁-C₁₈ qui peut éventuellement être interrompu par -O-, ou un alcoxy en C₁-C₁₈, et
BU représente 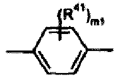 ou 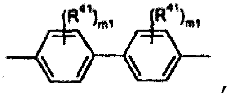 dans lesquels R⁴¹ peut être identique ou différent à chaque occurrence et représente un alkyle en C₁-C₁₈ qui peut éventuellement être interrompu par -O-, ou un alcoxy en C₁-C₁₈, et
m1 vaut 0, 1 ou 2.

6. Composé selon la revendication 4, dans lequel -L³-X¹ et -L⁴-X² représentent indépendamment l'un de l'autre un groupe de formule 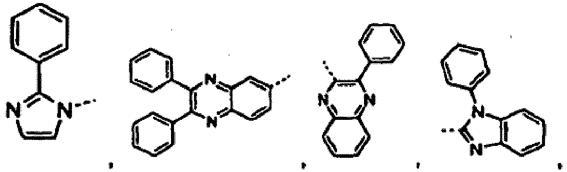 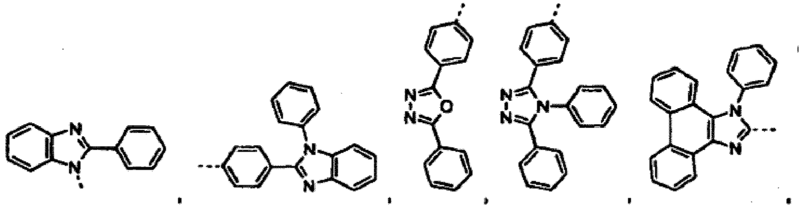 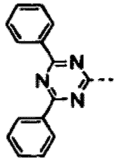 or

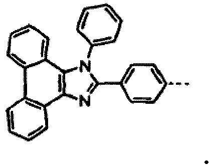

7. Composé selon la revendication 4, dans lequel -L³-X¹ et -L⁴-X² représentent indépendamment l'un de l'autre un groupe de formule 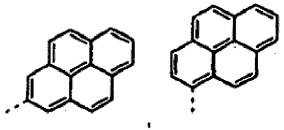 or

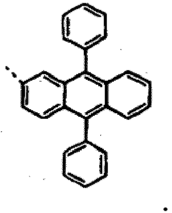

8. Composé selon l'une quelconque des revendications 1 à 7, qui est choisi parmi 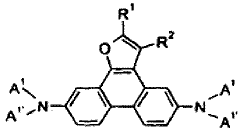
| **Cpd.** | **R¹=R²** | **A**¹ | **A^{1'}** |
|---|---|---|---|
| **A-1** | 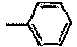 | 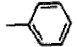 | 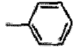 |
| **A-2** | 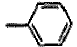 | 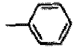 | 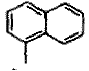 |
| **A-3** | 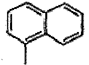 | 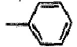 | 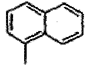 |
| **A-4** | 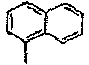 | 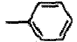 | 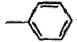 |
| **A-5** | 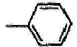 | 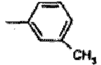 | 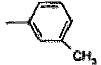 |
| **A-6** | ¹⁾ | 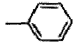 | 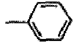 |
| **A-7** | ¹⁾ | ²⁾ | ²⁾ |
| **A-8** | 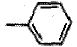 | ²⁾ | ²⁾ |
| **A-9** | 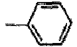 | 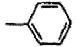 | 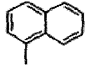 |
| | | | |
|---|---|---|---|
| 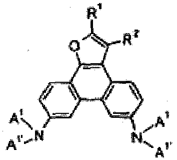 | | | |
| **Cpd.** | **R¹ = R²** | **A¹** | **A^{1'}** |
|---|---|---|---|
| **B**-**1** | 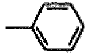 | 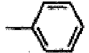 | 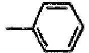 |
| **B-2** | 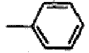 | 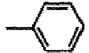 | 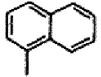 |
| **B-3** | 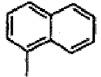 | 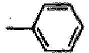 | 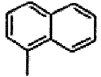 |
| **B-4** | 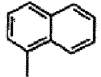 | 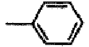 | 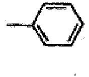 |
| **B-5** | 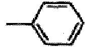 | 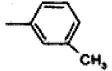 | 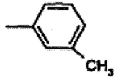 |
| **B-6** | ¹⁾ | 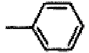 | 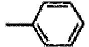 |
| **B-7** | ¹⁾ | ²⁾ | ²⁾ |
| **B-8** | 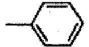 | ²⁾ | ²⁾ |
| **B-9** | 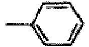 | 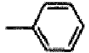 | 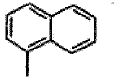 |
| | | | |
|---|---|---|---|
| 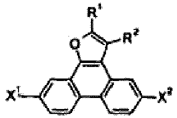 | | | |
| **Cpd.** | **R¹ = R²** | **X**¹ | **X**² |
|---|---|---|---|
| **C-1** | 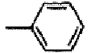 | **AR-1** | **AR-1** |
| **C-2** | 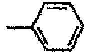 | **AR-2** | **AR-2** |
| **C-3** | 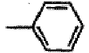 | **AR-3** | **AR-3** |
| **C-4** | ¹⁾ | **AR-1** | **AR-1** |
| **C-5** | ¹⁾ | **AR-2** | **AR-2** |
| **C**-**6** | ¹⁾ | **AR-3** | **AR-3** |
| | | | |
|---|---|---|---|
| 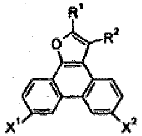 | | | |
| **Cpd**. | **R¹ = R²** | **X¹** | **X**² |
|---|---|---|---|
| **D-1** | 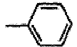 | **AR-1** | **AR-1** |
| **D-2** | 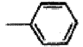 | **AR-2** | **AR-2** |
| **D-3** | 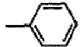 | **AR-3** | **AR-3** |
| **D-4** | ¹⁾ | **AR-1** | **AR-1** |
| **D-6** | ¹⁾ | **AR-2** | **AR-2** |
| **D-6** | ¹⁾ | **AR-3** | **AR-3** |
| | | | |
|---|---|---|---|
| 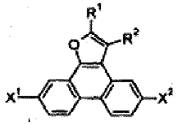 | | | |
| **Cpd**. | **R¹ = R²** | **X¹** | **X²** |
|---|---|---|---|
| **E-1** | 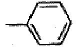 | **HE-1** | **HE-1** |
| **E-2** | 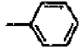 | H**E-2** | **HE-2** |
| **E-3** | 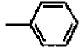 | **HE-3** | **HE-3** |
| **E-4** | 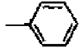 | **HE-4** | **NE-**4 |
| **E-5** | 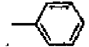 | **HE-5** | **HE-5** ^{.} |
| **E-6** | 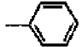 | **HE-6** | **HE-6** |
| **E-7** | 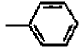 | **HE-7** | **HE-7** |
| **E-8** | 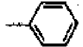 | **HE-8** | **HE-8** |
| **E-9** | 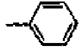 | **HE-9** | **HE-9** |
| **E-10** | ¹⁾ | **HE-1** | **HE-1** |
| **E-11** | ¹⁾ | **HE-2** | **HE-2** |
| **E-12** | ¹⁾ | **HE-3** | **HE-3** |
| **E-13** | ¹⁾ | **HE-4** | **HE-4** |
| **E-14** | ¹⁾ | **HE-5** | **HE-5** |
| **E-15** | ¹⁾ | **HE-6** | **HE-6** |
| **E-16** | ¹⁾ | **HE-7** | **HE-7** |
| **E-17** | ¹⁾ | **HE-8** | **HE-8** |
| **E-18** | ¹⁾ | **HE-9** | **HE-9** |
| **E-19** | 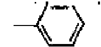 | **HE-10** | **HE-10** |
| **E-20** | ¹⁾ | **HE-10** | **HE-10** |
| | | | |
|---|---|---|---|
| 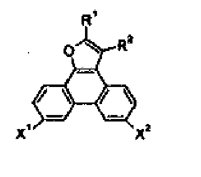 | | | |
| **Cpd**. | **R¹ = R²** | **X**¹ | **X²** |
|---|---|---|---|
| **F-1** | 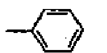 | **HE-1** | **HE-1** |
| **F-2** | 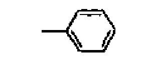 | **HE-2** | **HE-2** |
| **F-3** | 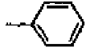 | **H-3** | **HE-3** |
| **F-4** | 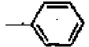 | **HE-4** | **HE-4** |
| **F-6** | 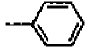 | **HE-5** | **HE-5** |
| **F-6** | 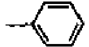 | **HE-6** | **HE-6** |
| **F-7** | 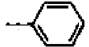 | **HE-7** | **HE-7** |
| **F-8** | 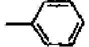 | **HE-8** | **HE-8** |
| **F-9** | 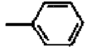 | **HE-9** | **HE-9** |
| **F-10** | ¹⁾ | **HE-1** | **HE-1** |
| **F-11** | ¹⁾ | **HE-2** | **HE-2** |
| **F-12** | ¹⁾ | **HE-3** | **HE-3** |
| **F-13** | ¹⁾ | **HE-4** | **HE**-**4** |
| **F-14** | ¹⁾ | **HE-5** | **HE-5** |
| **F-15** | ¹⁾ | **HE-6** | **HE-6** |
| **F-16** | ¹⁾ | **HE-7** | **HE-7** |
| **F-17** | ¹⁾ | **HE-8** | **HE-8** |
| **F-18** | ¹⁾ | **HE-9** | **HE-9** |
| **F-19** | 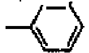 | **HE-10** | **HE-10** |
| **F-20** | ¹⁾ | **HE-10** | **HE-10** |
| | | | |
|---|---|---|---|
| ¹⁾ R¹ et R² forment ensemble un groupe 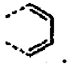 ²⁾ A¹ et A^{1'} forment ensemble un groupe 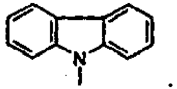 AR-1 est 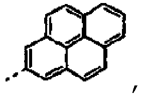 AR-2 est 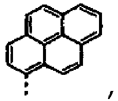 AR-3 est 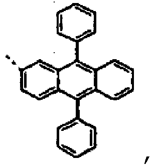 HE-1 est 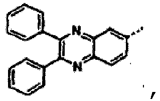 HE-2 est 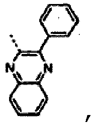 HE-3 est 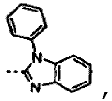 HE-4 est 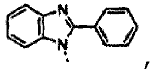 HE-5 est 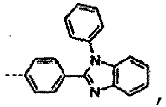 HE-6 est 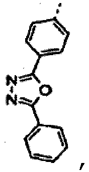 HE-7 est 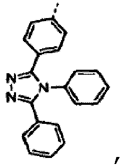 HE-8 est 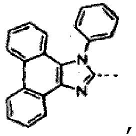 HE-9 est 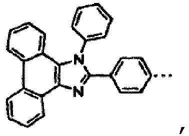 HE-10 est 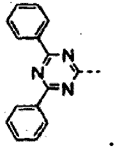 | | | |

9. Dispositif électronique comprenant un composé selon l'une quelconque des revendications 1 à 8.

10. Dispositif électronique selon la revendication 9, qui est un dispositif électroluminescent.

11. Couche de transport de trous, ou couche de transport d'électrons, comprenant un composé selon l'une quelconque des revendications 1 à 8.

12. Appareil choisi dans le groupe constitué par les unités d'affichage visuel stationnaires et les unités d'affichage visuel mobiles, les unités d'éclairage, les claviers, les éléments d'habillage, le mobilier et le papier peint, comprenant le dispositif électronique organique selon la revendication 9 ou 10, ou la couche de transport de trous ou de transport d'électrons selon la revendication 11.

13. Utilisation des composés de formule I selon la revendication 1 pour des photorécepteurs électrophotographiques, des convertisseurs photoélectriques, des cellules solaires organiques, des éléments de commutation, des transistors à effet de champ organiques émetteurs de lumière, des capteurs d'images, des lasers à colorants et des dispositifs électroluminescents.

14. Procédé de préparation de composés de formule I dans laquelle X¹ et X² représentent indépendamment l'un de l'autre -NA¹A^{1'}, 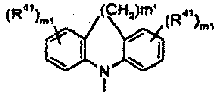 ou 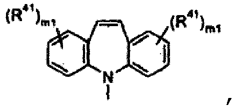 m' valant 0, 1 ou 2, qui comprend la réaction d'un composé de formule 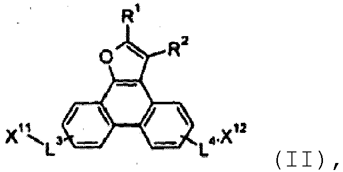 dans laquelle X¹¹ et X¹² représentent un halogène, avec un composé de formule -HNA¹A^{1'}, 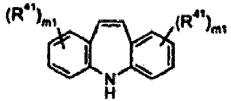 ou 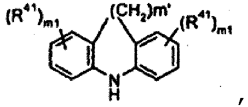 en présence d'une base et d'un catalyseur dans un solvant, dans lequel A¹, A^{1'}, L³, L⁴, R¹, R², R⁴¹ et m1 sont tels que définis dans la revendication 1.

15. Composé de formule 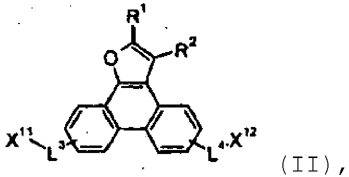 dans laquelle
L³, L⁴, R¹ et R² sont tels que définis dans la revendication 1 ; et
X¹¹ et X¹² représentent indépendamment à chaque occurrence un atome d'halogène, ou un groupe -OS(O)₂CF₃, -OS(O)₂-aryle, -OS(O)₂CH₃, -B(OH)₂, -B(OY¹)₂, 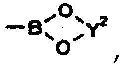 -BF₄Na ou -BF₄K, dans lesquels Y¹ représente indépendamment à chaque occurrence un groupe alkyle en C₁-C₁₀ et Y² représente indépendamment à chaque occurrence un groupe alkylène en C₂-C₁₀.
